# EUROPEAN PATENT APPLICATION

(11) **EP 4 491 623 A1**
(43) Date of publication of application: **15.01.2025**
(21) Application number: 23766149.1
(22) Date of filing: 10.03.2023
(51) Int. Cl.: C07D 403/14, A61K 31/53, A61P 31/12, A61P 31/14, A61P 31/16, A61P 31/18

(54) **TRIAZINE COMPOUND, INTERMEDIATE THEREOF, PREPARATION METHOD THEREFOR AND USE THEREOF**

(30) Priority: 10.03.2022 CN 202210245736; 20.05.2022 CN 202210557410
(71) Applicant: JKT Biopharma Co., Ltd., Wuhan, Hubei 430040 (CN)
(72) Inventor: SHEN, Xiaokun, Shanghai 201318 (CN); HUANG, Jinwen, Shanghai 201318 (CN)
(74) Representative: Potter Clarkson
(86) International application number: PCT/CN2023/080920
(87) International publication number: WO 2023/169572

(57) **Abstract**

A triazine compound, an intermediate thereof, and a preparation method therefor and the use thereof. Specifically, the present invention relates to a triazine derivative as represented by formula (I') or a pharmaceutically acceptable salt thereof. On the basis of retaining the effectiveness against SARS-CoV-2, the compound significantly prolongs the half-life period, reduces the dosage requirement, reduces side effects, expands the range of therapeutic window, and has very good prospects when used for manufacturing a drug for treating diseases related to coronavirus infections.

## Description

This application claims priority for the Chinese patent application 202210245736X with a filing date of 2022/3/10 and the Chinese patent application 2022105574100 with a filing date of 2022/5/20. This application quotes the full text of the above-mentioned Chinese patent application.

### Technical field

The invention relates to a triazine compound, an intermediate thereof, and a preparation method thereof and an application thereof.

### Background technique

Coronaviruses (CoV) are a class of pathogenic microorganisms that are seriously harmful to humans. To date, a total of seven coronaviruses that can infect humans have been found, namely SARS-CoV, MERS-CoV, SARS-CoV-2 (novel coronavirus, also known as 2019-nCoV, HCoV-229E, HCoV-OC43, HCoV-NL63 and HCoV-HKUI. The disease caused by the novel coronavirus (COVID-19) is caused by infection with the novel coronavirus (SARS-CoV-2). The general symptoms of SARS-CoV-2 infection are fever, fatigue, dry cough, and progressive dyspnea. some patients have mild onset symptoms, or even no obvious fever. Severe symptoms include: Acute respiratory distress syndrome, septic shock, refractory metabolic acidosis, and coagulation dysfunction. SARS-CoV-2 virus positive can be detected in nasopharyngeal swabs, sputum, respiratory secretions, blood, feces, etc., in patients infected with SARS-CoV-2. Chest imaging shows multiple small spots and interstitial changes in the early stage. It is obvious in the outer lung zone. Further, it develops into multiple grinding glass shadows and infiltrating shadows in both lungs. In severe cases, lung consolidation may occur, and pleural effusion is rare. So far, more than 400 million people have been infected worldwide, with more than 6 million cumulative deaths.

Although many vaccines have been approved and large-scale vaccination has begun, infections after vaccination are not uncommon due to the mutation of the virus and the protective effectiveness of the vaccine. At present, among the targeted drugs of COVID-19 virus, Gilead RNA polymerase (RdRp) inhibitor Radesivir and Merck CoV oral drug molnupiravir have been approved for marketing. Roche and REGN-COV2 (casirivimab/imdevimab), a regeneration-targeted S-protein cocktail, have been granted Emergency Use authorization (EUA) in the United States.

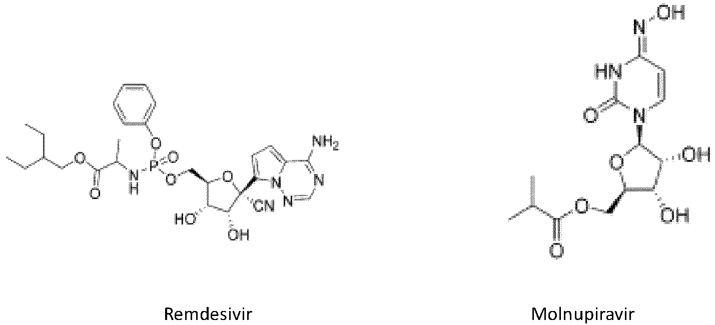

3CL protease (3C-likeprotease, 3CLpro) is the main protease that cuts and processes RNA in the virus's own coding, and it is the main protease produced by novel coronavirus (2019-nCoV, SARS-CoV-2). It plays a key role in the replication process of coronavirus, and its sequence is highly conserved. It is a popular target for anti-coronavirus drug research and development. At present, many 3CL protein inhibitors are in the clinical research and development stage. Among them, Pfizer's polypeptide 3CL protease inhibitor PF-07321332 has a single drug inhibitory activity on the virus at the molecular level: IC50 = 19nM. PF-07321332 single drug pair in human airway epithelial cells, HeLa and A549 cells expressing ACE2 protein viral inhibitory activity: EC50 is 62, 99 and 56nM respectively, showed good clinical effects, and its compound preparation Paxlovid (PF-07321332 / Ritonavir) was approved by the FDA for emergency use. S-217622 is a non-peptide small molecule 3CL protein inhibitor developed by Shionogi Company of Japan. It has been found in vitro experiments and has inhibitory activity against SARS-Cov-2, SAR, MERS and human coronavirus HCoV-229E, etc. It is effective against the mutation of novel coronavirus and has stronger inhibitory activity against Omicron strain. S-217622 is now in Phase 2-3 clinical trials.

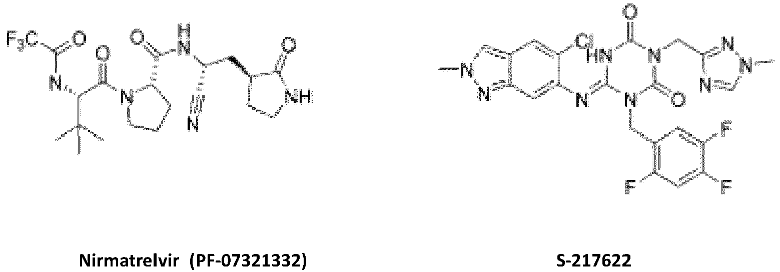

At present, there are no reports on the deuterated products and prodrugs of S-217622 compound and its analogues. In view of its important role in controlling the novel coronavirus epidemic, it is of great clinical significance to study its deuterated products and prodrugs to further improve the metabolism of S-217622 in vivo and expand the therapeutic window.

### Content of invention

The technical problem to be solved by the invention is the study of the deuterated compound lacking S-217622 in the prior art and the relatively simple structure of its analogs. Therefore, the invention provides a triazine compound, its intermediate, and a preparation method thereof and the use thereof. Based on retaining the effectiveness of SARS-CoV-2, the compound of the invention can significantly prolong the half-life, reduce the demand for dosage, reduce side effects, and expand the range of treatment window. Therefore, the invention has a very good prospect for making drugs for treating diseases related to coronavirus infection.

The invention provides a triazine derivative shown in formula (1) or a pharmaceutically acceptable salt thereof, 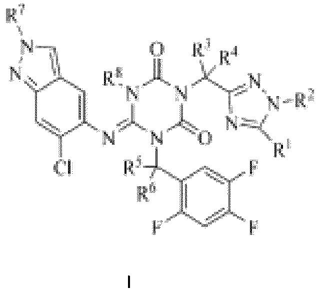
Where R¹ is hydrogen or deuterium,
R² is a methyl group or a methyl group replaced by 1, 2, or 3 tritium,
R³ is hydrogen or deuterium,
R⁴ is hydrogen or deuterium,
R⁵ is hydrogen or deuterium,
R⁶ is hydrogen or deuterium,
R⁷ is a methyl group or a methyl group replaced by 1, 2, or 3 tritium,
R⁸ is hydrogen, 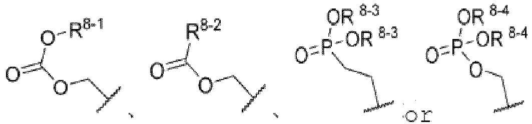
R⁸⁻¹ is C₁-C₁₀ alkyl,
R⁸⁻² is C₁-C₁₀ alkyl,
Each R⁸⁻³ is independently hydrogen, C₁-C₁₀ alkyl, or sodium,
Each R⁸⁻⁴ is independently hydrogen, C₁-C₁₀ alkyl, or sodium,

In addition, R¹, R², R³, R⁴, R⁵, R⁶, and R⁷ contain at least one deuterium.

The invention provides a triazine derivative shown in formula (1') or a pharmaceutically acceptable salt thereof, 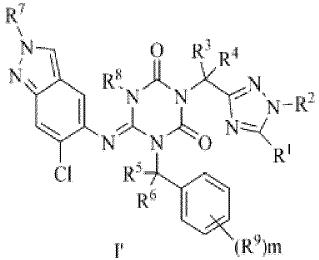
Where R¹ is hydrogen or deuterium,
R² is a methyl group or a methyl group replaced by 1, 2, or 3 tritium,
R³ is hydrogen or deuterium,
R⁴ is hydrogen or deuterium,
R⁵ is hydrogen or deuterium,
R⁶ is hydrogen or deuterium,
R⁷ is a methyl group or a methyl group replaced by 1, 2, or 3 tritium,
R⁸ is hydrogen, 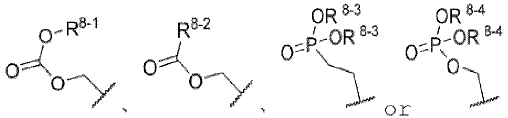
R⁸⁻¹ is C₁-C₁₀ alkyl,
R⁸⁻² is C₁-C₁₀ alkyl,
Each R⁸⁻³ is independently hydrogen, C₁-C₁₀ alkyl, or sodium,
Each R⁸⁻⁴ is independently hydrogen, C₁-C₁₀ alkyl, or sodium,
Each R9 is independently hydrogen or halogen,
m is 2, 3, 4 or 5,

Moreover, the compound shown in formula I' satisfies one or both of the following conditions:
(1) R¹, R², R³, R⁴, R⁵, R⁶ and R⁷ contain at least one tritium,
(2) R⁸ is

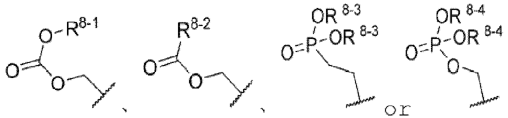

In one scheme, certain substituents in the triazine derivatives shown in formula (I) or their pharmaceutically acceptable salts may be further defined as follows, and substituents not covered below are defined as described in any scheme of the invention (hereinafter referred to as "in a scheme"):
In one scheme, certain substituents in a triazine derivative shown in formula (I') or in a pharmaceutically acceptable salt thereof may be further defined as follows, and substituents not covered below are defined as described in any scheme of the invention (hereinafter referred to as "in a scheme"):
In one scheme, R⁸⁻¹, the C₁-C₁₀ alkyl may be C₁-C₆ alkyl, preferably methyl, ethyl, n-propyl, isopropyl, n-butyl, isobutyl, sec-butyl, or tert-butyl, such as methyl, ethyl, or isopropyl.

In one scheme, R⁸⁻², the C₁-C₁₀ alkyl may be C₁-C₆ alkyl, preferably methyl, ethyl, n-propyl, isopropyl, n-butyl, isobutyl, sec-butyl, or tert-butyl, such as methyl, ethyl, isopropyl, n-butyl, or tert-butyl.

In a scheme, R⁸⁻³, the C₁-C₁₀ alkyl may be C₁-C₆ alkyl, preferably methyl, ethyl, n-propyl, isopropyl, n-butyl, isobutyl, sec-butyl, or tert-butyl.

In a scheme, R⁸⁻⁴, the C₁-C₁₀ alkyl group may be C₁-C₆ alkyl group, preferably methyl, ethyl, n-propyl, isopropyl, n-butyl, isobutyl, sec-butyl, or tert-butyl.

In a scheme, when R⁸ is hydrogen, the three classes of derivatives shown in formula (I) may also be their tautomers.

For example, 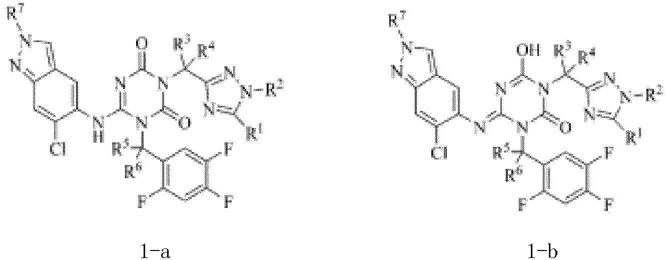 In one scheme, when R⁸ is hydrogen, the triazine derivative shown in the formula (I') may also be its tautomer, for example

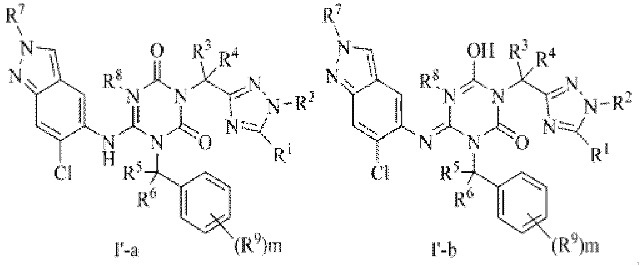

In one scheme, R⁹, the halogen may be fluorine, chlorine, bromine or iodine, such as fluorine.

In one scheme, R² is methyl or -CD₃.

In one scheme, R⁷ is methyl or -CD₃.

In one scheme, R⁸⁻¹ is C₁-C₆ alkyl.

In one scheme, R⁸⁻² is C₁-C₆ alkyl.

In one scheme, R⁸⁻³ is sodium.

In one scheme, R⁸⁻⁴ is sodium.

In one scheme, both R³ and R⁴ are deuterium.

In one scheme, both R⁵ and R⁶ are deuterium.

In one scheme, m is 2 or 3,

In one scheme, R⁹ is fluorine.

In one scheme, R⁸ is hydrogen, ,

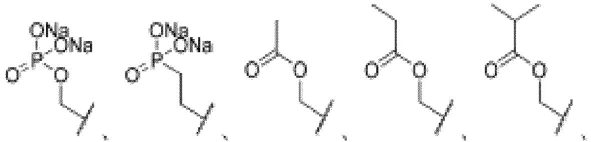

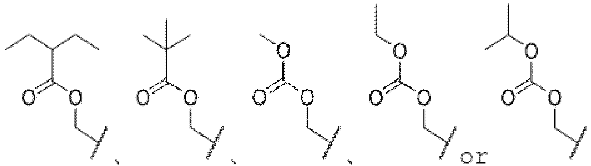

In one scheme,

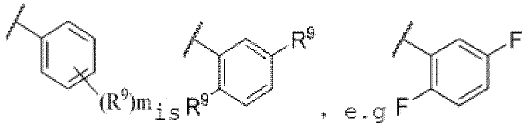

In one scheme, 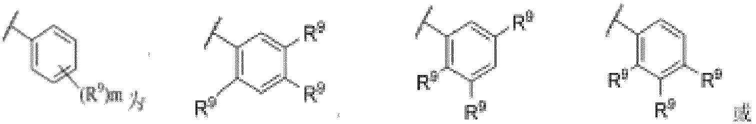 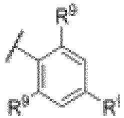 For example 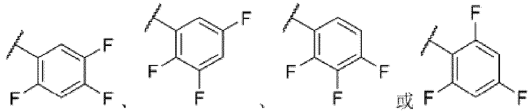 Better

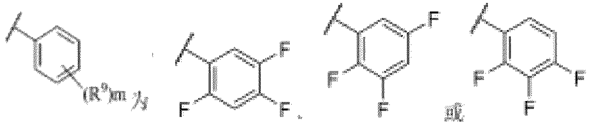

In one scheme, R¹ is deuterium and R² is methyl; R³ is hydrogen; R⁴ is hydrogen; R⁵ is hydrogen; R⁶ is hydrogen; R⁷ is methyl; R⁸ is hydrogen, 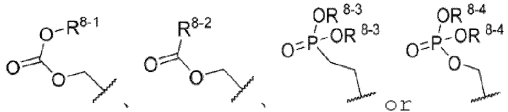
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium.

In one scheme, R¹ is deuterium and R² is methyl; R³ is hydrogen; R⁴ is hydrogen; R⁵ is hydrogen; R⁶ is hydrogen; R⁷ is methyl; R⁸ is hydrogen, 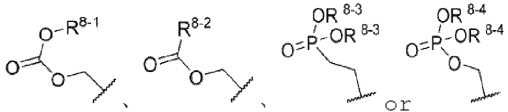
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium, each R⁹ is independently hydrogen or halogen:
   m is 2, 3, 4, or 5.

In one scheme, R¹ is deuterium and R² is -CD3; R³ is hydrogen; R⁴ is hydrogen; R⁵ is hydrogen; R⁶ is hydrogen; R⁷ is methyl; R⁸ is hydrogen, 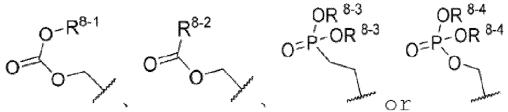
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium.

In one scheme, R¹ is deuterium and R² is -CD₃; R³ is hydrogen; R⁴ is hydrogen; R⁵ is hydrogen; R⁶ is hydrogen; R⁷ is methyl or -CD₃; R⁸ is hydrogen, 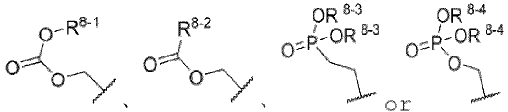
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium, each R⁹ is independently hydrogen or halogen:
   m is 2, 3, 4, or 5.

Better, in one scheme, R¹ is deuterium and R² is -CD₃; R³ is hydrogen; R⁴ is hydrogen; R⁵ is hydrogen; R⁶ is hydrogen; R⁷ is methyl or -CD₃; R⁸ is hydrogen, 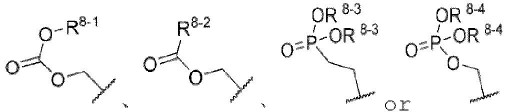
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydroge n, C₁-C₆ alkyl or sodium. R⁹ is fluorine.

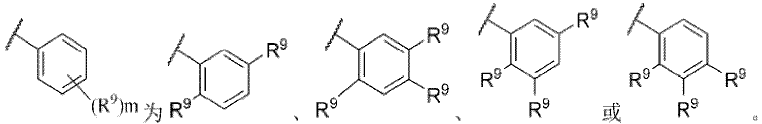

In one scheme, R¹ is hydrogen and R² is methyl; R³ is tritium; R⁴ is tritium; R⁵ is hydrogen; R⁶ is hydrogen; R⁷ is methyl; R⁸ is hydrogen, 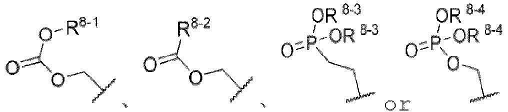 R⁸⁻¹ is C₁-C₆ alkyl, R⁸⁻² is C₁-C₆ alkyl.

Each R⁸⁻³ is independently hydrogen, C₁-C₆ alkyl or sodium, Each R⁸⁻⁴ is independently hydrogen, C₁-C₆ alkyl or sodium, each R⁹ is independently hydrogen or halogen. m is 2, 3, 4, or 5.

In one scheme, R¹ is hydrogen and R² is methyl; R³ is hydrogen; R⁴ is hydrogen; R⁵ is tritium; R⁶ is tritium; R⁷ is methyl; R⁸ is hydrogen, 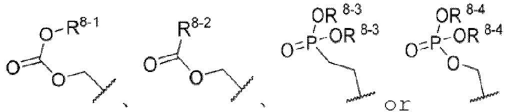
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium.

In one scheme, R¹ is hydrogen and R² is methyl; R³ is hydrogen; R⁴ is hydrogen; R⁵ is tritium; R⁶ is tritium; R⁷ is methyl; R⁸ is hydrogen, 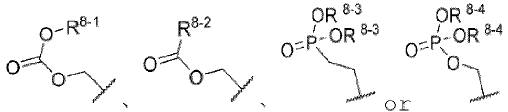
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium, each R⁹ is independently hydrogen or halogen, m is 2, 3, 4, or 5.

In one scheme, R¹ is hydrogen and R² is methyl; R³ is hydrogen; R⁴ is hydrogen; R⁵ is hydrogen; R⁶ is hydrogen; R⁷ is -CD₃; R⁸ is hydrogen, 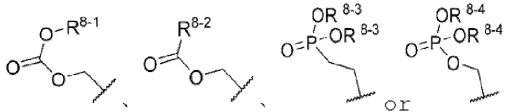
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium.

In one scheme, R¹ is hydrogen and R² is methyl or -CD₃; R³ is hydrogen; R⁴ is hydrogen; R⁵ is hydrogen; R⁶ is hydrogen; R⁷ is -CD₃; R⁸ is hydrogen, 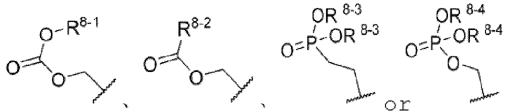
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium, each R⁹ is independently hydrogen or halogen, m is 2, 3, 4, or 5.

In one scheme, R¹ is hydrogen or deuterium, R² is methyl or -CD₃; R³ is hydrogen or deuterium; R⁴ is hydrogen or deuterium; R⁵ is hydrogen or deuterium; R⁶ is hydrogen or deuterium; R⁷ is methyl or -CD₃; R⁸ is 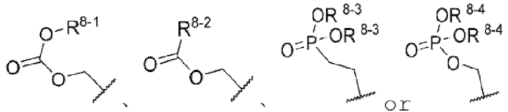
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hydrogen, C₁-C₆ alkyl or sodium, each R⁹ is independently hydrogen or halogen, m is 2, 3, 4, or 5.

Better, in one scheme, R¹ is hydrogen or deuterium, R² is methyl or -CD₃; R³ is hydrogen or deuterium; R⁴ is hydrogen or deuterium; R⁵ is hydrogen or deuterium; R⁶ is hydrogen or deuterium; R⁷ is methyl or -CD₃; R⁸ is 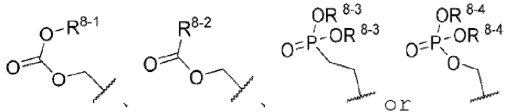
R⁸⁻¹ is C₁-C₆ alkyl, R⁸⁻² is C₁-C₆ alkyl.
Each R⁸⁻³ is independently hydrogen, C₁-C₆ alkyl or sodium, Each R⁸⁻⁴ is independently hy drogen, C₁-C₆ alkyl or sodium. R⁹ is fluorine,

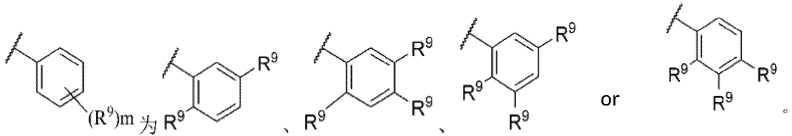

In a scheme, the triazine derivative shown in the formula (I') is any of the following compounds: 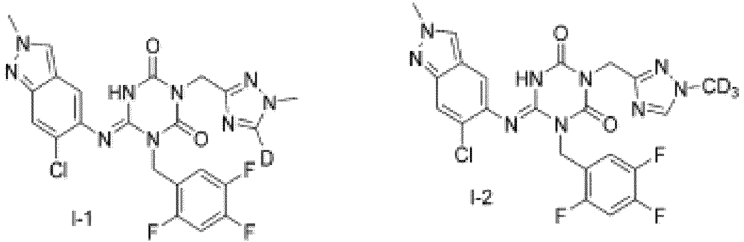 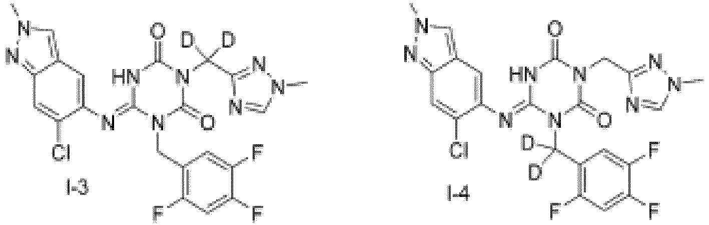 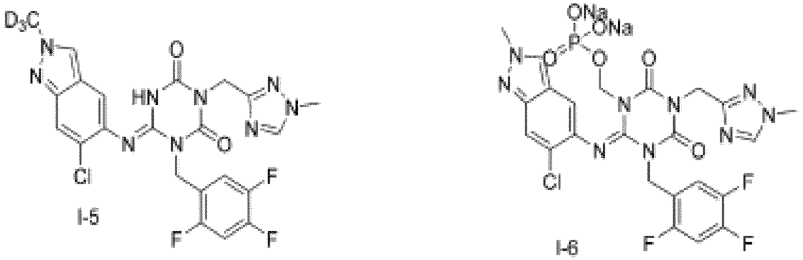 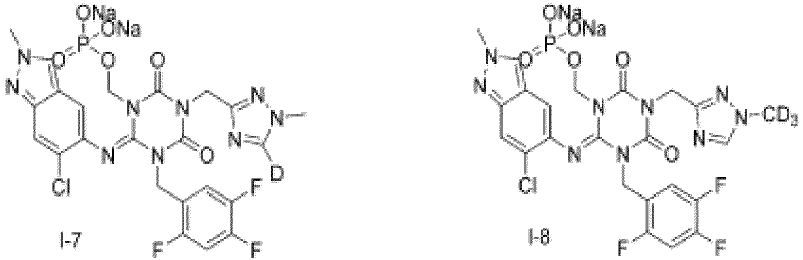 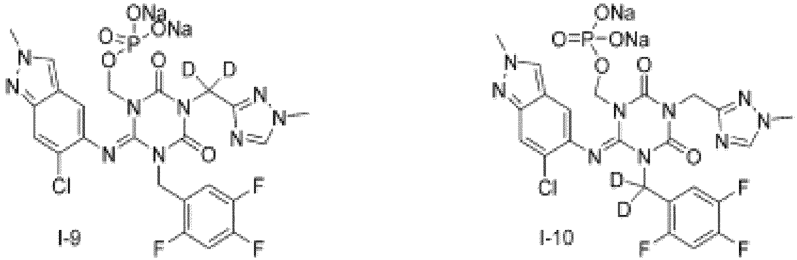 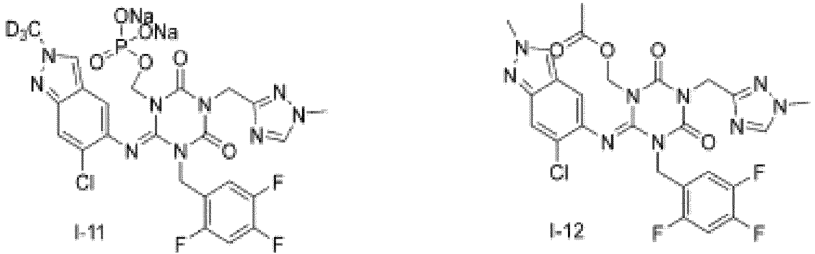 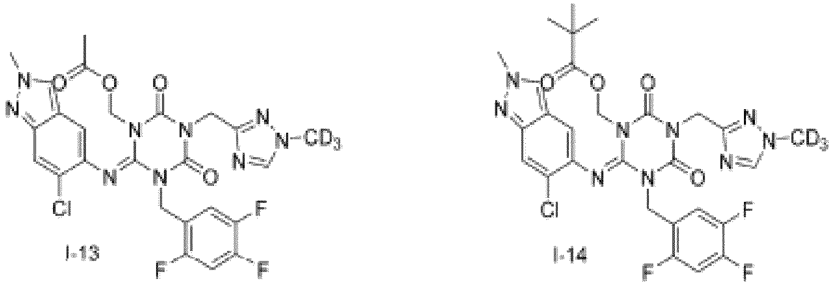 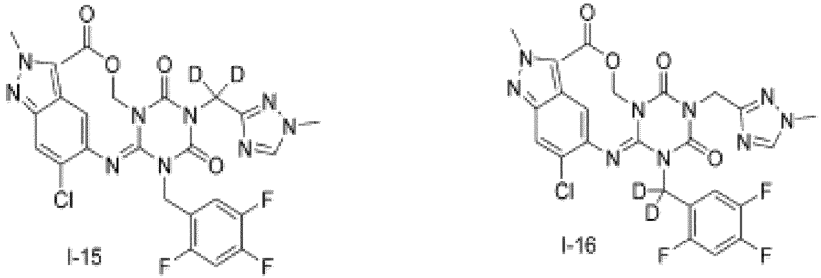 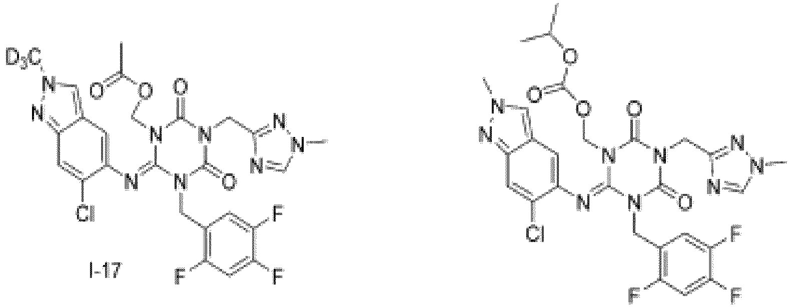 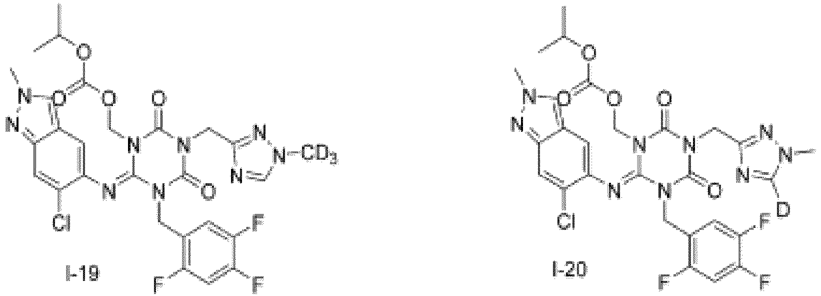 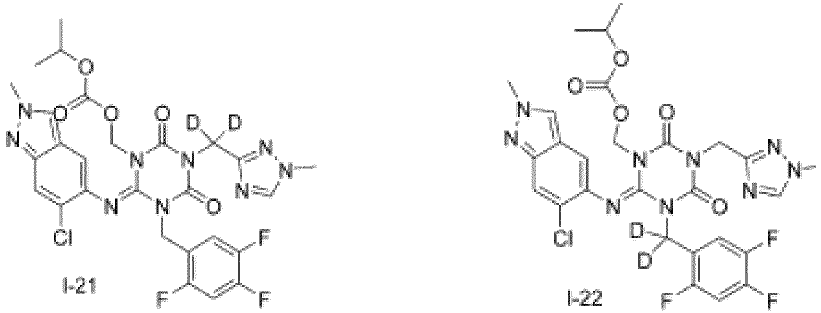 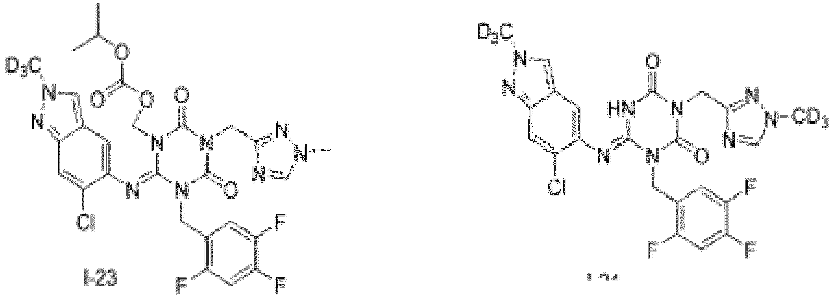 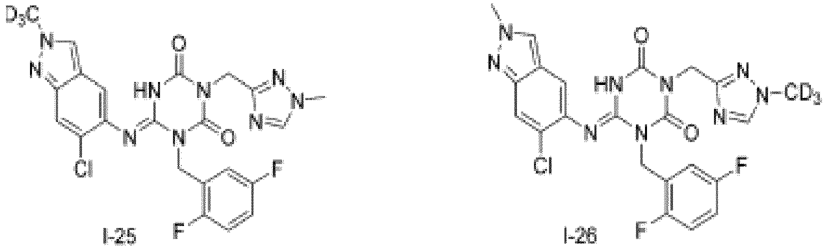 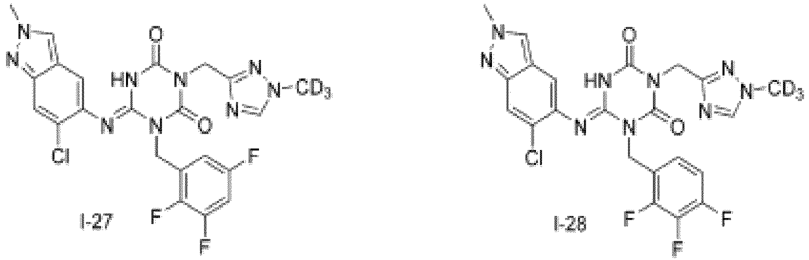 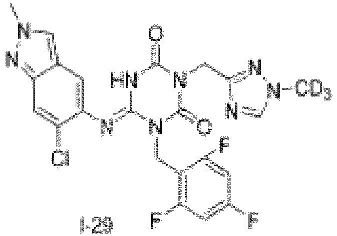 or

The invention also provides a triazine derivative or a pharmacologically acceptable salt as shown in formula (III). 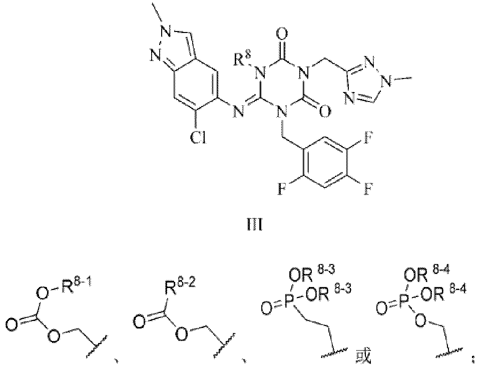
R⁸ is hydrogen,
R⁸⁻¹ is C₁-C₁₀ alkyl,
R⁸⁻² is C₁-C₁₀ alkyl,
Each R⁸⁻³ is independently hydrogen, C₁-C₁₀ alkyl, or sodium,
Each R⁸⁻⁴ is independently hydrogen, C₁-C₁₀ alkyl, or sodium, formula (III) is not

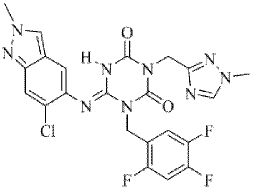

In one scheme, certain substituents in a deuterotriazine derivative shown in formula (III) or in a pharmaceutically acceptable salt thereof may be further defined as follows, and substituents not covered below are defined as described in any scheme of the invention (hereinafter referred to as "in one scheme"). In R⁸⁻¹, the C₁~C₁₀ alkyl may be C₁~C₆ alkyl, preferably methyl, ethyl, n-propyl, isopropyl, n-butyl, isobutyl, sec-butyl or tert-butyl, such as methyl, ethyl or isopropyl.

In one scheme, R⁸⁻², the C₁~C₁₀ alkyl group may be C₁~C₆ alkyl groups, preferably methyl, ethyl, n-propyl, isopropyl, n-butyl, isobutyl, sec-butyl or tert-butyl groups, such as methyl, ethyl, isopropyl, n-butyl or tert-butyl groups.

In a scheme, R⁸⁻³, the C₁~C₁₀ alkyl group may be C₁~C₆ alkyl group, preferably methyl, ethyl, n-propyl, isopropyl, n-butyl, isobutyl, sec-butyl, or tert-butyl.

In a scheme, R⁸⁻⁴, the C₁~C₁₀ alkyl group may be C₁~C₆ alkyl group, preferably methyl, ethyl, n-propyl, isopropyl, n-butyl, isobutyl, sec-butyl, or tert-butyl.

In one scheme, R⁸⁻¹ is C₁~C₆ alkyl.

In one scheme, R⁸⁻² is C₁~C₆ alkyl.

In one scenario, R⁸⁻³ is sodium.

In one scenario, R⁸⁻⁴ is sodium.

In one scheme, when R⁸ is hydrogen, the triazine derivative shown in the formula (III) may also be its tautomer, e.g.

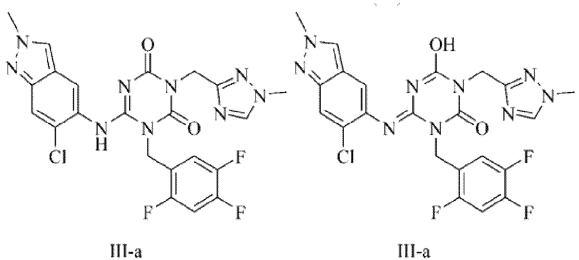

In one scheme, R⁸ is hydrogen,

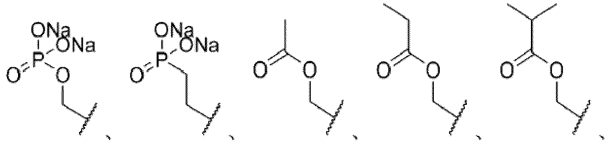

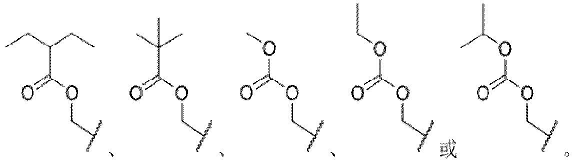

The invention also provides a triazine derivative shown in formula VI or a pharmaceutically acceptable salt thereof.

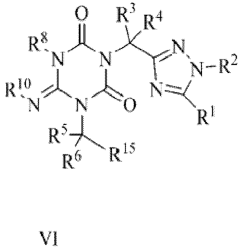

Where R¹ is hydrogen or deuterium.

R² is a methyl group or a methyl group replaced by 1, 2 or 3 deuterium.

R³ for hydrogen or deuterium.

R⁴ is hydrogen or deuterium.

R⁵ for hydrogen or deuterium.

R⁶ is hydrogen or deuterium.

R⁸ is hydrogen,

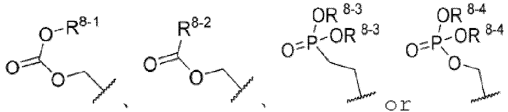

R⁸⁻¹ is C₁~C₁₀ alkyl.

R⁸⁻² is C₁~C₁₀ alkyl.

Each R⁸⁻³ is independently hydrogen, C₁-C₁₀ alkyl, or sodium.

Each R⁸⁻⁴ is independently hydrogen, C₁~C₁₀ alkyl, or sodium.

R¹⁰ is a 6-10 meta heteroaryl group that is not replaced or is replaced by one or more R¹⁰⁻¹; The type of heteroatom of the 6-10 meta heteroaryl group is selected from one or more of N, O and S, and the number of heteroatom is 1, 2 or 3;

Each R¹⁰⁻¹ is independently a C₁-C₆ alkyl, halogen, or deuterium that is not replaced or is replaced by one or more R¹⁰⁻¹⁻¹.

Each R¹⁰⁻¹⁻¹ is independently a halogen or deuterium. R15 is

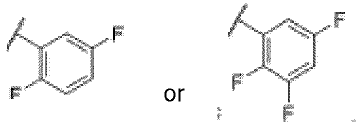

In a scheme, certain substituents in the triazine derivatives or pharmaceutically acceptable salts thereof shown in formula VI may be further defined as follows, and substituents not covered below are defined as described in any scheme of the invention (hereinafter referred to as "in one scheme");

In R⁸⁻², the C₁-C₁₀ alkyl may be C₁-C₆ alkyl, preferably methyl, ethyl, n-propyl, isopropyl, n-butyl, isobutyl, sec-butyl, or tert-butyl, such as methyl, ethyl, isopropyl, n-butyl, or tert-butyl.

In a certain scheme, R⁸⁻³, the C₁~C₁₀ alkyl group may be C₁~C₆.Alkyl, preferably methyl, ethyl, n-propyl, isopropyl, n-butyl, isobutyl, sec-butyl, or tert-butyl.

In a scheme, R⁸⁻⁴, the C₁~C₁₀ alkyl group may be C₁-C₆ alkyl group, preferably methyl, ethyl, n-propyl, isopropyl, n-butyl, isobutyl, sec-butyl, or tert-butyl.

In a scheme, R¹⁰, the 6-10 meta heteroaryl group may be 5 and 6 meta heteroaryl group, and/or the 6-10 meta heteroaryl group may have a heteroatom class of N and/or O, and/or the 6-10 meta heteroaryl group may have a heteroatom number of 2; The 6-10 meta heteraryl group is preferably

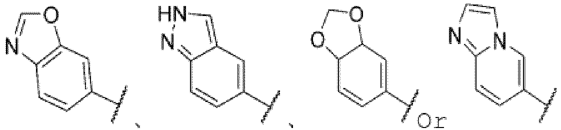

In one scheme, R¹⁰⁻¹, the C₁-C₆ alkyl group may be methyl, ethyl, n-propyl, isopropyl, n-butyl, isobutyl, sec-butyl, or tert-butyl, such as methyl.

In one scheme, R¹⁰⁻¹, the halogen may be fluorine, chlorine, bromine or iodine, such as fluorine or chlorine.

In one scheme, R¹⁰⁻¹⁻¹, the halogen may be a atmosphere, chlorine, bromine or iodine, such as fluorine or chlorine.

In one scheme, R¹ is hydrogen.

In one scheme, R² is methyl or -CD3.

In one scenario, R³ is hydrogen.

In one scenario, R⁴ is hydrogen.

In one scenario, R⁵ is hydrogen.

In one scenario, R⁶ is hydrogen.

In a scheme, each R¹⁰⁻¹ is independently chlorine, methyl, fluorine, or -CD₃ ;

In a scheme, R10, the 6-10 meta heteroaryl group is 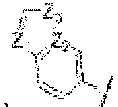 Z1 is N or O; Z2 is N or C; Z3 is C or O; In a better way, R¹⁰ is

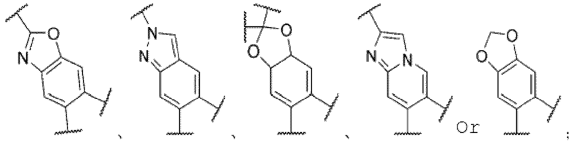

Another example

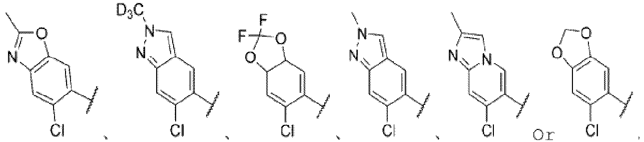

The invention also provides a preparation method of a triazine derivative as shown in formula (I') above, which is method 1, method 2, method 3, method 4 or method 5,
The method 1 comprises the following steps where, in a solvent (e.g., anhydrous tetrahydrofuran), in the presence of a base (e.g., hexamethyldisilylamine lithium), a compound shown in formula I'-S1 reacts with heavy water as shown below to obtain a compound shown in formula I'.

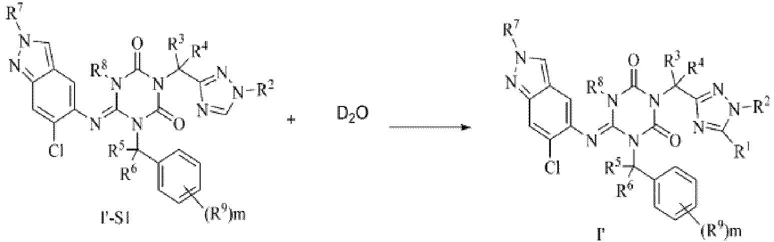

Where R¹ is deuterium; R², R³, R⁴, R⁵, R⁶, R⁷ and R⁸ are defined as described before.

Method 2 consists of the following steps: In a solvent (e.g., a mixed solution of acetic acid and tert-butanol), a compound shown in formula I'-S2 reacts with a compound shown in formula I'-S3 as shown below to obtain a compound shown in formula I'.

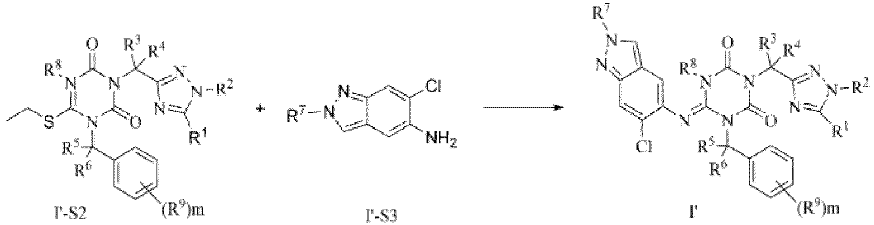

Where R¹, R², R³, R⁴, R⁵, R⁶, R⁷ and R⁸ are defined as described above;

Method 3 consists of the following steps in which, in a solvent (e.g., N, N-dimethylformamide or dimethylacetamide) and in the presence of a base (e.g., anhydrous cesium carbonate or potassium 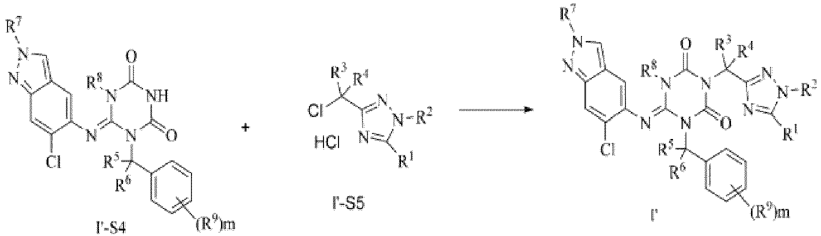 carbonate), a compound shown in formula I'-S4 reacts with a compound shown in formula I'-S5 as shown below to obtain a compound shown in formula I'

Where R¹, R², R³, R⁴, R⁵, R⁶, R⁷ and R⁸ are defined as described above.

The method 4 consists of the following steps, in a solvent (e.g., dichloromethane), a compound shown in formula I'-S6 reacts with sodium hydroxide (e.g., an ethanol solution of sodium hydroxide) as shown below to obtain a compound shown in formula I'

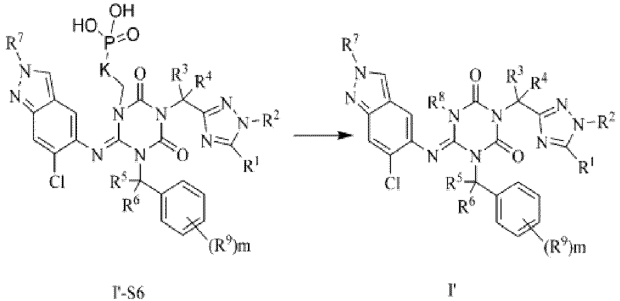

Where K is or CH₂, R⁸ is

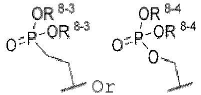

Each R⁸⁻³ and each R⁸⁻⁴ are sodium, Where R¹, R², R³, R⁴, R⁵, R⁶, R⁷ are defined as described above.

Method 5 consists of the following steps: In a solvent (e.g., N, N-dimethylformamide or dimethylacetamide), in the presence of a base (e.g., sodium hydride), a compound shown in formula I'-S7 reacts with a compound shown in formula I'-S8 as shown below to obtain a compound shown in formula I'.

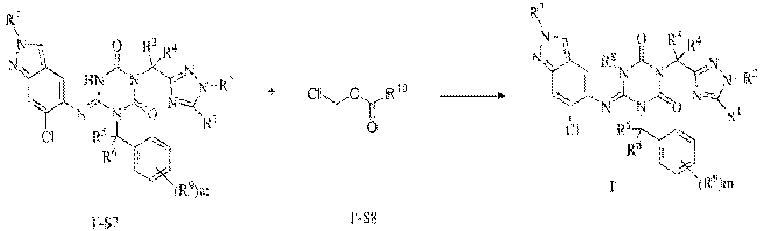

Where R¹⁰ is C₁~C₁₀ alkyl or-OC₁~C₁₀ alkyl, R⁸ is

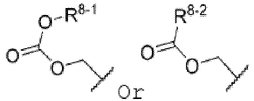

Each R⁸⁻¹ and each R⁸⁻² are independently C₁~C₁₀ alkyl. R¹, R², R³, R⁴, R⁵, R⁶, R⁷ are defined as described above.

Methods 1 to 5 may be conventional methods in this field, and the preparation conditions and operations may be conventional conditions and operations for this type of reaction in this field.

The invention also provides a binary co-product formed by a triazine derivative and an acid as shown in formula (I), (III) or (VI) above; The acids are malic acid, maleic acid, citric acid, ascorbic acid, mandelic acid, tartaric acid, fumaric acid, preferably fumaric acid.

The invention also provides a binary eutectic of a triazine derivative formed with an acid as shown in formula (I'), (D), (III) or (VI) above; The acids are acetic acid, malic acid, maleic acid, citric acid, ascorbic acid, mandelic acid, tartaric acid, fumaric acid or acetic acid, preferably acetic acid or fumaric acid.

In a scheme, the binary co-goods are 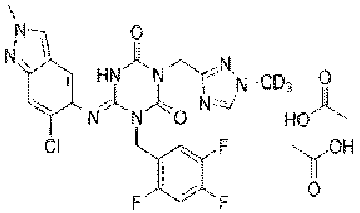 or

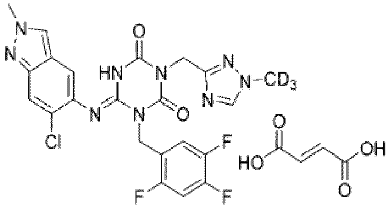

In a scheme, the binary co-goods are

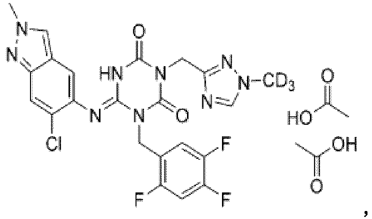

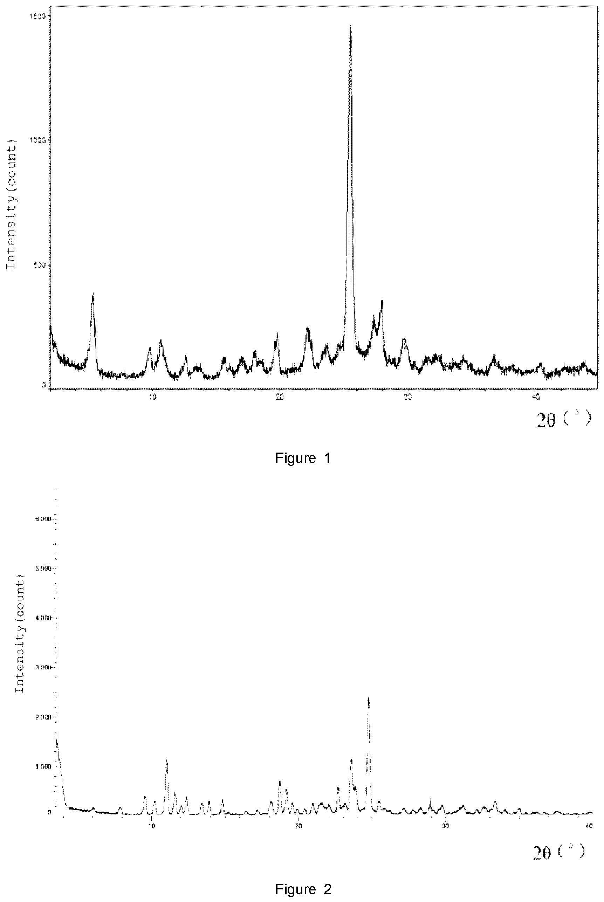

Its X-ray powder diffraction pattern represented by 2Θ angles has diffraction peaks at 5.4±0.2, 19.8±0.2,22.2±0.2 and 25.5±0.2. Preferably, the X-ray powder diffraction pattern expressed at 2Θ angles also has diffraction peaks at one or more of 9.9±0.2,10.7±0.2,12.6±0.2,27.3±0.2, and 28.0±0.2. Preferably, its X-ray powder diffraction pattern expressed at 2Θ angles is shown in Figure 1.

In a scheme, the binary co-goods are

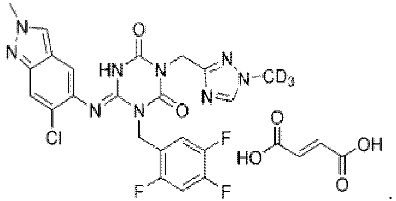

Its X-ray powder diffraction pattern represented by 2Θ angles has diffraction peaks at 7.85± 0.2° , 9.55±0.2° ,10.22±0.2° , 12.01 ±0.2° ,13.88±0.2° , 14.79±0.2° ,17.19±0.2° ,18.71 ± 0.2° , 19.16±0.2° ,23.60±0.2° , 23.85±0.2° ,24.76±0.2° , and 28.95±0.2° X-ray powder diffraction pattern represented by 2Θ angles also has diffraction peaks at one or more places of 6.05 ±0.2° ,11.02±0.2° ,11.60±0.2° ,12.39±0.2° ,13.43±0.2° , 15.21 ±0.2° , 16.41 ±0.2° , 18.13 ±0.2° ,19.59±0.2° , 19.91 ±0.2° , 20.43±0.2° , 20.97±0.2° ,21.53±0.2° , 22.04±0.2° , 22.70±0.2° ,23.16±0.2° ,25.47±0.2° ,27.13±0.2° , 27.77±0.2° , 28.28 ±0.2° , 29.76± 0.2° ,31.17±0.2° , 32.11 ±0.2° ,32.64±0.2° ,33.34±0.2° ,34.03±0.2° , and 35.00±0.2° ; Preferably, its X-ray powder diffraction pattern expressed at 2Θ angles is shown in Figure 2.

The invention also provides a method for preparing a binary coproduct of a triazine derivative with an acid as described in formula (I'), (I), (III) or (VI), which includes the following steps to react a triazine derivative as described in formula (I) with an acid. A binary coproduct of a triazine derivative and an acid shown in the formula (I'), (I), (III), or (VI) is obtained: the acid is defined as described above.

In a scheme, when the acid is fumaric acid, the preparation method of the triazine derivative shown in formula (I'), (I), (III) or (VI) as a binary coproduct of the acid with the triazine derivative shown in formula (I), (I), (III) or (VI) may include the following steps, in a solvent, to react with fumaric acid, as described above.The triazine derivative shown in formula (I'), (I), (III) or (VI) is obtained as a binary co-product with fumaric acid.

In a scheme, the solvent may be ethyl acetate.

The preparation method of binary coproducts of triazine derivatives and acids shown in the formula (I'), (I), (III) or (VI) may be the conventional method in the field, and the preparation conditions and operations may be the conventional conditions and operations of such reactions in the field.

The invention also provides a triazine derivative shown in formula (I), (III) or (VI) to form a ternary coproduct with nicotinamide and acid: the acids are malic acid, maleic acid, citric acid, ascorbic acid, mandelic acid, tartaric acid, fumaric acid, preferably fumaric acid.

The invention also provides a ternary eutectic formed by triazine derivatives and niacinamide and acid as shown in formula (I'), (I), (III) or (VI) above. The acids are malic acid, maleic acid, citric acid, ascorbic acid, mandelic acid, tartaric acid, fumaric acid or acetic acid, preferably fumaric acid.

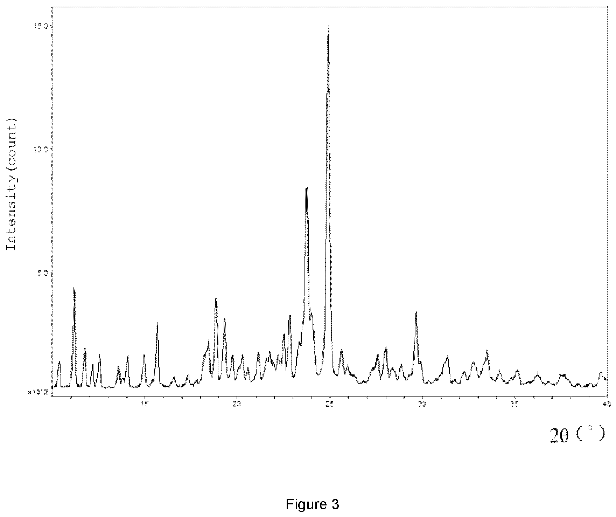

In one scheme, the ternary eutectic is, 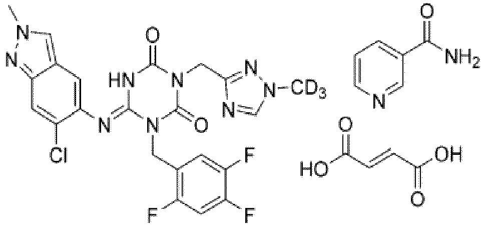 its X-ray powder diffraction pattern represented by 2Θ angles has diffraction peaks at 10.406, 11.188, 11.772, 12.202, 12.556, 13.589, 14.075, 14.973, 15.692, 17.37, 18.212, 18.464, 18.874, 19.34, 19.752, 20.104, 20.28, 20.59, 21.154, 21.743, 22.246, 22.536, 22.868, 23.342, 23.767, 24.003, 24.92, 25.641, 25.99, 27.589, 28.038, 28.859, 29.677, 29.911, 31.356, 32.779, 33.502, 35.14, 36.251 and 39.648±0.2. Preferably, its X-ray powder diffraction pattern expressed at 2Θ angles is shown in Figure 3.

In a scheme, the ternary eutectic is, 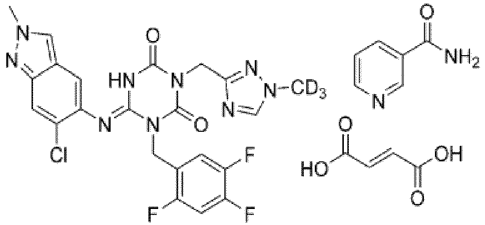 its X-ray powder diffraction pattern represented by 2Θ angles has diffraction peaks at 11.2±0.2° , 15.7±0.2° ,18.9±0.2° ,19.3±0.2° ,22.9±0.2° , 24.0±0.2° ,24.9±0.2° and 29.6±0.2° ; Preferably, It also has diffraction peaks at one or more places of 10.4±0.2° , 11.8±0.2° , 12.2± 0.2° ,12.6±0.2° ,13.6±0.2° , 14.1 ±0.2° , 15.0±0.2° ,17.4±0.2° ,18.2±0.2° ,18.5±0.2° , 19.8±0.2° , 20.1 ±0.2° , 20.3±0.2° ,20.6±0.2° ,21.2±0.2° ,21.7±0.2° ,22.2±0.2° , 22.5± 0.2° , 23.3±0.2° , 23.8±0.2° , 25.6±0.2° , 26.0±0.2° ,27.6±0.2° , 28.0±0.2° ,28.9±0.2° , 29.7±0.2° ,29.9±0.2° , 31.4±0.2° ,32.8±0.2° ,33.5±0.2° , 35.1 ±0.2° , 36.3±0.2° and 39.6 ±0.2° . Preferably, its X-ray powder diffraction pattern expressed at 2Θ angles is shown in Figure 3.

The invention also provides a method for preparing ternary coproducts of triazine derivatives shown in formula (I'), (I), (III) or (VI) with niacinamide and acid as described above, which includes the following steps to react triazine derivatives, niacinamide and acid as described in formula (I').To obtain a ternary coproduct of the triazine derivatives shown in the formula (I'), (I), (III) or (VI) with niacinamide and acid; The acid is defined as described above.

In a scheme, the preparation method of the triazine derivative shown in formula (I'), (I), (II) or (VI) with the ternary eutectic of niacinamide and acid may consist of the following steps, reaction in a solvent of the triazine derivative, niacinamide and fumaric acid shown in formula (I), (I), (III) or (VI), as described above. The triazine derivatives shown in formula (I'), (I), (III) or (VI) are obtained as ternary coproducts with niacinamide and acid.

In a scheme, the solvent can be ethyl acetate.

The triazine derivatives shown in formula (I'), (I), (III) or (VI) to form ternary coproducts with niacinamide and acid may be conventional methods in this field, and the preparation conditions and operations may be conventional conditions and operations for such reactions in this field.

The invention also provides a pharmaceutical composition comprising substance B and one or more pharmaceutically acceptable carriers; The substance B is a triazine derivative or a pharmaceutically acceptable salt thereof as indicated in formula (I), (III) or VI, or a binary eutectic or ternary eutectic as indicated previously. In the pharmaceutical composition, the amount of substance B may be a therapeutic effective amount.

The invention also provides a pharmaceutical composition comprising substance B' and one or more pharmaceutically acceptable carriers: the substance B' is a triazine derivative or a pharmaceutically acceptable salt thereof as indicated in formula (I'), (I), (III) or (VI), or a binary or ternary eutectic as described above. In the pharmaceutical composition, the amount of the substance B' may be a therapeutic effective amount.

Pharmaceutical compositions of the present invention may be prepared according to the disclosed contents using any method known to a person skilled in the art. For example, conventional mixing, dissolution, granulation, emulsification, grinding, encapsulation, embedding or freeze-drying processes.

The invention also provides a use of substance B in the preparation of antiviral drugs such as a triazine derivative or a pharmaceutically acceptable salt thereof as indicated in formula (I), (III) or VI above, or a binary or ternary co-product as described above; The viruses are coronaviruses, influenza viruses, respiratory syncytial viruses, flaviviridae viruses, filoviridae viruses, or porcine Epidemic diarrhea virus (PEDV).

The invention also provides a use of substance B' in the preparation of antiviral drugs, such as a triazine derivative or a pharmaceutically acceptable salt thereof as described in formula (I'), (I), (III) or (VI), such as a binary or ternary co-product or a pharmaceutical composition as described above; The viruses are coronaviruses, influenza viruses, respiratory syncytial viruses, flaviviridae viruses, filoviridae viruses, or porcine Epidemic diarrhea virus (PEDV).

Preferably, the coronavirus is selected from one or more of MERS-CoV, SARS-CoV, and SARS-CoV-2, and the coronavirus is preferred as SARS-CoV-2.

The invention also provides an application of substance B in the preparation of drugs for the treatment and/or prevention of coronavirus-related diseases, in the form of triazine derivatives or pharmaceutically acceptable salts thereof as indicated in formula (I), (III) or VI above, or in the form of binary or ternary co-products as described above.

The invention also provides an application of substance B' in the preparation of drugs for the treatment and/or prevention of coronavirus-related diseases, in the form of triazine derivatives or pharmaceutically acceptable salts thereof as described in formula (I'), (I), (III) or (VI), as described previously as binary or ternary co-products or as described previously as pharmaceutical compositions.

The invention also provides a pharmaceutical composition comprising substance A and one or more pharmaceutically acceptable carriers; Substance A is a triazine derivative or a pharmaceutically acceptable salt thereof as indicated in formula (I) or (III) above. In the pharmaceutical composition, the amount of the triazine derivative shown in formula (I) or (III), or the pharmaceutically acceptable salt thereof, may be a therapeutic effective amount.

The invention also provides A pharmaceutical composition comprising a substance A 'and one or more pharmaceutically acceptable carriers; The substance A' is a triazine derivative or a pharmaceutically acceptable salt thereof as indicated in formula (I'), (I), (III) or (VI) above. In the pharmaceutical composition, the amount of the triazine derivative shown in formula (I'), (I), (III) or (VI), or the pharmaceutically acceptable salt thereof, may be a therapeutic effective amount.

The pharmaceutically acceptable carriers (pharmaceutical excipients) may be those excipients widely used in the field of pharmaceutical production. Excipients are primarily intended to provide a safe, stable, and functional pharmaceutical composition, and may also provide a method for dissolution of the active ingredient at the desired rate after the subject receives the administration of the composition, or to facilitate the effective absorption of the active ingredient after the subject receives the administration of the composition. The pharmaceutical excipients may be inert fillers or provide a function, such as stabilizing the overall pH of the composition or preventing degradation of the active component of the composition. The pharmaceutical excipients may include one or more of the following excipients. Adhesives, suspension AIDS, emulsifiers, thinners, fillers, granulants, adhesives, disintegrants, lubricants, anti-adhesive agents, flow AIDS, wetting agents, gelling agents, absorption delay agents, dissolution inhibitors, enhancers, absorbents, buffers, chelators, preservatives, colorants, taste correction agents and sweeteners.

Pharmaceutical compositions of the present invention may be prepared according to the disclosed contents using any method known to a person skilled in the art. For example, conventional mixing, dissolution, granulation, emulsification, grinding, encapsulation, embedding or freeze-drying processes.

The invention also provides a substance A in the preparation of antiviral drugs, which is a triazine derivative or a pharmaceutically acceptable salt thereof as indicated in formula (I) or (III), or a composition as described above; The viruses are coronaviruses, influenza viruses, respiratory syncytial viruses, flaviviridae viruses, filoviridae viruses, or porcine Epidemic diarrhea virus (PEDV).

The invention also provides a substance A' in the preparation of an antiviral drug, which is a triazine derivative or a pharmaceutically acceptable salt thereof as indicated in formula (I'), (I), (III) or (VI), or a composition as described above. The viruses are coronaviruses, influenza viruses, respiratory syncytial viruses, flaviviridae viruses, filoviridae viruses, or porcine Epidemic diarrhea virus (PEDV).

Preferably, the coronavirus is selected from one or more of MERS-CoV, SARS-CoV, and SARS-CoV-2, and the coronavirus is preferred as SARS-CoV-2.

The invention also provides an application of substance A in the preparation of drugs for the treatment and/or prevention of coronavirus-related diseases, in the form of A triazine derivative or a pharmaceutically acceptable salt thereof as indicated in formula (I') or (III), or a composition as described above.

The invention also provides the application of a substance A' in the preparation of drugs for the treatment and/or prevention of coronavirus-related diseases in the form of a triazine derivative or a pharmaceutically acceptable salt thereof as indicated in formula (I'), (I), (III) or (VI), or a composition as described above.

The coronavirus-associated disease can be MERS, SARS, or COVID-19, preferably COVID-19.

Unless otherwise stated, the following terms appearing in the specification and claims of the invention have the following meanings:
The term "pharmaceutically acceptable" means that salts, solvents, excipients, etc., are generally non-toxic, safe, and suitable for use by patients. The "patient" is preferably a mammal, and more preferably a human.

The term "pharmaceutically acceptable salt" means a salt prepared from the compound of the invention with a relatively non-toxic, pharmaceutically acceptable acid or base. When the compounds of the invention contain relatively acidic functional groups, alkali addition salts can be obtained by contacting the prototype of such compounds with a sufficient amount of pharmaceutically acceptable alkali in a suitable inert solvent. Pharmaceutically acceptable alkali addition salts include but are not limited to; Lithium salt, sodium salt, potassium salt, calcium salt, aluminum salt, magnesium salt, zinc salt, bismuth salt, ammonium salt, diethanolamine salt. When the compounds of the invention contain relatively basic functional groups, acid addition salts can be obtained by contacting the prototype of such compounds with enough pharmaceutically acceptable acid in a suitable inert solvent. The pharmaceutically acceptable acids include inorganic acids, which include but are not limited to: Hydrochloric acid, hydrobromic acid, hydroiodic acid, nitric acid, carbonic acid, phosphoric acid, phosphorous acid, sulfuric acid, etc., said pharmaceutically acceptable acids include organic acids, said organic acids include but are not limited to: Acetic acid, propionic acid, oxalic acid, isobutyric acid, maleic acid, benzoic acid, succinic acid, octoic acid, fumaric acid, lactic acid, mandelic acid, phthalic acid, benzenesulfonic acid, p-toluenesulfonic acid, citric acid, salicylic acid, tartaric acid, mesylate, isonicotinic acid, acid citric acid, oleic acid, tannic acid, pantothenic acid, hydrogen tartrate, ascorbic acid, gentian acid, fumaric acid, gluconic acid, Saccharic acid, formic acid, ethanesulfonic acid, dihydroxynaphthoic acid (i.e. 4,4 "-methylene - bis (3-hydroxy-2-naphthoic acid)), amino acids (e.g., glutamic acid, arginine), etc.When the compound of the invention contains relatively acidic and relatively basic functional groups, it can be converted into an alkali addition salt or an acid addition salt. See Berge et al for details., "Pharmaceutical Salts", Journal of Pharmaceutical Science 66:1-19 (1977), or, faced of Pharmaceutical Salts: Properties, Selection, and Use (PH einrich Stahl and Camille G.Wermuth,cd.,Wiley-VCH,2002).

An alkyl group means an alkyl group having a total of 1, 2, 3, 4, 5 or 6 carbon atoms as defined below. The total number of carbon atoms in the simplified symbol does not include the carbon that may be present in the substituent of the group.

The term "therapy" refers to therapeutic therapy. When it comes to specific conditions, treatment means:(1) alleviates one or more biological manifestations of a disease or condition, (2) interferes with (a) one or more points in the biological cascade that causes or causes the condition, or (b) one or more biological manifestations of the condition, (3) ameliorates one or more symptoms, effects or side effects associated with the condition, or one or more symptoms, effects, or side effects associated with the condition or its treatment, or (4) slowing down the development of the condition or one or more biological manifestations of the condition.

The term "therapeutic effective amount" means the amount of a compound that, when administered to a patient, is sufficient to effectively treat the disease or condition described herein. The "therapeutic effective amount" will vary according to the compound, the condition and its severity, and the age of the patient to be treated, and can be adjusted according to the needs of those skilled in the field.

On the basis of not violating the common sense in the field, the above optimal conditions can be arbitrarily combined to obtain the best examples of the invention.

The reagents and raw materials used in the invention are commercially available.

The positive improvement effect of the invention is that the compound of the invention can significantly prolong the half-life, reduce the demand for dosage, reduce side effects, and expand the range of treatment window on the basis of retaining the effectiveness of SARS-CoV-2. Therefore, the invention has a very good prospect for making drugs for treating diseases related to coronavirus infection.

### Illustrated description

- Figure 1:: Powder diffraction pattern of SHEN210 (coacetate of compound 1-2).
- Figure 2:: Powder diffraction pattern of SHEN211 (fumaric acid eutectic of compound 1-2).
- Figure 3:: Powder diffraction pattern of SHEN212 (ternary eutectic of I-2).

### Concrete implementation mode

The invention is further described by means of embodiments below, but does not therefore limit the invention to the scope of the embodiments. Experimental methods not specified in the following embodiments shall be selected in accordance with the conventional methods and conditions, or in accordance with the product specification.

### Embodiment 1 Synthesis of deuterated compound 1-1

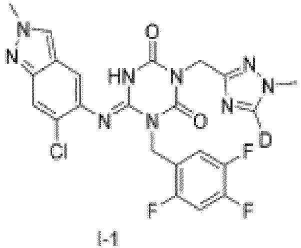

### Synthesis of intermediate 1-03

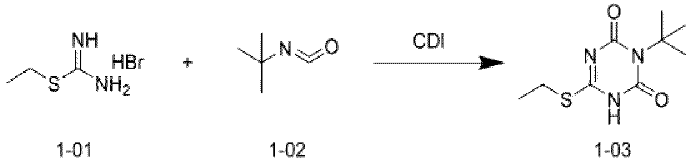

Tert-butyl isocyanate (1.2mL, 10.5mmol) and DBU (1.9mL, 12.8mmol) were added to the mixture of S-ethylthiurea hydrobromide (1.85g, 10mmol) and DMF (9.3mL) under ice bath cooling and stirred for 6 hours.1,1 '-carbonyl diimidazole (1.95g, 12mmol) and DBU (1.9mL, 12.8mmol) were added to the reaction solution under an ice bath and stirred for 2 hours. Then 2mol/L hydrochloric acid (80mL) was slowly added, solid matter was precipitated, filtered, the solid was dissolved in ethyl acetate, dried with anhydrous magnesium sulfate, concentrated under reduced pressure, and purified by column chromatography. 1-03 was obtained with a yield of about 50%.

### Synthesis of intermediate 1-6

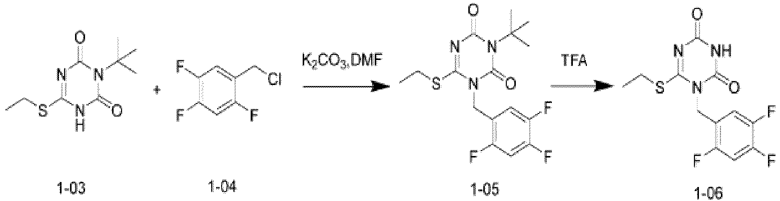

Add potassium carbonate (17.97g, 130 mmol) into acetonitrile (200mL) of 1-03 (22.93g, 100 mmol), 1-04 (22.60g, 110 mmol) and stir for 3 hours under heating reflux. Filter the reaction liquid, concentrate and reduce the pressure to obtain the rent product of 3-tert-butyl-1- (2,4, 5-trifluorobenzyl) -6- (ethylthioyl) -1,3,5-triazine-2, 4-dione (1-05) as a slightly brown oil.

Trichloroacetic acid (100mL) is added to the resulting rental product under cold temperature and stirred at room temperature for 17 hours. The reaction liquid is concentrated under reduced pressure to obtain 1-06 (2-step yield of about 85%), ¹H-NMR (400 MHz, CDC13): δ 7.04-6.97 (m, 2H), 5.15 (s, 2H), 3.25-3.20 (q, 2H), 1.38-1.35 (t, 3H). LC-MS (ESI, m/z) 318 [M+H]⁺.

### Synthesis of intermediate 1-08

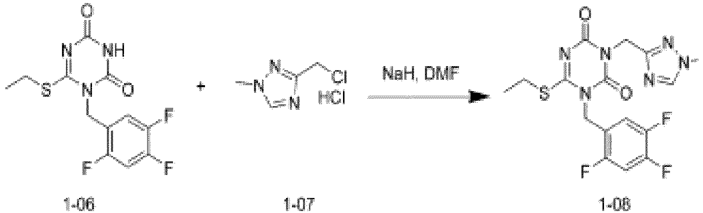

Dissolve 1-06 (2.50g, 7.88mmol) and 1-07 (1.99g, 11.8mmol) in DMF (23mL) and add anhydrous potassium carbonate (3.27g).23.6mmol), rising to 60° C, stirring reaction for 4h, cooling to room temperature, the reaction liquid was poured into ammonium chloride aqueous solution, solid precipitation, filtration, water washing, solid recrystallization with ethanol to 1-08, the yield of about 50%, LC-MS (ESI) : 413 [M+H]⁺.

### Synthesis of intermediates 1-10

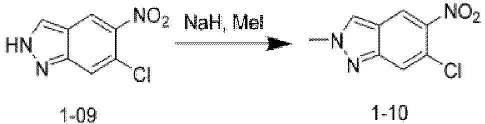

Add carbonic acid (562mg, 4.07mmol) to DMF (10mL) solution of 5-nitro-6-chloro-1h-indazole (334mg, 1.35mmol), stir at room temperature for 1h, then slowly add iodomethanes (0.17mL, 2.71mmol), then stir for 2h.The reaction mixture was diluted with methylene chloride, washed in saturated salt water, and dried with anhydrous sodium sulfate. Intermediates 1-10A and 1-10B purified by silica gel column chromatography, LC-MS (ESI) : 214 [M+H]⁺,

### Synthesis of intermediates 1-11

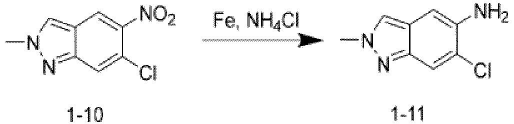

Dissolve 1-10 (0.39g, 1.5mmol) in ethanol (15mL), add iron powder (0.42g, 7.5mL) and 0.4mL hydrochloric acid, rise temperature for reflux reaction for 1h, cool to room temperature, dilute the reaction solution with ethanol, filter with diatomaceous earth, concentrate, dissolve the residue with ethyl acetate, wash with sodium bicarbonate solution, water and salt water. Anhydrous sodium sulfate drying. Concentrated under vacuum and purified by silica gel column chromatography (PE/EA = 4/1), 1-11, LC-MS(ESD):182 [M+H]⁺.

### Synthesis of intermediate 1-12 (i.e., control compound S-217622)

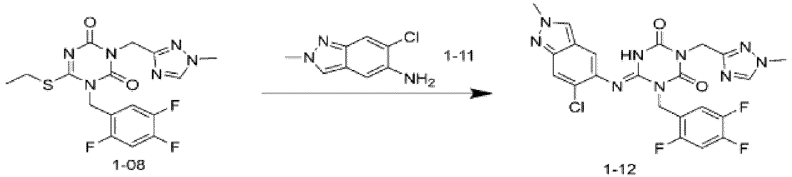

Dissolve 1-08(5.0g, 12.6mmol), 1-11 (2.06g, 18.9mmol) in the mixture of acetic acid (11.32g, 189mmol) and tert-butanol (100mL), stir at reflux at high temperature for 3 hours, cool to room temperature, and add the reaction solution to saturated sodium bicarbonate aqueous solution (500mL).It was extracted with ethyl acetate (500mL), combined with organic phase, washed, dried with anhydrous sodium sulfate, filtered, concentrated under reduced pressure, and the residue was separated by silica gel column chromatography (gradient elelation with dichloromethane and methanol, 0-20% MeOH) to obtain 1-12, ¹HNMR (400 MHz, DMSO-80DCl=4:1) δ9.05 (s, 1H), 8.41 (s, 1H), 7.80 (s, 1H), 7.57 (s, 1H), 7.46-7.55 (m, 2H), 5.29 (s, 2H), 5.08 (s, 2H), 3.92 (s, 3H); LC-MS(ESI):532 [M+H]⁺.

### Synthesis of deuterated compound 1-1

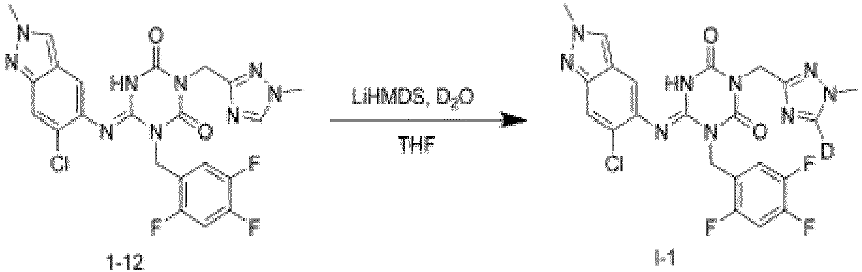

Dissolve 1-12 in anhydrous tetrahydrofuran, cool it in ice bath, add LiHMDS in drops, then add heavy water in drops after 30min, stir for 1h, pour the reaction liquid into water, adjust pH to about 6-7 with hydrochloric acid, extract with ethyl acetate, combine organic phase, dry and concentrate with anhydrous sodium sulfate, and purify the compound by silica gel column chromatography.LC-MS (ESI): 533 [M+H]⁺.

### Embodiment 2 Synthesis of deuterated compounds 1-2

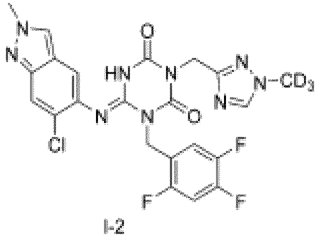

### Synthesis method1 of deuterated compound 1-2

### Synthesis of intermediate 2-04

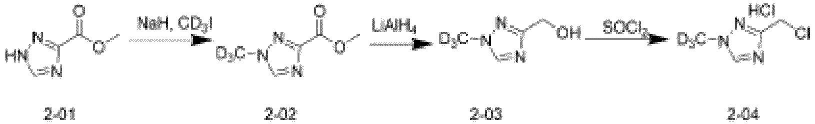

Dissolve 2-01 in anhydrous tetrahydrofuran, cool it in ice bath, add LiHMDS, react for about 30min, then add deuteriodomethane in drops, then when it rise to room temperature, stir it to react for 2h, then quenching with water, extract dichloromethane, combine organic phase, wash in saturated salt water, dry with anhydrous sodium sulfate, filter and concentrate, and purify 2-02 by column chromatography. ¹HNMR (400 MHz, CDC13) δ 8.17 (s, 1H), 4.01 (s, 3H).

Will 2-01 (127.1g.1.0mo!) dissolved in anhydrous tetrahydrofuran (1000mL), cooled in ice bath, added sodium hydride (60%, 44g), reacted for about 30min, then added deuteriodomethane (159.5g, 1.1mol) in drops, then raised to room temperature for stirring reaction for 2h, quenched with water, extracted dichloromethane, combined with organic phase, and dissolved in water. Wash in saturated salt water and dry with anhydrous sodium sulfate.2-02 (65.3g, yield 45%), ¹HNMR (400 MHz, CDCl₃) δ 8.17(s,111),4.01(s,3H) were obtained by filtration and column chromatography.LC - MS (ESI) 145.1 [M+H]⁺.

Under the protection of nitrogen, 2-02 was dissolved in anhydrous tetrahydrofuran, and the tetrahydrofuran solution of lithium aluminum tetrahydrowas added in drops, the reflux reaction was heated for 1h, and then cooled to room temperature, 1ml of water was slowly added for quenching, and then anhydrous magnesium sulfate was added, stirred for 1h, filtered and concentrated.2-03, 'H NMR (400 MHz,CDCl3) δ 8.04(d, J= 36.0Hz,1H),4.76(s,2H),3.48(s,1H) were obtained by column chromatography.

Under nitrogen protection, 2-02 (57.6g, 400mmol) was dissolved in anhydrous tetrahydrofuran (500mL), and lithium aluminum tetrahydro(18.5g) was added in drops.440mmol) tetrahydrofurmine (100mL) solution was heated, the reflux reaction 1h was heated, cooled to room temperature, slowly added to water quenching, then added anhydrous magnesium sulfate, stirred for 1h, filtered, concentrated, and purified by column chromatography to obtain 2-03 (37.9g, yield 81%), ¹HNMR (400 MHz,CDC13) δ 8.04 (d, J = 36.0 Hz, 1H), 4.76 (s, 2H), 3.48 (s, 1H); LC- MS (ESI) 117.1 [M+1]⁺.

Dissolve 2-03 in anhydrous dichloromethane, add sulfoxide chloride, stir at room temperature for 3h, concentrate under reduced pressure to remove solvent and excess to obtain sulfoxide chloride, residue with ether beating, filtration, vacuum drying, 2-04.LC-MS (ESI): 135 [M-H]⁺.

Dissolve 2-03 (35.1g, 300mmol) in anhydrous dichloromethane (100mL), add sulfoxide chloride (50mL), stir at room temperature for 3h, concentrate under pressure to remove solvent and excess to sulfoxide chloride, and the residue is beaten with ether, filtered, vacuum dried to obtain 2-04 (46.5g, 90% yield). ¹HNMR (400 MHz, DMSO) δ 8.06 (s, 1H), 4.78 (s, 2H); LC-MS (ESI): 135 [M+H]⁺.

### Synthesis of intermediate 2-05

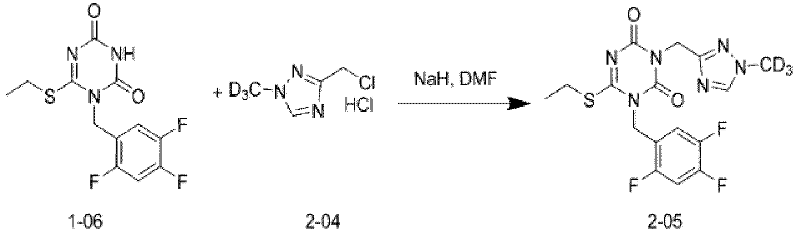

Dissolve 1-06 (2.50g, 7.88mmol) and 2-04 (1.99g, 11.8mmol) in DMF (23mL) and add anhydrous potassium carbonate (3.27g, 23.6mmol), temperature rise to 60°C, stirring reaction for 4h, cooling to room temperature, the reaction liquid is poured into ammonium chloride aqueous solution, solid precipitation, filtration, water washing, solid recrystallization with ethanol to 2-05 (2.32g, yield 71%), ¹HNMR (400 MHz, DMSO-d6), δ 8.36 (s, 1H), 7.57 7.70 (m, 1H), 7.38-7.52 (m, 1H), 5.12 (s, 2H), 4.98 (s, 2H), 3.15 (q, 2H), 1.28 (L, 3H); LC-MS(ESI): 416[M+H]⁺.

### Synthesis of target compounds 1-2

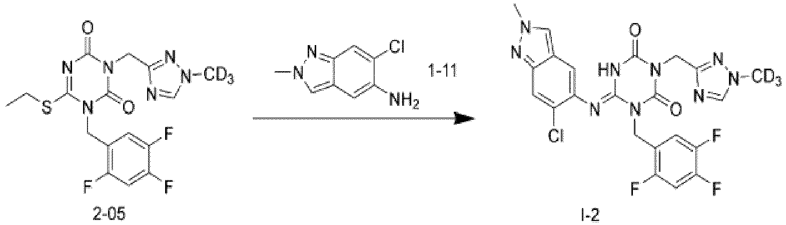

Dissolve 2-05 (5.0g, 12.6mmol), 1-11 (2.06g, 18.9mmol) in the mixture of acetic acid (11.32g, 189mmol) and tert-butanol (100mL), stir at reflux at high temperature for 3 hours, cool to room temperature, and add the reaction solution to saturated sodium bicarbonate aqueous solution (500mL). It was extracted with ethyl acetate (500mL), combined with organic phase, washed, dried with anhydrous sodium sulfate, filtered, concentrated under reduced pressure, and the residue was separated by silica gel column chromatography (gradient elelation with dichloromethane and methanol) to obtain 1-2 (3.22g, 50% yield), ¹HNMR (400 MHZ, DMSO-d6): 4.14 (s, 3H), 4.92 (s, 2H), 5.23 (s, 2H), 7.45 7.65 (m, 2H), 7.71 (s, 1H), 8.29 (s, 1H), 8.35 (s, 1H); ¹HNMR (400MHz, Pyridine-d5) δ 8.33 (s, 1H), 8.02-7.91 (m, 1H), 7.87 (s, 2H), 7.86 (s, 1H), 7.38 (s, 1H), 7.13-7.22 (m, 1H), 5.66 (s, 3H), 5.57 (s, 3H), 3.99 (s, 3H); LC-MS(ESI, m/z) 535[M+H]⁺.

### Synthesis method 2 of deuterated compound 1-2:

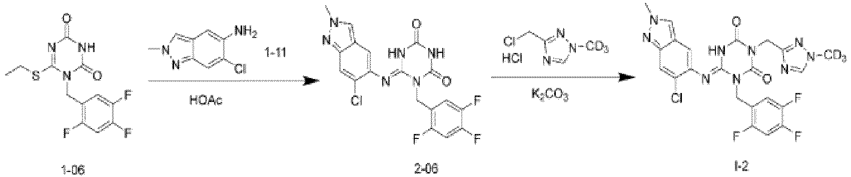

### Synthesis of compound 2-06

Make 1-06 (6.35g.20.0mmol) and 1-11 (5.45g, 30.0mmol) dissolved in acetic acid (50mL), stir and reflux at high temperature for 5 hours, cool to room temperature, solid precipitates, filtration, washing, drying, 2-06 (4.63g, 53% yield). ¹H-NMR (400 MHz, DMSO-d6): δ 11.33 (s, 1H), 11.63 (s, 1H), 8.26 (s, 1H), 7.69-7.56 (m, 3H), 7.13 (s, 1H), 5.15 (s, 2H), 4.16 (s, 3H); MS (ESI, m/z) 437.2 [m+H]⁺.

### Synthesis of compounds 1-2

Put 2-06 (4.19g, 10.0mmol) and 2-04 (2.56g, 15.0 mmol) dissolved in DMA (23mL), then anhydrous cesium carbonate (4.14g, 30.0 mmol) is added, and the temperature is raised to 60°C for stirring reaction for 6h. Cool to room temperature, the reaction liquid is poured into ammonium chloride aqueous solution, solid precipitation, filtration, water washing, drying to obtain crude products, purified by silica gel column chromatography (gradient elution of dioxomethane/methanol), 1-2 (2.25g yield 35%).¹H NMR (400 MHZ, DMSO-d6): 4.14 (s, 3H), 4.92 (s, 2H), 5.23 (s, 2H), 7.45-7.65 (m, 2H), 7.71 (s, 1H), 8.29 (s, 1H), 8.35 (s, 1H); ¹H NMR (400 MHz, Pyridine-d5) δ 8.33 (s, 1H), 8.02-7.91 (m, 1H), 7.87 (s, 2H), 7.86 (s, 1H), 7.38 (s, 1H), 7.13-7.22 (m, 1H), 5.66 (s, 3H), 5.57 (s, 3H), 3.99 (s, 3H); LC-MS(ESI, m/z) 535[M+H]⁺.

### Synthesis of compounds 1-3 in Embodiment 3

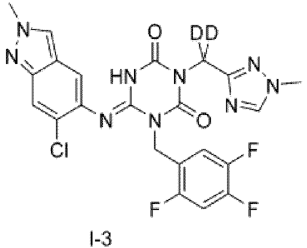

### Synthesis method 1 of deuterated compound 1-3:

### Synthesis of intermediate 3-03

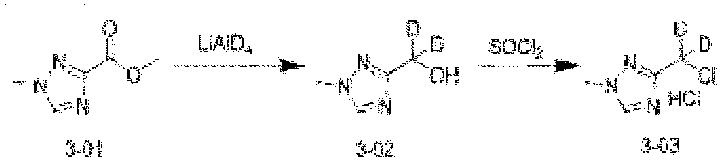

Under the protection of nitrogen, put tetratritium aluminum lithium suspended in anhydrous tetrahydrofuran, drip into the solution of tetrahydrofuran dissolved in 1-methyl-1h-1,2, 4-triazole-3-methyl formate, raise the temperature for reflux for 1h, cool, quench with 1mL, then add anhydrous magnesium sulfate, stir for 30min, filter, concentrate to dry, and 3-02 is obtained. ¹HNMR (400 MHz, DMSO-d6) δ 8.23 (s, 1H), 3.81 (s, 3H), 3.20 (s, 1H).

Under the protection of nitrogen, put tetratritium aluminum-lithium (5.1g, 110mmol) suspended in anhydrous tetrahydrofuran (50mL), and make the solution of tetrahydrofuran (200mL) in 1-methyl-1h-1,2, 4-triazole-3-methyl formate (14.1g, 100mmol) added in drops, and then heat it and reflux for 1h. Cool and add 5mL water to quench, then add anhydrous magnesium sulfate, stir for 30min, filter, concentrate until dry, 3-02 (8.1g, 70% yield) is obtained. ¹H NMR (400 MHz, DMSO - d6) δ 8.23 (s, IH), 3.81 (s, 3H), 3.20 (s, 1H): LC - MS (ESI): 116.1 [M+H]⁺.

Redissolve the 3-02 obtained above with anhydrous dichloromethane, add with sulphoxide chloride, stir at room temperature for 3h, the reaction is detected by TLC, concentrate under reduced pressure, and the residue is beaten with ether, filter, and dry in vacuum to obtain 3-03, ¹HNMR (400MHz, DMSO-d6) δ 8.43 (s, 1H), 3.82(s,3H); LC-MS(ESI):134[M+H]⁺.

Put the 3-02 obtained above redissolved with anhydrous dichloromethane (100mL), and add the sulfone chloride (10mL), stir at room temperature for 3h. After the TLC test is completed, the reaction is concentrated under reduced pressure, and the residue is beater with ether, filter, and dry in vacuum to obtain 3-03 (9.38g, 90% yield). ¹H NMR (400 MHz, DMSO-d6) δ 8.43 (s,1H),3.82(s,3H); LC-MS(ESD):134[M+H]⁺.

### Synthesis of intermediate 3-04

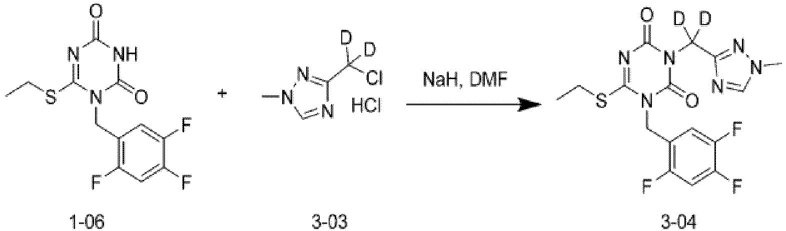

Let 1-06 (2.50g, 7.88 mmol) and 3-03 (1.99g, 11.8 mmol) dissolved in DMF (23mL), adding anhydrous potassium carbonate (3.27g, 23.6mmol), stirring at 60°C for 4h, cooling to room temperature, pouring the reaction liquid into ammonium chloride aqueous solution, solid precipitation, filtration, water washing, solid recrystallization with ethanol to produce intermediates 3-04 (2.28g, 70%).¹H NMR (400 MHz, DMSO d6) δ 8.36 (s, 1H), 7.58-7.69 (m, 1H), 7.51-7.37 (m, 1H), 5.12 (s, 2H), 3.80 (s, 3H), 3.14 (q, 2H), 1.28 (t, 3H); LC-MS(ESI):415[M+H]⁺.

### Synthesis of deuterated compounds 1-3

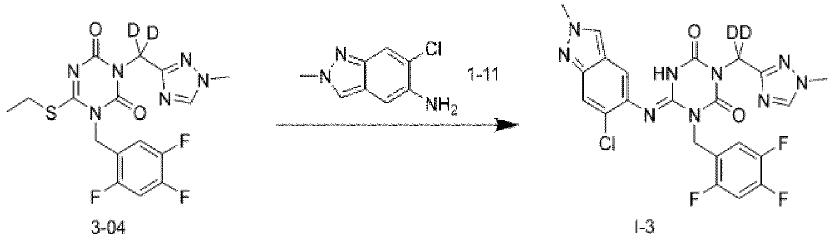

Dissolve 3-04 (5.0 g, 12.6mmol) and 1-11 (2.06g, 18.9 mmol) in the mixture of acetic acid (11.32g, 189mmol) and tert-butanol (100mL), heating, refluxing, stirring and reacting for 3 hours, cool to room temperature, and add the reaction solution to saturated sodium bicarbonate aqueous solution (500mL).It was extracted with ethyl acetate (500mL), combined with organic phase, washed, dried with anhydrous sodium sulfate, filtered, concentrated under reduced pressure, and the residue was separated by silica gel column chromatography (gradient elelation with dichloromethane and methanol, 0-20% MeOH) to obtain 1-3 (3.34g), 50% yield), ¹H NMR (400 MHz, DMSO - d6) δ 11.05 (s, 0.6 H, NH), 9.70 (s, 0.4H, NH), 8.36 (s, 1H), 8.28 (s, 1H), 7.71 (s, 1H), 7.40-7.65 (m, 2H), 7.15 (s, 1H), 5.23 (s, 2H), 4.15 (s, 3H), 3.80 (s, 3H);LC-MS(ESI): 534[M+H]⁺.

### Synthesis method 2 of deuterated compound

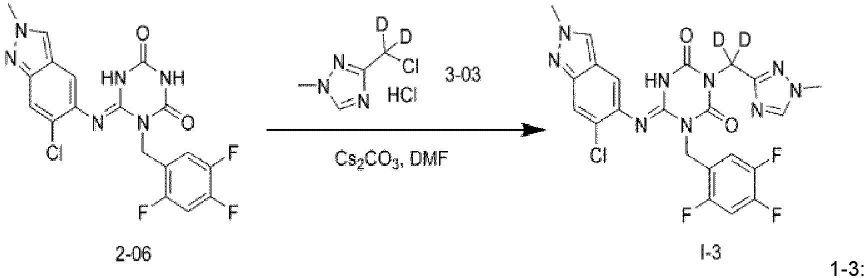

Dissolve 2-06 (4.19 g.10.0 mmol) and 3-03 (2.56g, 15.0 mmol) in DMA (23mL) and add anhydrous cesium carbonate (4.14g). Then raise the temperature to 50°C and stir it for reaction for 3 hours. Cool it to room temperature. Pour reaction liquid into ammonium chloride solution, solid precipitated, filtered, washed in water, dried to coarse product, purified by silica gel column chromatography (gradient elution of dichloromethane/methanol), get I-3 (2.25g yield 35%).¹H NMR (400 MHz, DMSO-d6) δ 11.05 (s, 0.6H, NH), 9.70 (s, 0.4H, NH), 8.36 (s, 1H),8.28 (s, 1H), 7.71 (s, 1H), 7.40-7.65 (m, 2H), 7.15 (s, 1H), 5.23 (s, 2H), 4.15 (s, 3H), 3.80 (s, 3H);LC-MS(ESI, m/z) 534[M+H]+.

### Embodiment 4 Synthesis of deuterated compounds 1-4

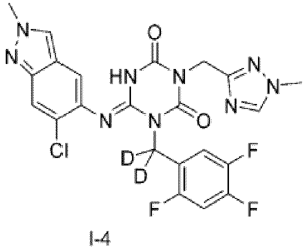

### Synthesis of intermediate 4-03

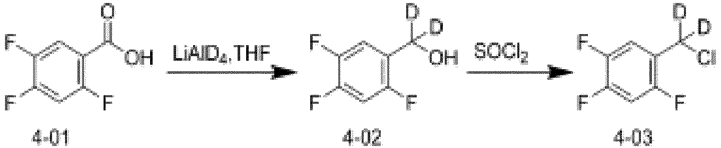

Under the protection of nitrogen, Suspend and dissolve tetradeuterium aluminum lithium in anhydrous tetrahydrofuran, drip into the solution of tetrahydrofuran dissolved in 2,4,5-trifluorobenzoic acid, then raise temperature and reflux for 1h, cooling, quench with 1mL, then add anhydrous magnesium sulfate, stirring for 30min, filtered, concentrated to dry, 4-02 was obtained, which was directly used in the next step.

Under nitrogen protection, Suspend and dissolve tetratritium aluminum lithium (10.2g, 220mmol) in anhydrous tetrahydrofuran (100mL), and add solution of tetrahydrofuran(200mL) dissolved in 2,4,5-trifluorobenzoic acid (35.2g, 200mmol), , then raise temperature and reflux for 1h, cooling, quench with 5mL water, then anhydrous magnesium sulfate was added, stirred for 30min, filtered, concentrated to dry, 4-02 (29.8g, 90% yield) was obtained.

Dissolve 4-02 with anhydrous dichloromethane, add sulphoxide chloride, stir at room temperature for 3h, TLC test is completed, pour the reaction liquid into ice water, extract with dichloromethane 3 times, combine organic phase, wash with saturated salt water, sodium bicarbonate and water, anhydrous sodium sulfate dry, filter, concentrate, obtain 4-03, LC-MS (ESI) :183 [M+H] ⁺.

Redissolve 4-02 (29.8g) with anhydrous dichloromethane, add sulphoxide chloride (20mL), stir at room temperature for 3h, TLC test is completed, pour the reaction liquid into ice water, extract with dichloromethane 3 times, combine organic phase, wash with saturated salt water, sodium bicarbonate and water, anhydrous sodium sulfate dry, filter and concentrate,4-03 (32.4 g, yield of 98%), ¹H NMR (300 MHz, CDCl₃) δ 7.34-7.24 (m, 1H), 6.69-6.88 (m, 1H); LC-MS(ESI): 183[M+H]⁺.

### Synthesis of intermediate 4-05

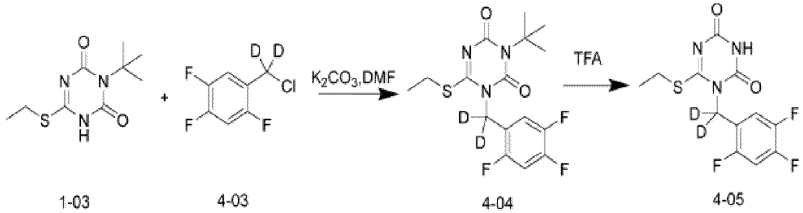

Add potassium carbonate (17.97g, 130mmol) into acetonitrile solution(200mL) of 1-03 (22.93g, 100 mmol) and 4-03 (22.60g, 110mmol). Stir for 3 hours while heating and refluxing. Filter the reaction liquid, concentrate the filtrate under reduced pressure, and obtain 4-04 (33.8g, 90%), which is directly used for the next reaction. ¹H NMR (400 MHz, CDCl₃) δ 7.04-6.92 (m, 2H), 3.18 (q.2H), 1.67 (s, 9H), 1.36 (t, 3H)

Dissolve 4-05 obtained in the previous step in dichloromethane under ice bath cooling, and add trifluoroacetic acid and stir it at room temperature for 24 hours. The reaction liquid is concentrated under reduced pressure to obtain 4-05, LC-MS (ESI): 320 [M+H]⁺.

Dissolve 4-05 (30.0g, 0.69 mmol) obtained in the previous step in dioxomethane (100mL) under ice bath cooling, and trifluoroacetic acid (100mL) is added and stirred at room temperature for 24 hours. The reaction solution is reduced pressure and concentrated to obtain 4-05 (25.5g.99%), 1H NMR (400 MHZ, CDCl₃) δ (s, 1H). 8.81 7.20-6.93 (m, 2H), 3.28 (q. 2H), 1.42 (t, 3H); LC-MS(ESI): 320[M-H]⁺.

### Synthesis of intermediate 4-06

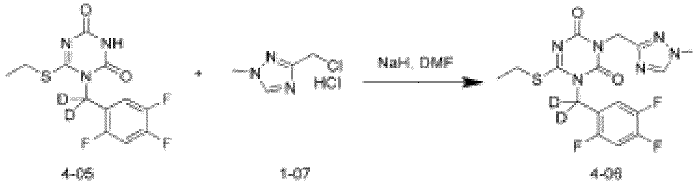

Dissolve 4-05 (2.50g, 7.88 mmol) and 1-07 (1.99 g, 11.8mmol) in DMF (23mL) and add anhydrous potassium carbonate (3.27g, 23.6 mmol), raise temperature to 60°C and stir for reaction for 4h. Cool to room temperature and pour the reaction liquid into ammonium chloride solution, solid precipitation, filtration, water washing, solid recrystallization with ethanol to 1-08 (2.36g, yield 72%), ¹H NMR (400 MHz, CDCl₃) δ 8.00 (d, 1H), 7.26-7.13 (m, 1H), 7.05-6.96 (m, 1H), 5.30 (s, 2H), 3.92 (d, 3H), 3.27 (q, 2H), 1.40 (t, 3H); LC-MS(ESI):415.1[M+H]⁺.

### Synthesis of deuterated compounds 1-4

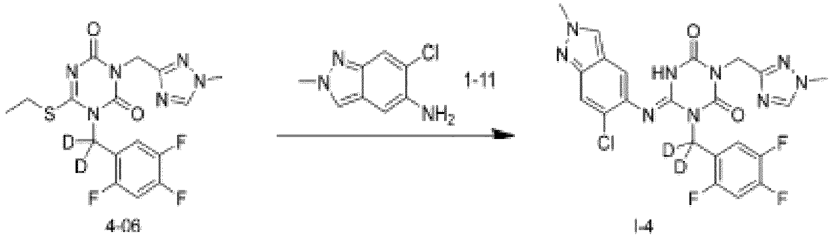

Dissolve 4-06 (5.0g, 12.6 mmol) and 1-11 (2.06g, 18.9mmol) in a mixture of acetic acid (11.32g, 189mmol) and tert-butanol (100mL), raise the temperature, reflux and stir it for reaction for 3 hours, cool to room temperature. Add the reaction solution into saturated sodium bicarbonate aqueous solution (500mL), extract with ethyl acetate (500mL), combine with organic phase, washed, dried with anhydrous sodium sulfate, filtered, concentrated under reduced pressure, and make the residue separated by silica gel column chromatography (gradient elelation with dichloromethane and methanol, 0-20% MeOH) to obtain compounds 1-4 (3.09g, 48%). ¹H NMR (400 MHz, DMSO) δ 11.07 (s, 0.65H), 9.68 (s, 0.35H), 8.23-8.44 (m, 2H), 7.59-7.81 (m, 2H), 7.13 (s, 1H), 4.92 (s, 2H), 4.14 (s, 3H), 3.81 (s, 3H); LC - MS (ESD): 534.1 [M+H] ⁺.

### Embodiment 5 Synthesis of deuterated compounds 1-5

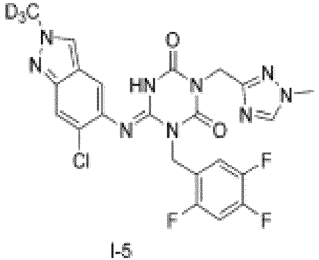

### Synthesis of intermediate 5-01

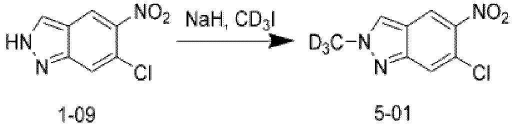

Dissolve 1-09 (334mg, 1.35 mmol) in DMF (10mL), cool it in ice bath, add sodium hydride (562mg, 4.07mmol), stir for 30min, then slowly add deiodomethanes (0.17mL, 2.71mmol), increase the temperature to room temperature and continue to stir for 2h.The reaction mixture is diluted with dichloromethane, washed in saturated salt water, dried with anhydrous sodium sulfate, filtered and concentrated, and the residue is purified by silica gel column chromatography to obtain 5-01.

¹HNMR (400 MHz, DMSO-d6) δ 8.74 (s, 1H), 8.69 (s, 1H), 8.01 (s, 1H); LC-MS(ESI): 215[M +H]⁺.

### Synthesis of intermediate 5-02

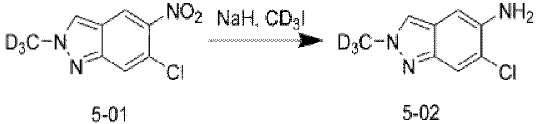

Dissolve 5-01 (0.39g, 1.5 mmol) in ethanol (15mL), add iron powder (0.42g, 7.5mL) and 0.4mL hydrochloric acid, rise temperature for reflux reaction for 1h, cool to room temperature, dilute the reaction solution with ethanol, filter with diatomaceous earth, concentrate, dissolve the residue with ethyl acetate, wash with sodium bicarbonate solution, water and salt water. Anhydrous sodium sulfate drying. Concentrated under reduced pressure and purified by silica gel column chromatography (PE/EA = 4/1), 5-02(0.24 g, 87%) is obtained. ¹H NMR (400 MHz, DMSO - d6) δ 7.98 (s, 1H), 7.58 (s, 1H), 6.87 (s, 1H), 4.95 (s, 2H); LC-MS(ESI): 185 [M+H]⁺.

### Synthesis of compounds 1-5

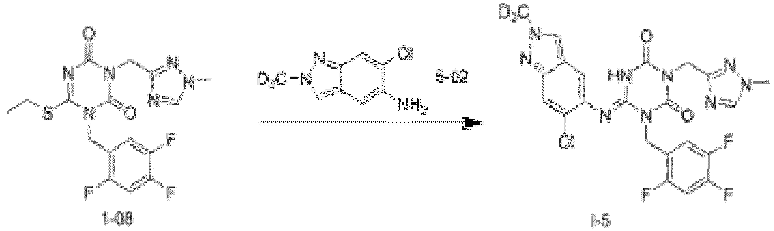

Dissolve 1-08 (5.0g, 12.6mmol) and 5-02 (2.06g, 18.9mmol) in a mixture of acetic acid (11.32g, 189mmol) and tert-butanol (100mL), raise the temperature, reflux and stir it for reaction for 3 hours, cool to room temperature. Add the reaction solution into saturated sodium bi carbonate aqueous solution (500mL), extract with ethyl acetate (500mL), combined with orga nic phase, washed with water, dried with anhydrous sodium sulfate, filtered, concentrated un der reduced pressure. The residue is separated by silica gel column chromatography (gradie nt elution with dichloromethane-methanol, 0-20% MeOH) to obtain compounds 1-5, ¹HNMR (400 MHz, DMSO-d6) δ (11.07s, 0.6H, NH), 9.68 (s, 0.4H, NH), 8.36 (s, 1H), 8.23 (s, 1H), 7.35-7.85 (m, 3H), 7.13 (s, 1H), 5.20 (s, 2H), 4.92 (s, 2H), 3.80 (s, 3H); LC-MS(ESI): 535[M +H]+.

### Embodiment 6 Synthesis of deuterated compounds 1-6

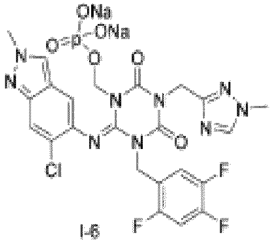

### Synthesis of intermediate 6-01

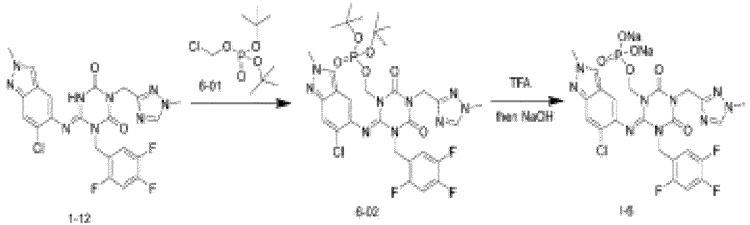

Dissolve compounds 1-12 in DMF, cool it in ice bath, add with sodium hydride, stir for about 30min, then slowly added with di-tert-butyl chloromethyl phosphate, increase to room temperature and stir for reaction for 3h.Compound 6-02, LC-MS (ESI) : 754 [M+H]⁺ is obtained after the reaction solution is diluted with ethyl acetate, washed in saline solution, dried with anhydrous sodium sulfate, concentrated and purified by silica gel column chromatography.

Dissolve 6-02 in dichloromethane, add trifluoroacetic acid, stir for reaction 1h, reduce pressure and concentrate to remove solvent and excess trifluoroacetic acid, the residue is re-dissolved with ethanol, add sodium hydroxide ethanol solution, stir 1h, solid precipitation, filtration, dried, then obtain compounds 1-6.

### Examples 7-11

Using the same synthesis method of Embodiment 6, let 1-1, 1-2, 1-3, 1-4, 1-5, etc., reacted with 6-01 respectively, and then the tert-butyl group is removed by trifluoroacetic acid to obtain compounds 1-7, 1-8, 1-9, 1-10, and 1-11, respectively.

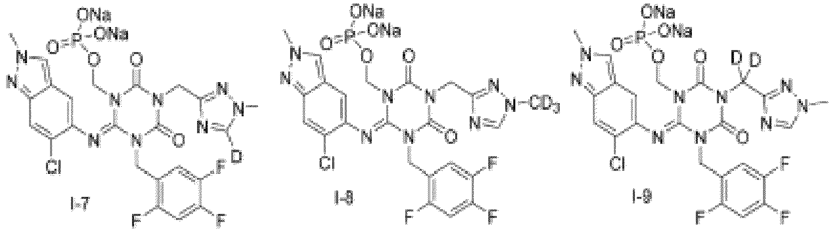

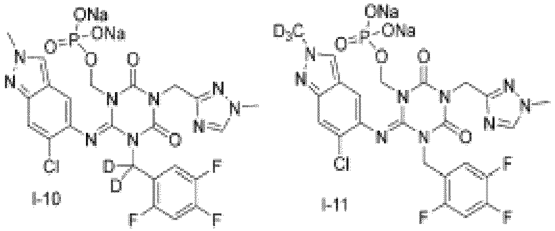

### Embodiment 12 Synthesis of compounds 1-12

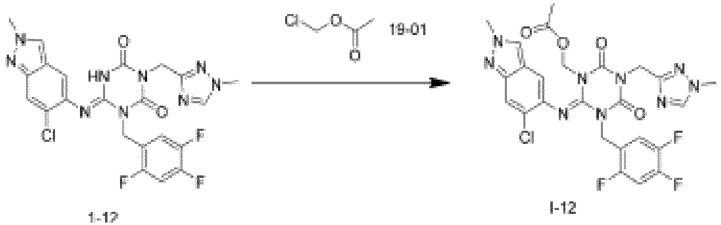

Dissolve compounds 1-12 in DMF, cool in ice bath, add sodium hydride, stir for about 30min, and then slowly add chloromethyl acetate, heat to room temperature stirring reaction for 3h.The reaction solution is diluted with ethyl acetate, washed in salt water, dried with anhydrous sodium sulfate, concentrated, and purified by silica gel column chromatography to obtain compound I-12, LC-MS (ESI) : 604 [M+H]⁺.

### Embodiment 13 synthesis of compounds 1-13

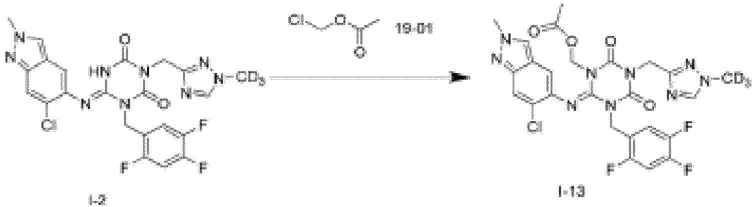

Dissolve compounds 1-2 in DMF, cool in ice bath, add sodium hydride, stir for about 30min, then slowly add chloromethyl acetate, , increase to room temperature and stir for reaction for 3h.The reaction solution is diluted with ethyl acetate, washed in salt water, dried and concentrated with anhydrous sodium sulfate, and purified by silica gel column chromatography and compounds 1-13 is obtained, H NMR (400 MHz, DMSO-d6) 1.23 (s, 3H), 4.12 (s, 3H), 4.97 (s, 2H), 5.23 (s, 2H), 5.67 (s, 2H), 7.34 (s, 1H), 7.48-7.61 (m, 2H), 7.64 (s, 1H), 8.20 (x, 1H), 8.43 (s, 1H); LC-MS(ESI): 607.1[M+H] ⁺.

### Embodiment 14 Synthesis of compounds 1-14

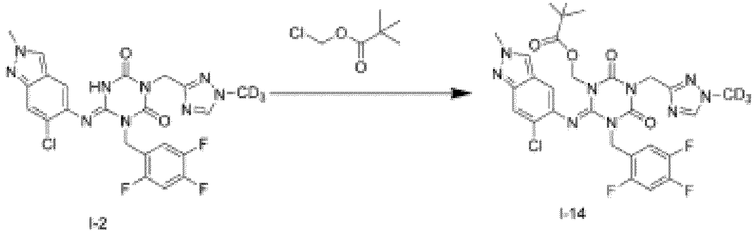

Dissolve compounds I-2 in DMF, cool in ice bath, add with sodium hydride, stir for about 30min, then slowly add with chloromethyl tervalerate, then increase to room temperature and stir for reaction for 3h. The reaction solution was diluted with ethyl acetate, washed with salt water, dried with anhydrous sodium sulfate, concentrated, and purified by silica gel column chromatography and compounds I-14 is obtained, H NMR (400 MHz, DMSO-d6) 0.99 (s, 9H), 4.11 (s, 3H), 6.03 (s, 2H), 5.05 (s, 2H), 5.58 (s, 2H), 7.07 (s, 1H), 7.30-8.45 (m, 1H), 8.45-7.62 (m, 1H), 7.67 (s, 1H), 8.20 (s, 1H), 8.40 (s, 1H); LC-MS(ESI): 649.2[M+H]⁺.

Dissolve compounds I-2 (5.2g, 9.72mmol) in DMF (50mL), cool in ice bath, add sodium hydride, stir for about 30min, then slowly added chloromethyl tervalonate (2.19g, 14.58mmol), then increase to room temperature and stir for reaction for 3h.The reaction solution is diluted with ethyl acetate and washed with salt water, dried with anhydrous sodium sulfate and purified by silica gel column chromatography, then compound I-14(1.89g, 30% yield) is obtained.

### Embodiment 15-17

Compounds 1-15, 1-16, and 1-17 are obtained by reacting 1-3, 1-4, and 1-5 with chloromethyl acetate, respectively, using the same synthesis method as in Embodiment 6.

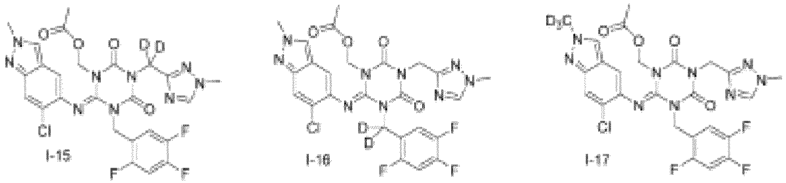

### Embodiment 18 Synthesis of compounds 1-18

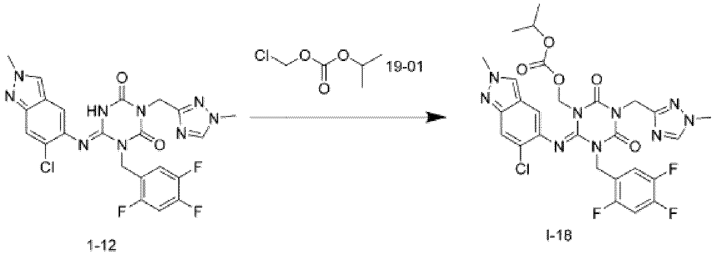

Dissolve compounds I-12 in DMF, cool in ice bath, add with sodium hydride, stir for about 30min, then slowly add with isopropyl chloromethyl carbonate, then increase to room temperature and stir for reaction for 3h. The reaction solution is diluted with ethyl acetate, washed with salt water, dried with anhydrous sodium sulfate, concentrated, and purified by silica gel column chromatography to obtain compound 1-18. LC-MS (ESI): 648 [M+H]⁺.

### Embodiment 19 Synthesis of compound 1-19

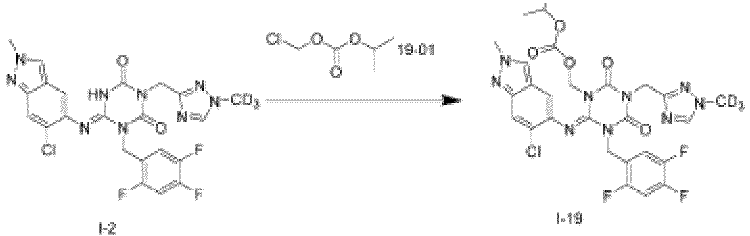

Dissolve compound I in DMF, cool in ice bath, add sodium hydride, stir for about 30min, then slowly add isopropyl chloromethyl carbonate, increase to room temperature and stir for reaction for 3h.The reaction solution is diluted with ethyl acetate, washed in salt water, dried with anhydrous sodium sulfate, concentrated, and purified by silica gel column chromatography to obtain compound 1-19, H NMR (400 MHz, DMSO-d6) :1.04 (d, 6H), 4.10 (s, 3H),4.30-4.41 (m, 1H), 5.02 (s, 4H), 7.05 (s, 1H), 7.28-7.40 (m, 1H), 7.51-7.60 (m, 1H), 7.64 (s, 1H), 8.18 (s, 1H), 8.41 (s, 1H); LC-MS (ESD): 651.2 [M+H]⁺.

Dissolve compounds I-2 (5.2g, 9.78mmol) in DMF, cool in ice bath, add sodium hydride, stir for about 30min, and then slowly added isopropyl chloromethyl carbonate (2.24g, 14.67mmol), then increase to room temperature and stir for reaction for 3h.The reaction solution Is diluted with ethyl acetate, washed in salt water, dried with anhydrous sodium sulfate, concentrated, and purified by silica gel column chromatography to obtain compounds 1-19 (1.78g, 28% yield).

### Embodiments 20-23

The compounds 1-20, 1-21, 1-22, 1-23 are obtained by reacting 1-1, 1-3, 1-4, 1-5, etc., with methyl chloride acetate by the same synthesis method as in embodiment 6.

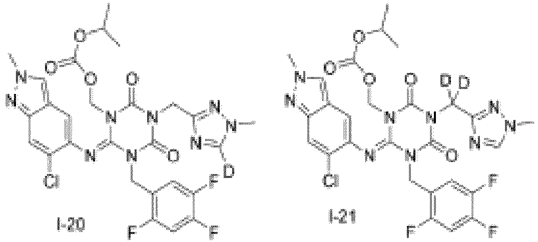

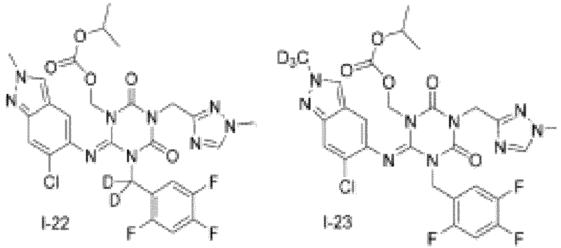

### Embodiment 24 Synthesis of deuterated compound 1-24

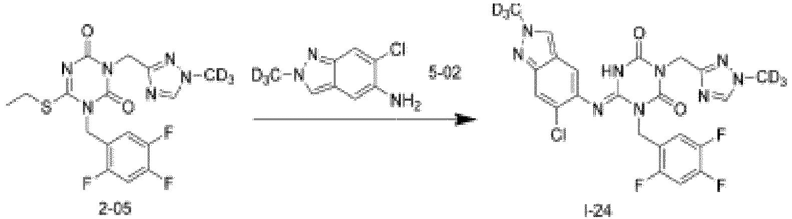

Dissolve 2-05 (5.0g, 12.6 mmol) and 5-02 (2.06g, 18.9 mmol) in the mixture of acetic acid (11.32g, 189mmol) and tert-butanol (100mL), ), raise the temperature, reflux and stir it for reaction for 3 hours, cool to room temperature, and add the reaction solution to sa turated sodium bicarbonate aqueous solution (500mL).The compounds 1-24 is extracted w ith ethyl acetate (500mL), combined with organic phase, washed, dried with anhydrous so dium sulfate, filtered, concentrated under reduced pressure, and the residue is isolated by silica gel column chromatography, ¹H NMR (400 MHz, DMSO-d6) 4.88 (s, 2H), 5.16 (s, 2H), 7.13 (s,1H), 7.30-7.90 (m, 3H), 8.33 (s, 2H), 12.01 (BRS, 1H); LC-MS (ESI, m/z) 65 1.2 [M+H]⁺; LC-MS (ESI): 538.1[M+H]⁺.

### Embodiment 25 Synthesis of compound 1-25

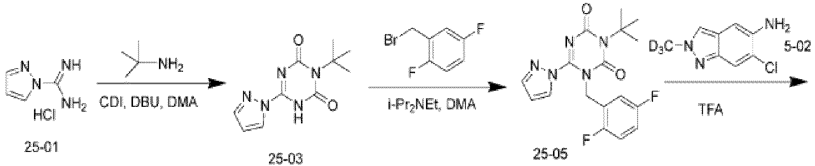

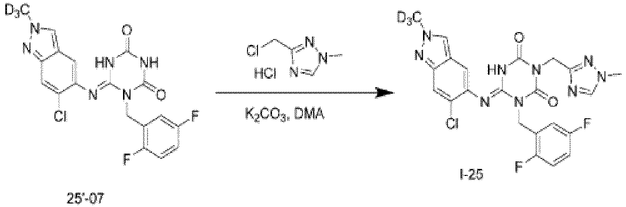

### Synthesis of intermediate 25-03

Cooled in an ice bath, Add 1,1-bitazole CDI (170.3g, 1.05 mmol) and DBU (167.5g, 1.10m mol) into the DMA solution (2L) of tert-butylamine (73.14g, 1.0mol), stir for 3h and add pyrazol e-1-methylformamidine (110.12g, 1.0mmol) for 5h, and then add 1,1'-carbonyl dimisazole (194.5 8g, 1.2mol) and DBU (182.7g).120mmol), raise temperature to 50°C for stirring reaction overnig ht (about 15h). After cooling, the reaction liquid is poured into ice water, the pH is adjusted to 5-6 with dilute hydrochloric acid, solid matter is precipitates, filtered, washed and dried to obtai n the compound 25-03, 200.1g yield: 85%. LC-MS(ESI):236.1[M+H]⁺.

### Synthesis of intermediate 25-05

Dissolve compound 25-03 (23.5g, 100mmol) and diisopropylethylamine (10.6g, 120mmol) in DMA, stir at room temperature for 30min, and then slowly add 2,5-difluorobenyl bromide (4a, 2 4.75g, 110mmol), and then reacted at 60°C for 8h. TLC test reaction complete. The reaction liq uid is cooled to room temperature, then pour into ice water, the pH value is adjusted to acidity with dilute hydrochloric acid 1, extracted with ethyl acetate, combined with organic phase, was hed with saturated salt water, and dried with anhydrous sodium sulfate. The compound 25-05, 36.1g is purified by silica gel column chromatography with a yield of 95.2%. LC-MS (ESI): 362. 1 [M+H]⁺.

### Synthesis of intermediate 25'-07

Dissolve Compound 25-05 (7.22g, 20.0mmol) and 6-chloro-2 - (methyl-d3)-2H-indazole-5-am ine (4.43g, 24.0 mmol) in trifluoroacetic acid (100mL), heat to 60°C and stir for about 5h. After cooling to room temperature, pour the reaction liquid into ice water, solid precipitated, filtered and washed, then beaten with ethanol for 1h, filtered and dried, the compound was 25'-07 (6. 92g, yield 82%). LC-MS (ESI): 422.1[M+H]⁺.

### Synthesis of compound I-25

Dissolve 25'-07 (4.21g, 10.0mmol) and (1-methyl-1h-1,2, 4-triazole-3-yl) chloromethane hydro chloride (2.52g, 15.0mmol) in DMA (50mL), and add anhydrous potassium carbonate (3.27g, 2 3.6mmol), rise temperature to 60°C and stir for reaction for 4h. Cool to room temperature, pour the reaction liquid into ice water, solid precipitated, filtered, washed in water, dried to coarse product, purified by silica gel column chromatography (gradient elution of dichloromethane/meth anol), obtain I-25 (2.25g yield 35%). ¹H NMR (400 MHz, DMSO-d₆): δ 11.10 (s, 0.5H), 9.7(s, 0.5H), 8.39 (d, 1H), 8.31 (d, 1H), 7.75 (d, 1H), 7.67 (s, 1H), 7.34 (m, 1H), 7.26 (m, 1H), 7.17 (m, 1H), 5.28 (d, 2H), 4.92 (d, 2H), 3.80 (d, 3H); LC - MS (ESI): 644.1 [M+H]⁺.

### Embodiment 26 Synthesis of compound 1-26

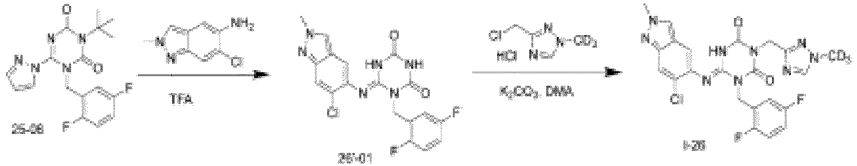

### Synthesis of intermediate 26'-01

Dissolve compound 25-06 (7.22g, 20.0 mmol) and 6-chloro-2-methyl-2h-indazole-5-amine (4. 43g, 24.0 mmol) in trifluoroacetic acid (100mL), heat to 60°C and stir for about 5h, cool to roo m temperature, pour the reaction liquid into ice water, some solid precipitate, filter and wash, t hen beat with ethanol for 1h, filter and dry it. Compound 26'-01 is obtained. (7.11g, yield 8 5%), LC-MS(ESD): 419.1 [M+H]^{+.}

### Synthesis of compound I-26

Dissolve 26'-01 (4.19g, 10.0mmol) and (1-methyl-D3-1h-1,2, 4-triazole-3-yl) chloromethane h ydrochloride (2.56g, 15.0 mmol) in DMA (23mL) and add anhydrous potassium carbonate (4.14 g, 30.0 mmol), rise temperature to 60°C for stirring reaction for 6h. Cool to room temperature, pour the reaction liquid into ammonium chloride aqueous solution, solid precipitation, filtration, water washing, drying to obtain crude products, purified by silica gel column chromatography (d ichloromethane/methanol gradient elution), I-26 is obtained (2.25g yield 35%). ¹H NMR (400 M Hz, DMSO-d₆): δ 11.11 (s, 0.5H), 9.71 (s, 0.5H), 8.38 (d, 1H), 8.31 (d (1H), 7.75 (d, 1H), 7.67 (s, 1H), 7.34 (m, 1H), 7.26 (m, 1H), 7.17 (m, 1H), 5.27 (d, 2H), 4.92 (d, 2H), 4.15 (d, 3H); L C-MS(ESI): 644.1 [M+H]⁺.

### Embodiment 27 Synthesis of compound 1-27

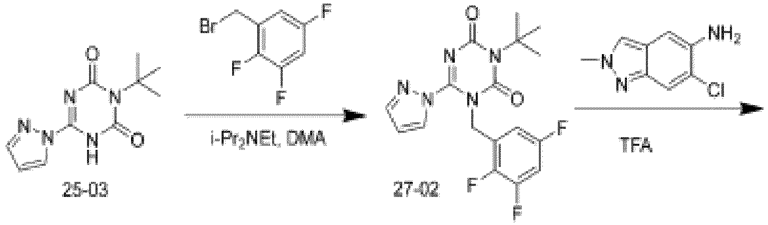

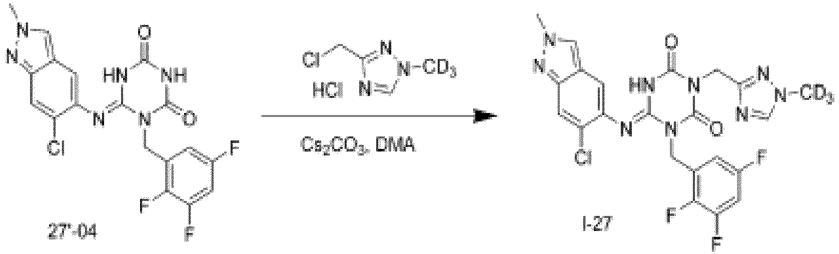

### Synthesis of intermediate 27-02

Dissolve compound 25-03 (7.06g, 30 mmol) and diisopropylethylamine (3.78g, 36.0mmol) in DMF, stir for 30min at room temperature, then slowly add 2,3, 5-difluorobenyl bromide (7.43g, 33.0mmol), and then increase the temperature to 60°C for 8h, and the TLC test is completed. Cool the reaction liquid to room temperature, pour into ice water, the pH value is adjusted to acidity with dilute hydrochloric acid 1, extracted by ethyl acetate, combined with organic phase, washed in saturated salt water, dried with anhydrous sodium sulfate, concentrated under reduc ed pressure, and the crude product is purified by silica gel column chromatography to obtain c ompound 27-02 (10.31g, yield 91%). LC-MS (ESI): 380.1 [M+HJ]⁺.

### Synthesis of intermediate 27'-04

Dissolve the compound 27-02 (7.58g, 20.0mmol) and 6-chloro-2-methyl-2h-indazole-5-amine (4.36g, 24.0mmol) in trifluoroacetic acid (100mL) and heat to 60°C and stir for reaction for abo ut 5h. After cooling to room temperature, the reaction liquid is poured into ice water, solid prec ipitated, filtered and washed, then beaten with ethanol 1h, filtered and dried to obtain compoun d 27'-04 (6.78g, yield 78%). LC-MS(ESI): 437.1 [M+H]⁺.

### Synthesis of compound 1-27

Dissolve 27'-04 (4.37g, 10.0 mmol) and (1-methyl-D3-1h-1,2, 4-triazole-3-yl) chloromethane hydrochloride (2.57g, 15.0mmol) in DMA (23mL), and add anhydrous potassium carbonate (3.27 g, 23.6mmol), increase temperature to 60°C and stir for reaction for 4h, pour the reaction liquid into ammonium chloride aqueous solution, some solid precipitated, filtered, washed in water, d ried to a crude product, purified by silica gel column chromatography (gradient elution of dichlo romethane/methanol), and obtain I-27 (1.82g, yield 34%). ¹H NMR (400 MHz, DMSO-d₆): δ 11. 10 (brs, 0.5H), 9.70(brs, 0.5H), 8.35 (s, 1H), 8.21 (d, 1H), 7.73 (m, 1H), 7.48 (m, 1H), 7.21 (m, 1H), 7.11 (m, 1H), 5.31 (d, 2H), 4.92 (s, 2H), 4.13 (s, 3H); L.C-MS (ESI): 535.1 [M+H]⁺.

### Embodiment 28 Synthesis of compound 1-28

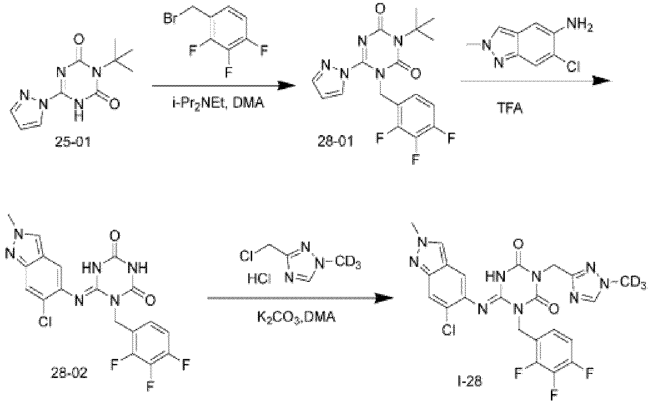

### Synthesis of intermediate 28-01

Dissolve compound 25-01 (7.06g, 30mmol) and diisopropylethylamine (3.78g, 36.0mmol) in DMF, stir at room temperature for 30min, and then slowly add 2,3, 4-difluorobenzyl bromide (7. 43g, 33.0 mmol), and then reacted at 60°C for 8h. After the reaction was completed by TLC t est, the reaction liquid is cooled to room temperature, poured into ice water, the pH value was adjusted to acidity with dilute hydrochloric acid 1, extracted by ethyl acetate, combined with or ganic phase, washed in saturated salt water, dried with anhydrous sodium sulfate, concentrated under pressure, and the crude product is purified by silica gel column chromatography to obta in compound 28-01 (9.66g, yield 85%). LC-MS (ESI): 380.1 [M+H] ⁺.

### Synthesis of intermediate 28-02

Dissolve compound 28-01 (7.58g, 20.0- mmol) and 6-chloro-2-methyl-2h-indazole-5-amine (4.36g, 24.0 mmol) in trifluoroacetic acid (100mL) and heat at 60°C for about 5h. After cooling to room temperature, the reaction liquid is poured into ice water, solid precipitated, filtered and washed, then beaten with ethanol for 1h, filtered and dried to obtain compound 28-02 (6.54g, yield 75%). LC-MS(ESI): 437.1[M+H] ⁺.

### Synthesis of compound I-28

Dissolve 28-02 (4.36g, 10.0mmol) and (1-methyl-D3-1h-1,2, 4-triazole-3-yl) oxymethane hydr ochloride (2.57g, 15.0mmol) in DMA (23mL), then add anhydrous potassium carbonate (3.27g, 23.6mmol), and the reaction is stirred at 60°C for 4h. Cool to room temperature, the reaction li quid is poured into ammonium chloride aqueous solution, solid precipitation, filtration, water was h, dried to obtain crude products, purified by silica gel column chromatography (gradient elution of dichloromethane/methanol), 1-27 (1.87g yield 35%). ¹H NMR (400 MHz, DMSO-d₆): δ 11.04 (BRS, 0.5H), 9.64 (BRS, 0.5H), 8.33 (s, 1H), 7.19 (s, 1H), 7.63 (s, 1H), 7.30 (m, 2H), 7.15 (m, 1H), 5.26 (d, 2H), 4.88 (s, 2H), 4.12 (d, 3H); LC-MS (ESI): 535.1 [M+H] ⁺.

### Embodiment 29 Synthesis of compounds 1-29

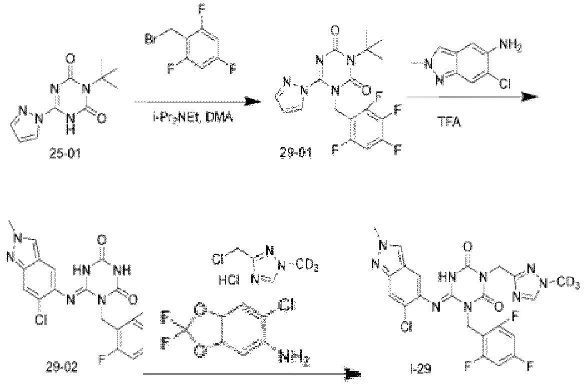

### Synthesis of intermediate 29-01

Dissolve compounds 25-01 (7.06g, 30mmol) and diisopropylethylamine (3.78g, 36.0mmol) in DMA, stir at room temperature for 30min, and then slowly add 2,4, 6-difluorobenzyl bromide (7. 43g, 33.0mmol) in drops, and then reacted at 60°C for 8h. After the reaction is completed, coo I the reaction liquid to room temperature, pour into ice water, the pH value is adjusted to acidit y with dilute hydrochloric acid 1, extracted by ethyl acetate, combined with organic phase, was hed with saturated salt water, dried with anhydrous sodium sulfate, concentrated under pressur e, and the crude product is purified by silica gel column chromatography to obtain compound 2 8-01 (9.66g, yield 85%). LC-MS (ESI): 380.1 [M+H] ⁺.

### Synthesis of intermediate 29-02

Dissolve compound 29-01 (7.58g, 20.0 mmol) and 6-chloro-2-methyl-2h-indazole-5-amine (4. 36g, 24.0mmol) in trifluoroacetic acid (100mL), heat at 60°C and stir for about 5h. After cooling to room temperature, pour the reaction liquid into ice water, solid precipitates, filter and wash, then beat with ethanol for 1h, filter and dry to obtain compound 29-02 (6.54g, yield 75%), LC-MS(ESI): 437.1[M+H] ⁺.

### Synthesis of compound I-29

Dissolve combine 29-02 (4.36g, 10.0mmol) and (1-methyl-D3-111-1,2, 4-triazole-3-yl) chlorom ethanes hydrochloride (2.57g.15.0mmol) into DMF (23mL), add anhydrous potassium carbonate (3.27g).23.6mmol), rise temperature to 60°C and stir reaction for 4h. Cool to room temperature, the reaction liquid is poured into ammonium chloride aqueous solution, solid precipitates, filter, water washing, dry to obtain crude products, purified by silica gel column chromatography (dich loromethane/methanol gradient elution) to get 1-27 (2.03g yield 38%). ¹H NMR (400 MHz, DMS O-d₆): δ 11.04 (brs, 0.5H), 9.64 (BRS, 0.5H), 8.33 (s, 1H), 8.20 (s, 1H), 7.62 (s, 1H), 7.14 (m, 2H), 7.03 (m, 1H), 5.26 (d, 2H), 4.88 (s, 2H), 4.15 (d, 3H); LC-MS(ESI): 535.1[M+H]⁺.

### Comparative example 1: Synthesis of example compound I-30

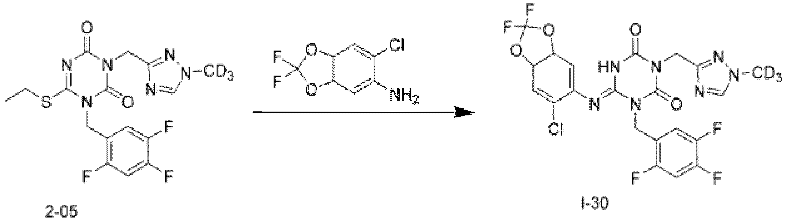

Dissolve 2-05 (5.0g, 12.6mmol), 2, 2-difluoro-5-amino-6-chloro-1, 3-benzodioxazole (2.06g, 1 8.9mmol) in the mixture of acetic acid (11.32g, 189mmol) and isoamyl alcohol (100mL), raise t he temperature, reflux and stir it for reaction for 3 hours, cool to room temperature. Add the r eaction solution to saturated sodium bicarbonate aqueous solution (500mL), extract with ethyl a cetate (500mL), combine with organic phase, wash in water, dry with anhydrous sodium sulfat e, filter, concentrate under reduced pressure, and the residue is purified by silica gel column c hromatography to obtain compound 1-30, LC-MS(ESI): 563.1 [M+H]⁺. Dissolved 2-05 (4.15g, 10. 0 mmol), 6-chloro-2, 2-diamine-3a, 7a-dihydrobenzo [d] [1,3] dioxacyclopentadiene-5-amine (2.06 g, 12.0 mmol) in a mixture of acetic acid (50mL) and tert-butanol (50mL), raise the temperatur e, reflux and stir it for reaction for 3 hours, cool to room temperature. Add the reaction liquid t o saturated sodium bicarbonate aqueous solution (500mL), extract with ethyl acetate (500mL), combine with organic phase, wash in water, dry with anhydrous sodium sulfate, filter, concentra t under reduced pressure, and the residue is separated by silica gel column chromatography to obtain compound 1-30 (2.74g, yield 49%). 1H NMR (400 MHZ, DMSO-d6): δ 11.13 (BRS, 1 H), 8.37 (s, 1H), 7.45-7.65 (m, 3H), 7.07 (m, 1H), 5.14 (s, 2H), 4.92 (s, 2H); LC-MS(ESI): 56 1.1 [M+H]⁺.

### Embodiment 30 Preparation of the acetic acid coproduct (1:2, SHEN210) of Compound I-2

Suspend and dissolve I-2 (2.14g, 4.0 mmol) in acetic acid (10mL), heat to 100°C to compl ete dissolve, cool to room temperature (25°C), and stir for 3h. After filtration, water washing, et hanol washing and drying, 2.34g solid is obtained. 1H NMR (400MHz, Pyridine-d5) δ 8.33 (s, 1H), 7.98-7.90 (m, 1H), 7.89 (s, 1H), 7.81 (s, 1H), 7.21-7.17 (m, 2H), 5.61 (s, 2H), 5.57(s,2H), 3.99 (s,3H), 2.16 (s, 6H). The powder diffraction pattern of compound I-2 acetate (XPRD, As s hown in Figure 1) at the diffraction angles of (2θ) 5.4±0.2° , 9.9±0.2° , 10.7±0.2° , 12.6± 0.2° ,19.8±0.2° , 22.2±0.2° , 25.5±0.2° , 27.3±0.2° , 28.0±0.2° in the diffraction peak. In particular, diffraction peak at 5.4±0.2° , 19.8±0.2° , 22.2±0.2° and 25.5±0.2° is especially o bvious.

### Embodiment 31 Preparation of Compound I-2 fumaric acid coproduct (1:1, SHEN211)

Dissolve and suspend I-2 (2.14g, 4.0mmol) in ethyl acetate (20mL), add fumaric acid (0.49 g, 4.2 mmol) and stir at room temperature for 1h. Filter and dry, 2.34g solid is obtained.

¹H NMR (400 MHz, DMSO-d6) δ 8.35 (s, 1H), 8.30 (s, 1H), 7.71 (s, 1H), 7.48-7.64 (m, 2 H), 6.60 (s, 3H), 5.23 (s, 2H), 4.92 (s, 2H), 4.14 (s, 3H). ¹H NMR (400 MHz, Pyridine-d5) δ 8. 33 (s, 1H), 8.00-7.92 (m, 1H), 7.90 (s, 1H), 7.81 (s, 1H), 7.44 (s, 3H), 7.30-7.13 (m, 1H), 5.61 (s, 2H), 5.57 (s, 2H), 3.99 (s, 3H).

Dissolve I-2 (2.14g, 4.0 mmol) in ethyl acetate (20mL), add fumaric acid (0.49g, 4.2mmo), raise temperature and stir at 50°C for 3h. Cool, filter and dry, 2.34g solid is obtained.

I-2 fumaric acid coproduct powder diffraction pattern (XPRD, as shown in figure 2) has diffr action peaks at on the diffraction angle (2θ) 6.05±0.2° , 7.85 ±0.2° , 9.55±0.2° , 10.22±0. 2° , 11.02 ±0.2° , 11.60 ±0.2° , 12.01±0.2 ° , 12.39±0.2° , 13.43±0.2° , 13.88±0.2° , 1 4.79±0.2° , 15.21±0.2° , 16.41±0.2 ° , 17.19±0.2° , 18.13 ±0.2° , 18.71 ±0.2° , 19. 16±0.2° , 19.59±0.2° , 19.91±0.2° , 20.43±0.2° , 20.97±0.2° , 21.53±0.2° , 22.04±0. 2° , 22.70±0.2° , 23.16±0.2° , 23.60±0.2° , 23.85±0.2° , 24.76+0.2° , 25.47±0.2° , 27.1 3±0.2° , 27.77±0.2° , 28.28±0.2° , 28.95±0.2° , 29.76±0.2° , 31.17±0.2° , 32.11 ±0.2° , 32.64±0.2° , 33.34 soil 0.2,34.03±0.2° and 35.00±0.2.

Refer to embodiment S-217622 for the preparation of fumaric acid coproduct (1:1, CVL201)

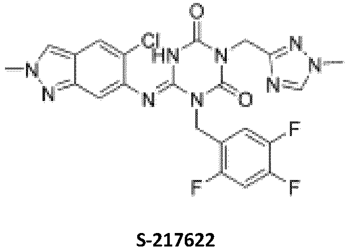

Dissolve S-217622 (2.14g, 4.0mmol) in ethyl acetate (20mL), add fumaric acid (0.49g, 4.2 mmo) and stir at room temperature for 1h. Filtered and dried, 2.34g solid was obtained. ¹H N MR (400MHz, Pyridine-d5) δ 3.64 (s, 3H), 3.98 (s, 3H),5.57 (s, 2H), 5.61 (s, 2H), 7.16-7.25 (m, 2H), 7.44 (s, 2H), 7.80 (s, 1H), 7.88 (s, 1H), 7.89-7.97 (m, 1H), 8.31 (s, 1H); Purity, 99. 0% (HPLC)

Dissolve S-217622 (2.14g, 4.0mmol) in ethyl acetate (20mL), add fumaric acid (0.49g, 4.2 mmo) and stir at 50°C for 3h. Cool, filter and dry, then 2.34g solid is obtained.

### Embodiment 32 Preparation of the acetic acid coproduct (1:2; CVL202) of S-217622

Suspend and dissolve S-217622 (2.14g, 4.0mmol) in acetic acid (10mL), heat to 100°C to complete dissolve, and cool to room temperature (25°C). Stir for 3h. Filter, wash with water, wa sh with ethanol and dry, 2.32g white solid is obtained. 1H NMR (400MHz, CDCI3), δ 7.98 (s, 1H), 7.89 (BRS, 1H), 7.80 (s, 2H), 7.41 (dd, 1H), 7.07 (s, 1H),6.92 (dd, 1H), 5.34 (s, 2H), 5.1 3 (s, 2H), 4.21 (s, 3H), 3.88 (s, 3H), 2.09 (s, 6H).

### Embodiment 32 Preparation of a ternary coproduct (1:1:1, SHEN212) of compound I-2

Dissolve I-2 (2.14g, 4.0 mmol) and niacinamide (0.51g, 4.18 mmol) in ethyl acetate) with f umaric acid (0.49g, 4.2mmol) at room temperature for 1h. After iltering, 2.84gr white solid is ob tained. 1H NMR (400 MHz, DMSO-d6) δ 13.12 (brs, 2H), 9.03 (s, 1H), 8.70 (d, J=4.0 Hz, 1H), 8.36 (s, 1H), 8.10-8.25 (m, 2H), 7.52-7.90 (m, 4H), 7.50 (dd, J-4.0, 8.0Hz, 1H), 6.63 (s, 3H), 5.22 (s, 2H), 4.92 (s, 2H), 4.15 (s, 3H). 1H NMR (400 MHz, Pyridine - d5), δ 9.71 (d, 1H), 9. 14 (BRS, 1H), 8.83 (dd, 1H),8.64 (BRS,1H), 8.59-8.53 (m, 1H), 8.32 (s, 1H), 7.99-7.90 (m, 1 H), 7.89 (s, 1H), 7.81 (s, 1H), 7.44 (s, 2H), 7.36 (DDD, 1H), 7.21-7.14 (m, 1H), 5.61 (s, 2H), 5.57 (s, 2H), 3.98 (s, 3H).

Or, dissolve I-2 (2.14g.4.0mmol) and niacinamide (0.51g, 4.18mmol ) in ethyl acetate, add fumaric acid (0.49g, 4.2mmo), stir at 50°C for 3h. Cool and filter to obtain 2.84g white solid.

Ternary coproduct powder Diffraction pattern (XPRD, as shown in Figure 3 below) of I-2 h ave diffraction peaks at 2-theta angles at 10.406, 11.188, 11.772, 12.202, 12.556, 13.589, 14.0 75, 14.973, 15.692, 17.37, 18.212, 18.464, 18.874, 19.34, 19.752, 20.104, 20.28, 20.59, 21.15 4, 21.743, 22.246, 22.536, 22.868, 23.342, 23.767, 24.003, 24.92, 25.641, 25.99, 27.589, 28.03 8, 28.859, 29.677, 29.911, 31.356, 32.779, 33.502, 35.14, 36.251 and 39.648±0.2° .

Ternary coproduct Powder Diffraction Pattern of I-2 (XPRD, As shown in figure 3) on the d iffraction Angle (2θ) 10.4±0.2°, 11.2±0.2°, 11.8±0.2°, 12.2+0.2°, 12.6±0.2°, 13.6±0.2°, 14.1± 0.2°, 15.0±0.2°, 15.7±0.2°, 17.4+0.2°, 18.2±0.2°, 18.5±0.2°, 18.9±0.2°, 19.3 ±0.2°, 19.8±0. 2°, 20.1 ±0.2°, 20.3±0.2°, 20.6+0.2°, 21.2±0.2°, 21.7±0.2°, 22.2±0.2° , 22.5±0.2°, 22.9±0. 2°, 23.3+0.2°, 23.8±0.2°, 24.0±0.2°, 24.9±0.2°, 25.6±0.2°, 26.0±0.2°, 27.6±0.2°, 28.0±0.2°, 28.9+0.2°, 29.7±0.2°, 29.9±0.2°, 31.4±0.2°, 32.8±0.2°, 33.5±0.2°, 35.1+0.2°, 36.3±0.2° an d 39.6±0.2° have diffraction peak.

Particularly, in the place of 11.2±0.2°, 15.7±0.2°, 18.9±0.2°, 19.3±0.2°, 22.9°+0.2°, 24.0 ±0.2°, 24.9±0.2°, 29.6±0.2° the diffraction peak is especially striking.

The beneficial effects of the compound of the invention are illustrated by the following effe ct experiments.

Effect Embodiment 1 Inhibition test of 3CL protease by some compounds of the invention.

The fluorescence resonance energy transfer method reported in the literature is adopted (J in et al .2020. Structure of Mprofrom SARS-CoV-2 and discovery of its inhibitors. Nature, 582: 289-293), the enzyme inhibitory activity of the compound is determined. The catalytic activity a nd initial rate of 3CL enzyme are determined by enzyme kinetics using commercially available f luorescence-labeled polypeptide MCA-AVLQSGFR-Lys (Dmp) -Lys-NH2 as substrate (GLBioche m, Shanghai). The incubation system contained 3CL protease of 2019-nCoV (0.2 µM), fluoresce ntly labeled peptides (20 µM) and a series of concentrations of compounds to be tested (0-20 µM). The fluorescence intensity of the system during incubation for 2-3 minutes is measured b y enzyme marker, and the excitation wavelength and detection wavelength are 320mm and 405 mm, respectively. According to the change rate of the initial velocity of substrateHydrolysis cata lyzed by enzyme after the addition of inhibitor, the enzyme inhibition rate of the tested substan ce at different concentrations was calculated. All experiments are repeated three times, and IC 50 values of inhibitory enzymes are calculated by Prism5 software. We determined the inhibitor y activities of some of the compounds described in embodiments 1-29 against the novel coron avirus 3CL protease, and the specific results are shown in Table 1.

**Table 1**

| Compound number | IC50(nM) |
|---|---|
| S-217622 | 18.9 |
| 1-2 | 14.9 |
| I-3 | 15.9 |
| I-4 | 14.3 |
| I-5 | 19.2 |
| I-24 | 20.1 |
| I-25 | 34 |
| I-26 | 29 |
| I-27 | 30 |
| I-28 | 18 |
| I-29 | 407 |
| I-30 | > 1000 |

S-217622 is selected as the positive control drug in the enzyme inhibitory activity experime nt. According to the above results, it can be seen that the inhibitory activity of some compoun ds in the invention, that is, the tritium substitute with specific structure in S-217622, on the 3C L proteolytic enzyme of SARS-CoV-2 novel coronavirus is equivalent to that of S-217622, and t he inhibitory activity of some deuterated compounds is significantly stronger than S217622.

### Effect Embodiment 2 Evaluation of Mpro activity of tested compounds against coronavirus i n vitro (SARS-CoV-2 wild-type WT, E166V mutant and Omicron P132H mutant Mpro protease)

The inhibitory activity of the tested compound against the novel coronavirus Mpco protease (SARS-CoV-2 wild type WT, E166V mutant and OmicromP132H mutant Mpro protease) is dete cted in vitro. Ensitrelvir (i.e. CVL201, lot No. Y62200-01) served as a positive control compoun d for the Mpeo protease test. The compound is tested at 10 concentrations, 3 times gradient d ilution, and 3 multiple pores. The initial test concentration of the tested compound is 5 µM, an d the compound is diluted for 10 concentration points, 3 times gradient dilution, and 3 multiple Holes, and add to the test plate.Coronavirus. Mpro protease (WT, Omicron P132H mutant, and SARS-CoV-2 E166V mutant) is added to the experimental plate containing the compound, and pre-incubated at room temperature for 30 minutes, and then reacted with the reaction substrate at 30°C for 60 minutes. The negative controlHole, which contains enzymes and substrates but no compounds, serves as a control without inhibition. The positive controlHole containing substr ate, enzyme and high concentration of positive control compound is used as the 100% inhibitio n control. The inhibitory activity of compounds against coronavirus Mpro protease is analyzed a nd calculated by GraphPad Prism software, and the results are shown in Table 2.

**Table 2**

| Compound number | Enzymatic Determination of IC50 (nM) of compounds by Mpro | | |
|---|---|---|---|
| | SARS-CoV-2 WT | SARS-CoV-2 WT | SARS-CoV-2 |
| | | E166V mutant | Omicron P132Hmutant |
| SHEN211 | 25 | 1354 | 51 |
| CVL201 | 27 | 1467 | 55 |
| PF-07321332 | 18 | 36730 | 30 |

The results show that SHEN211 have broad-spectrum coronavirus Mpro protease inhibitory activity, which is similar to CVL201. In addition, the 3CL protein resistance mutation E166V aga inst Nematavir (PF-07321332) also shows enzyme inhibitory activity, which is far better than Fe rri Nematavir, more than 27 times. It is suggested that SHEN211 has the potential of effective treatment of nematovir resistance.

### Effect Embodiment 3: Mpro activity of the tested compound against other human coronavir uses (6 strains of virus Mpro, 1 experiment)

The inhibitory activity of the tested compounds against coronavirus Mpro protease is detect ed by enzyme assay in vitro. Test for Mpro protease can be seen in Table 3. Ensitrelvir and P F-07321332 are used as positive control compounds for Mpro protease tests. The compound is tested at 10 concentrations, 3 times gradient dilution, and 3 multiple pores.10.The initial test c ioncentration of the tested compound s 30uM. The compound will be diluted at 10 concentratio n points, with a triple gradient dilution. The coronavirus Mpro protease (Table 3) is added to th e experimental plate containing the compound, which is incubated at room temperature for 30 minutes, and then the reaction substrate is added for 60 minutes at 30°C. The negative control Hole contained the enzyme and substrate but do not contain the compound, as the control wit hout inhibition. The positive controlHole contains substrates, enzymes, and a high concentration of positive control compounds as a 100% inhibition control. Fluorescent readings are detected with a multifunctional ELISA reading board. The inhibitory activity of compounds against corona virus Mpro protease is analyzed and calculated by GraphPad Prism software, and the results a re shown in Table 3.

**Table 3**

| Compound number | Enzymatic Determination of IC50 (*µ* M) of compound by Mpro | | | | | |
|---|---|---|---|---|---|---|
| | SARS | MERS | 229E | OC43 | HKU1 | NL63 |
| SHEN211 | 0.045 | 4.671 | 6010 | 0.133 | 0.038 | 25.98 |
| CVL201 | 0.043 | 4959 | 5.627 | 0.134 | 0.044 | 27.79 |
| GC-376 | 0.040 | 0.274 | 0.100 | 0.042 | 0.022 | 0.107 |

The results suggest that SHEN211 has broad-spectrum anti-coronavirus activity, which issi milar to CVL201 (S-217622 fumaric acid coproduct) and has inhibitory activity against other hu man coronaviruses except novel coronavirus.

### Effect Embodiment 4 Evaluation of the anti-SARS-CoV-2 activity of the tested compound (1 replicator model, 2 conditions (with or without physiological concentration of HSA+AAG), 1 exp eriment)

The in vitro anti-SARS-CoV-2 activity of the tested compounds is evaluated using the SAR S-CoV-2 replicator model, with Remdesivir and EIDD-1931 as control compounds. The activity of the tested compounds is evaluated in the presence of HSA and AAG without or with biologi cal concentrations. The compound is tested at 8 concentrations, 3 times gradient dilution, and 3 times multiple pores. After the initial test concentration of the tested compound is 1 *µ* MSARS -CoV-2 replicon RNA is electrocuted intoHuh7 cells and inoculated into microplates containing t he compound with double dilution at a certain density. HPE control is set up (cells with SARS-CoV-2 replicons transferred by electric transmission without chemical compound treatment), and the cells are cultured in 5% CO2 and 37°C for 1 day, and the number of GFP expressing cell s in each well is detected. The cytotoxicity test is the same as the antiviral test. Cell viability i s measured using cell viability assay kit CellTiter Glo (Promega). The antiviral activity and cytot oxicity of the compound are calculated by the effect of the compound at different concentration s on the changes in pseudoviral reporter gene expression and cell viability, respectively. The n eutralization activity and cell viability of the samples are analyzed using GraphPad Prism with nonlinear fitting, and the EC50 and CC50 values of the compounds are calculated in the prese nce of HSA and AAG without or with biological concentrations, as shown in Table 4.

**Table 4**

| Compound number | NoHSA+AAG | | HSA (45 mg/mL) + AAG (0.7 mg/mL) | | EC50 Shift |
|---|---|---|---|---|---|
| | EC50 (microns) | CC50 (microns) | EC50 (microns) | CC50 (microns) | |
| SHEN211 | 0.054 | >1 | 0.874 | > 1 | 16.33 |
| Remdesivir | 0.0076 | >1 | 0.123 | > 1 | 16.29 |
| EIDD-1931 | 1.578 | > 10 | 1437 | > 10 | 0.91 |
| CVL201 | 0.051 | >1 | 0.877 | > 1 | 17.13 |

The results show that in the presence of biological concentration HAS+AAG, the antiv iral activity of SHEN211 and CVL201 decreased by 16 times and 17 times, which are sim ilar.

### Effect Embodiments 5 Evaluation of cytotoxicity of tested compounds (9 cell lines, 1 experiment)

Nine cell lines originated from different human tissues (Table 2) are selected to evalu ate the cytotoxicity of the tested compounds. Sorafenib or Taxol are used as cytotoxic co ntrol compounds. The cells and compounds are added to the 96-well test plate. Cells are cultured in culture medium containing 10% FBS, 5% CO2 and 37°C for 3 days, and then cell viability was detected by CellTiter-Glo reagent. The raw data are used for compound cytotoxicity calculations. The dose-response curves of the compounds are analyzed by Gr aphPad Prism software and CC50 values are calculated. The compound will be tested at 8 concentration points, 3-fold series dilution, and 3-compound pores. The initial test conce ntration of the tested compound is 100 µM, and the results are shown in Table 5.

**Table 5**

| Compound knitting No. | CC50(uM) | | | | | | | | |
|---|---|---|---|---|---|---|---|---|---|
| | Colo-205 | HepG2 | HEK293 | M RC-5 | A375 | Molt-4 | HEp2 | HIHeLa | CCRF-CEM |
| CVL201 | > 100 | > 100 | > 100 | > 100 | > 100 | > 100 | > 100 | > 100 | > 100 |
| SHEN211 | > 100 | > 100 | > 100 | > 100 | > 100 | > 100 | > 100 | > 100 | > 100 |
| Sorafenib | 8.725 | 5.324 | 5.578 | 13.82 | ND | ND | ND | ND | ND |
| Pacitaxel | ND | ND | ND | ND | 0.0089 | 0.0027 | 0.030 | 0.020 | 0.0034 |

The results shows that SHEN211 had no cytotoxic problem to 9 kinds of human cell s.

### Effect Example 6 Mitochondrial toxicity evaluation of the tested compound (HepG2 glu cose/galactose test, 1 experiment)

In vitro mitochondrial toxicity of the tested samples will be determined by HepG2 gluc ose/galactose assay. The tested compound is SHEN211. Rotenone as a control compoun d. The compound is tested at 9 concentrations, 3 times gradient dilution, and 3 multiple p ores. The initial concentration of tested compounds is 500 µ M. HepG2 cells cultured with glucose and HepG2 cells cultured with galactose are inoculated in microplates at a certain density and cultured overnight in a 5% CO2 incubator at 37°C.The next day, the compou nd is added with a double dilution ratio. Cell control group and compound test group are set up. The cells are cultured in a 5% CO2 incubator at 37°C for 24h. Cell viability is me asured using CellTiter Glo, a cell activity assay, and the raw data are used to evaluate th e mitochondrial toxicity of the compounds. The dose-response curve of the compound is a nalyzed by GraphPad Prism software and the CC50 value is calculated. The results are s hown in Table 6.

**Table 6**

| Compound number | G glucose culture HepG2 | G galactose culture HepG2 | R ratio (CC50 Glu/CC50 Gal) |
|---|---|---|---|
| | Ab CC50(*µ* M) | Ab CC50(*µ* M) | |
| SHEN211 | 202.33 | 206.28 | 0.98 * |
| Rotenone | 2.33 | 0.03 | 91.7 |

The results show that the mitochondrial toxicity of SHEN211 to human HepG2 cells is more than 200 *µ* M. The difference between the CC50 and EC50 (0.084-0.210 µ M) of S ARS-CoV-2 strain is more than 476 times, suggesting that there is no problem of mitocho ndrial toxicity to human.

Effect Example 7 Study on liver particle metabolism of some compounds of the inven tion.

Kunming mouse liver microsomes (IPHASE/Huizhi taikang) are prepared by ultrafast c entrifugal method. Fresh mouse liver is weighed, crushed in Tris-HC1 buffer liquid with 3 t imes the volume, and then Homogenized with Homogenizer. The above operations are all carried out in an ice bath below 4°C, and the Homogenate is centrifugated at 7000g and 4°C for 20 minutes. The upper suspension is taken at 10000g and centrifuged at 4°C for 30 min. The supernatant is discarded and precipitated into mouse liver microsomes, which are prepared into suspension in 0.25mol/L sucrose solution and preserved in liquid nitrog en. The protein content of mouse liver microsome is 7.8mg/mL by Lowry method.

Mouse liver microsomes are composed of a warm incubation system in vitro. The fin al volume of the warm incubation system is 5ml, containing mouse liver microsomes 2.0m g/mL, glucose 6-phosphate 0.01mmol/mL, G6-PDH 1U/mL, magnesium chloride 4.0umol/m L and NADPO.5 umol per mL, NADH 1. Oumol/mL is mixed and shaken well, and then o scillated in a water bath at 37°C, two parts of each sample are prepared, and the tested substance is added to the mouse liver microsome enzyme incubation solution, so that the concentration of the tested substance is 50mg/L, fully oscillated, and incubated at 37°C. A t the same time, blank control experiments are performed with the heated inactivated liver Homogenate. Oxygen is given to the surface of the incubation solution every 0.5 hour for 1 minute, and 0.5mL of the sample is taken at 0, 5, 15, 30 and 60 minutes, respectively. At that time, 3 times the volume of acetonitrile is added to terminate the metabolic reacti on, and the metabolic clearance rate and half-life are calculated, as shown in Table 7.

**Table 7**

| Compound number | species | k | Tic (min) | Cla (ml/min/mg) | Clape (ml/min/kg) | Cl (mL/min/kg) | Eₕ(%) |
|---|---|---|---|---|---|---|---|
| S-217622 | rat | -0.0002 | ∞ | 0.0000 | 0.0 | 0.0 | 0.0 |
| I-2 | rat | -0.0012 | ∞ | 0.0000 | 0.0 | 0.0 | 0.0 |
| I-3 | rat | -0.0002 | ∞ | 0.0000 | 0.0 | 0.0 | 0.0 |
| I-5 | rat | -0.0005 | ∞ | 0.0000 | 0.0 | 0.0 | 0.0 |
| Testosterone | rat | 1.2226 | 0.6 | 6.1128 | 11003.1 | 54.7 | 99.5 |

According to the metabolic test results of mouse liver particles, it can be seen that d euterium-substituted triazine derivatives at different positions of the invention are basically not metabolized in mouse liver particles, and there is no significant difference compared w ith S-217622.

Effect Example 7 Study on Hepatocyte metabolism of some compound of the inventio n

### 1. Preparation of solution

The compound to be tested is configured as a DMSO reserve solution at a concentr ation of 10mM, and then the reserve solution of the compound to be tested is diluted to a 200uM solution with acetonitrile.

### 2. Liver cell body incubation

A incubation mixture with a total volume of 200 uL is prepared with the following final component concentrations: william's E medium, liver cells (1 million/mL) and test compou nd or positive control (0.5 µ M). After pre-incubating all other components in a 37° C±5% CO2 incubator for 10 minutes, the compound is added. Mix with a pipette to obtain a unif orm suspension, and immediately transfer the sample incubated at 20 µ L for 0 minutes int o the Hole of the "quenched" plate, then mix with a pipette. At 15, 30, 60, 120, and 240 minutes, the cultures are mixed with pipettes, and at each time point samples of the 20L culture are successively transferred to Holes in separate "quenched" plates, which are the n mixed with pipettes. 200 µ L acetonitrile containing IS is added to the "quenched" plate, and the results are shown in Table 8.

**Table 8**

| S species | L Liver weight (g/kg BW) | Liver blood flow (mL/min/kg) | S-scale factor (million cells/g liver wt.) |
|---|---|---|---|
| rat | 40 | 55 | 117 |
| H people | 26 | 21 | 139 |

### 3. Sample analysis

Centrifuge the 96-well plate at 4000rpm for 10 minutes. The 50uL supernatant is mix ed with 50uL deionized water and then injected into the LC-MS/MS system for analysis. A fter data processing, parameters such as Clᵢₙₜ, Clₐₚₚ, Clₕ and Eh, etc. are calculated, and the results are shown in Table 9.

**Table 9**

| Compound number | species | k | T_{1/2}(min) | Clᵢₙₜ (mL/min/million cell) | Clapp (mL/min) | Clₕ (mL/min) | Eₕ(%) |
|---|---|---|---|---|---|---|---|
| S-217622 | rat | 0.0008 | 888.1 | 0.0008 | 3.7 | 3.4 | 6.2 |
| | people | 0.0001 | 5909.4 | 0.0001 | 0.4 | 0.4 | 2.0 |
| I-2 | rat | 0.0001 | 5337.3 | 0.0001 | 0.6 | 0.6 | 1.1 |
| | people | 0.0006 | 0 | 0.0000 | 0.0 | 0.0 | 0.0 |
| I-3 | rat | 0.0014 | 496.7 | 0.0014 | 6.5 | 5.8 | 10.6 |
| | people | 0.0001 | 12226.5 | 0.0001 | 0.2 | 0.2 | 1.0 |
| I-5 | rat | 0.0008 | 833.5 | 0.0008 | 3.9 | 3.6 | 6.6 |
| | people | 0.0004 | 1794.4 | 0.0004 | 1.4 | 13 | 6.2 |

According to the results of liver cell metabolism test, it can be seen that compared w ith S-217622, the metabolic clearance rate of the deuterated triazine derivative in different positions of the invention is significantly reduced, and the metabolic half-life of S-217622 i s prolonged after deuteration. Based on maintaining the effectiveness against SARS-CoV-2, the metabolic half-life of S-217622 is prolonged. The half-life is significantly prolonged, t he demand for dosage is reduced, side effects are reduced, and the therapeutic window r ange is expanded. Therefore, the invention has a very good prospect for making drugs fo r treating diseases related to coronavirus infection.

### Effect Embodiment 9 in vivo pharmacokinetic study of some compound of the inventio n

### 1. Preparation of test products

IV:5%DMSO+95%PG
PO: 5% DMSO+95% MC (0.5%)
G10: CVL201: nicotinamide mass ratio is 5.3:1, for example, weigh 6.09mg (5*1.218) CVL201, then weigh 1.149mg (6.09mg/5.3) nicotinamide, and then use 5ml of solvent to p repare a suspension.

### 2. Experimental animals

Species: Healthy male SD (Sprague Dawley) rats (SPF class), weighing 180-220g.
Source: Laboratory Animal Management Department, Shanghai Institute of Family Pla nning Science, animals were transferred from animal reserve of experimental institutions (9 99M-017).
Number: 30 males
Animal selection: No random grouping

### 3. Drug administration and blood collection time

Weigh before dosing, calculate dosage according to body weight. The drug is adminis tered intravenously or by appetite irrigation.

The drug is administrated by intragastric administration (5mg/kg), and 0.2ml of blood i s collected from the jugular vein of rats at 0.25h, 0.5h, 1h, 2h, 4h, 6h, 8h and 24h after administration, and plasma is separated and prepared. The concentration of compounds in plasma is determined by LC-MS/MS.

The drug is administered intravenously (0.5mg/kg), and 0.2ml of blood is collected fro m the jugular vein of rats at 0.033h, 0.25h, 0.5h, 1h, 2h, 4h, 8h, and 24h after administr ation, with EDTA-K2 anticoagulant and placed on ice.

### 5. Plasma sample treatment

The blood samples are collected and placed on ice and the plasma is centrifuged wit hin 1 hour (centrifugation condition: 6800g, 6 minutes, 2-8°C). Plasma samples are stored in -80°C refrigerator before analysis.

### 6. Sample analysis

### 1) Sample preparation for LC-MS/MS determination:

S-217622 and 1-5: Protein precipitation of 40 µL plasma sample with 400 µ L methano I containing 10ng/mLIS (IS Verapamil, Verapamil), swirl mixture for 1 min, then centrifuge at 18000g for 7 min. The 400 µ L supernatant is transferred to the 96-well plate. LC-MS/M S analysis is performed with 1 µ L supernatant.

1-2: Protein precipitation is performed on a 20uL plasma sample with 400 µ L methan ol containing 10ng/mL IS (IS is Verapamil), the mixture is vortex for 1 min, then centrifug ed at 18000g for 7 min, and 300 µ L supernatant is transferred to a 96-well plate.LC-MS/ MS analysis is performed with 8 µ L supernatant.

### 2) LC-MS/MS analysis method:

Mobile phase A: 0.1% formic acid aqueous solution.

Mobile phase B: 0.1% ethyl formate solution:
Pillar: ACQUITY UPLC BEH C18 1.7 µ m 2.1*50mm:
Flow rate: 0.60mL/min.
Column temperature: 40°C

The gradient elution procedure is shown in Table 10:

**Table 10**

| S-217622 | | I-2 | | I-24 | |
|---|---|---|---|---|---|
| T time (min) | M Mobile phase B (%) | T time (min) | M Mobile phase B (%) | T time (min) | M Mobile phase B (%) |
| 1 Initial value | 10 | 0.01 | 10 | Initial | 10 |
| 0.60 | 90 | 0.60 | 90 | 0.60 | 90 |
| 1.20 | 90 | 1.10 | 90 | 1.20 | 90 |
| 1.21 | 10 | 1.11 | 10 | 1.21 | 10 |
| 1.50 | 10 | 1.40 | Stop | 1.50 | 10 |

S-217622 peak time: 0.69min: MS: Q1/Q3 Masses: 532.10/394.00 Da Peak time: 0.63min: MS: Q1/Q3 Masses: 455.30/165.20 Da
I-2 Peak time: 0.86min: MS: Q1/Q3 Masses: 535.10/286.00 Da Peak time marked inside: 0.82min: MS: Q1/Q3 Masses: 455.30/165.20 Da
1-24 Peak time: 0.69min; MS: Q1/Q3 Masses: 538.10/285.90 Da Peak time: 0.63min: MS: Q1/Q3 Masses: 455.30/165.20 Da.

### 3) Result analysis

Through the blood concentration data at different time points, the pharmacokinetic par ameters are calculated by Phoenix WinNonlin7.0 non-atrioventricular model, and the param eters such as AUC_{0-∞}, Cₘₐₓ, Tₘₐₓ and T_{1/2} and their mean values and standard deviations are provided. The results are shown in Figure 11 below.

**Table 11**

| compound | administration route | Administration dose | T_{1/2} | Tmax | Cmax | AUC_{0-∞} |
|---|---|---|---|---|---|---|
| | | (mg/kg) | (h) | (h) | (ng/mL) | (h*ng/mL) |
| S-217622 | IV | 0.5 | 2.70 | 0.03 | 2307.23 | 4516.78 |
| S-217622 | PO | 5.0 | 2.80 | 3.33 | 4156.41 | 45240.04 |
| CVL201 (S-217622 fumaric acid coproduct) | PO | 5.0 | 2.89 | 4.67 | 4516.47 | 53851.04 |
| CVL201+ nicotinamide | PO | 5.0 | 2.64 | 3.00 | 4851.48 | 49816.19 |
| I-2 | IV | 0.5 | 3.36 | 0.03 | 2882.80 | 7943.98 |
| I- 2 | PO | 5.0 | 3.04 | 2.33 | 6019.80 | 65325.11 |
| SHEN211 (I-2 fumaric acid coproduct) | PO | 5.0 | 3.22 | 2.00 | 4710.93 | 45466.17 |
| SHEN212 (Ternary products) (I-2 ternary coproduct) | PO | 5.0 | 3.19 | 3.67 | 5969.50 | 62126.14 |
| 1-19 (Prodrug) | PO | 5.0 | 2.91 | 3.33 | 6182.77 | 70850.41 |
| 1-24 | PO | 5.0 | 3.51 | 4.00 | 5211.96 | 58251.56 |

The experimental results show that compared with S-217622, deuterium compound I-2 provide 125% Cₘₐₓ, increases 198% AUC, extends 124% T_{1/2}, increases 145% Cₘₐₓ after oral administration, increases 144% AUC, and increases 149% Cₘₐₓ after prodrug modificat ion, the AUC increased by 156%. Compared with S-217 fumaric acid eutectic group, Cₘₐₓ and AUC increased by 132% and 115% after administration of 1-2 ternary coproduct PO, Cₘₐₓ increased by 123% and AUC increased by 124% after administration of 1-2 ternary coproduct PO compared with S-217622 fumaric acid coproduct combined with niacinamide group. T_{1/2} is extended by 121%. It can be seen that the deuterium modification I-2 of S-217622 can significantly increase blood drug concentration and prolong metabolic half-life on the basis of retaining the effectiveness against SARS-CoV-2, which helps to reduce th e used dose, reduce side effects, and expand the therapeutic window. Therefore, the inve ntion has a very good prospect for preparing drugs for treating diseases related to corona virus infection.

The above embodiments are only examples for clear illustration and are not limitations on embodiments. For ordinary technicians in the field, other changes or changes in different forms can be made on the basis of the above description. It is not necessary and cannot be exhaus tive here. The obvious changes or alterations induced by this mountain are still within the scop e of protection of the invention.

Although the specific embodiments of the invention are described above, it should be unde rstood by those skilled in the art that these embodiments are only examples and that a variety of changes or modifications may be made to them without deviating from the principle and su bstance of the invention. Therefore, the scope of protection of the invention is limited by the at tached claims.

## Claims

1. A triazine derivative shown in formula (I') or a pharmaceutically acceptable salt thereof, 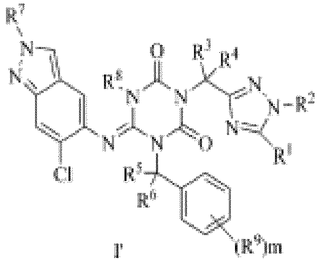
Where R' is Hydrogen or deuterium.
R2 is a methyl group, or a methyl group replaced by 1, 2 or 3 tritium;
R³ is hydrogen or deuterium,
R⁴ is hydrogen or deuterium,
R⁵ is hydrogen or deuterium,
R⁶ is hydrogen or deuterium,
R⁷ is a methyl group or a methyl group replaced by 1, 2, or 3 tritium,
R8 is Hydrogen, 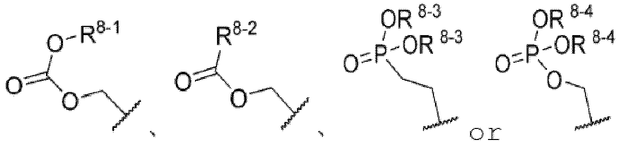
R⁸⁻¹ is C₁-C₁₀ alkyl,
R⁸⁻² is C₁-C₁₀ alkyl,
Each R⁸⁻³ is independently hydrogen, C₁-C₁₀ alkyl, or sodium,
Each R⁸⁻⁴ is independently hydrogen, C₁-C₁₀ alkyl, or sodium,
Each R9 is independently hydrogen or halogen,
m is 2, 3, 4 or 5.
Moreover, the compound shown in formula I' satisfies one or both of the following conditions:
(1) R¹, R², R³, R⁴, R⁵, R⁶ and R⁷ contain at least one deuterium:
(2) R8 is

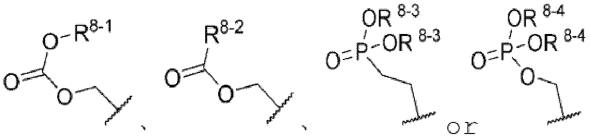

2. A triazine derivative, or a pharmaceutically acceptable salt thereof, as described in formula (I') of claim 1, is **characterized by** the fact that a triazine derivative as described in formula (I') satisfies one or more of the following conditions:
(1) In R⁸⁻¹, the mentioned C₁-C₁₀ alkyl group is C₁~C₆ alkyl groups, preferably methyl, ethyl, n-propyl, isopropyl, n-butyl, isobutyl, sec-butyl or tert-butyl groups, such as methyl, ethyl, isopropyl.
(2) In R⁸⁻², the mentioned C₁-C₁₀ alkyl group is C₁~C₆ alkyl groups, preferably methyl, ethyl, n-propyl, isopropyl, n-butyl, isobutyl, sec-butyl or tert-butyl, such as methyl, ethyl, isopropyl, n-butyl or tert-butyl.
(3) In R⁸⁻³, the mentioned C₁-C₁₀ alkyl group is C₁~C₆ alkyl groups, preferably methyl, ethyl, n-propyl, isopropyl, n-butyl, isobutyl, sec-butyl or tert-butyl.
(4) In R⁸⁻⁴, the mentioned C₁-C₁₀ alkyl group is C₁~C₆ alkyl groups, preferably methyl, ethyl, n-propyl, isopropyl, n-butyl, isobutyl, sec-butyl or tert-butyl.
(5) When R⁸ is Hydrogen, the three classes of derivatives shown in the formula (I') may also be tautomers of them, e.g. 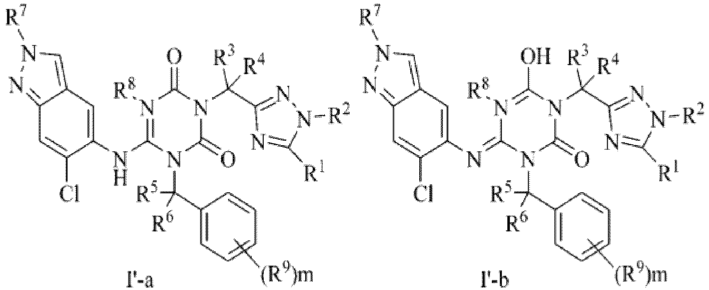 And (6) R⁹, the Halogens are neon, chlorine, bromine, or iodine, such as fluorine.

3. A triazine derivative, or a pharmaceutically acceptable salt thereof, as shown in formula I') of claim 1, is **characterized by** the fact that a triazine derivative as shown in formula (I') satisfies one or more of the following conditions:
(1) R² is methyl or -CDs.
(2) R⁷ is methyl or -CDs.
(3) R⁸⁻¹ is C₁~C₆ alkyl.
(4) R⁸⁻² is C₁~C₆ alkyl.
(5) R⁸⁻³ is sodium.
(6) R⁸⁻⁴ is sodium.
(7) both R³and R⁴ are deuterium.
(8) both R⁵ and R⁶ are deuterium.
(9) R⁹ is fluorine:
And (10) m is 2 or 3.

4. A triazine derivative or a pharmaceutically acceptable salt thereof as indicated in formula (I) of any of the claims 1-3 is **characterized by** the fact that the triazine derivative as indicated in formula (I) satisfies one or both of the following conditions.
(1) R⁸ is Hydrogen, 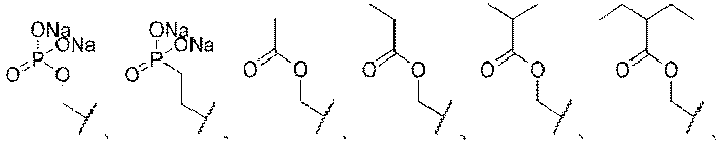 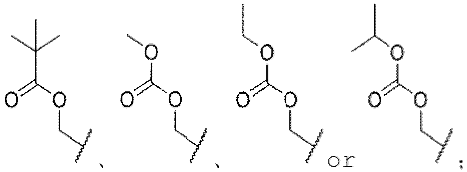 and (2) 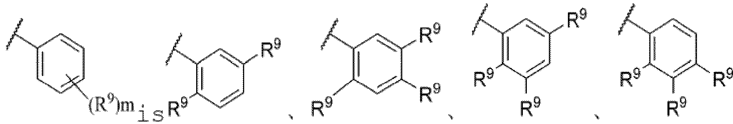 or

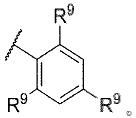

5. A triazine derivative or a pharmaceutically acceptable salt thereof as indicated in formula (I') of claim 1, characterized that the triazine derivative as indicated in formula (I') is option 1, option 2, option 3, option 4, option 5 or option 6,
Option 1.
R¹ is deuterium, R² is methyl, R³ is Hydrogen, R⁴ is Hydrogen, R5 is Hydrogen. R⁶ is Hydrogen,
R⁷ is methyl group, R8 is Hydrogen, 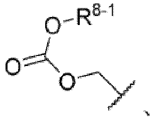 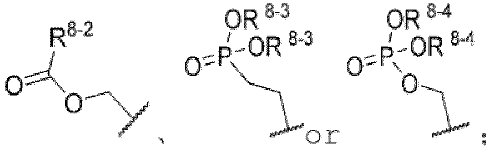
R⁸⁻¹ is C₁~C₆ alkyl, R⁸⁻² is C₁~C₆ alkyl, Each R⁸⁻³ is independently hydrogen, C₁~C₆ alkyl or sodium,
Each R⁸⁻⁴ is independently hydrogen, C₁~C₆ alkyl or sodium, each R⁹ is independently hydrogen or halogen, m is 2, 3, 4, or 5.
Option 2
R' is deuterium and R² is -CD₃; R³ is hydrogen; R⁴ is hydrogen; R⁵ is hydrogen; R⁶ is hydrogen; R⁷ is methyl or -CD₃; R⁸ is hydrogen, 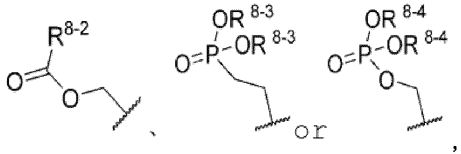
R⁸⁻¹ is C₁~C₆ alkyl, R⁸⁻² is C₁~C₆ alkyl.
Each R⁸⁻³ is independently hydrogen, C₁~C₆ alkyl or sodium, Each R⁸⁻⁴ is independently hydrogen, C₁-C₆ alkyl or sodium, each R⁹ is independently hydrogen or halogen, m is 2, 3, 4, or 5.
Better, in one scheme, R' is deuterium and R² is -CD₃; R³ is hydrogen; R⁴ is hydrogen; R⁵ is hydrogen; R⁶ is hydrogen; R⁷ is methyl or-CD₃; R⁸ is hydrogen, 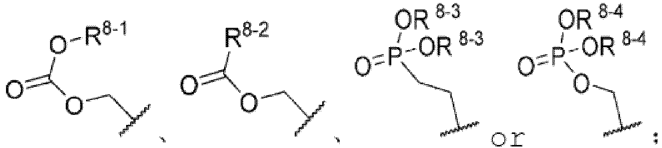 R8-1 is C1-C6 alkyl, R8-2 is C1-C6 alkyl. Each R8-3 is independently hydrogen, C1-C6 alkyl o r sodium, Each R8-4 is independently hydrogen, C1-C6 alkyl or sodium. R9 is fluorine, 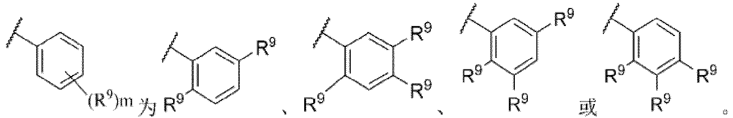
Option 3
R' is hydrogen and R² is methyl; R³ is tritium; R⁴ is tritium; R⁵ is hydrogen; R⁶ is hydrogen; R ⁷ is methyl; R⁸ is hydrogen, 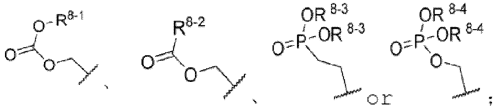
R⁸⁻¹ is C₁~C₆ alkyl, R⁸⁻² is C₁~C₆ alkyl. Each R⁸⁻³ is independently hydrogen, C₁~C₆ alkyl or sodiu m, Each R⁸⁻⁴ is independently hydrogen, C₁~C₆ alkyl or sodium, each R⁹ is independently hydro gen or halogen. m is 2, 3, 4, or 5.
Option 4
R' is hydrogen and R² is methyl; R³ is hydrogen; R⁴ is hydrogen; R⁵ is tritium; R⁶ is tritium; R ⁷ is methyl; R⁸ is hydrogen, 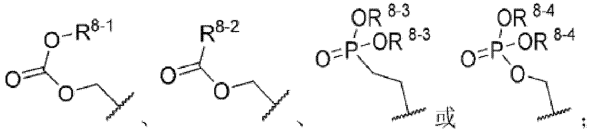
R8-1 is C1-C6 alkyl, R8-2 is C1-C6 alkyl. Each R8-3 is independently hydrogen, C1-C6 alkyl o r sodium, Each R8-4 is independently hydrogen, C1-C6 alkyl or sodium, each R9 is independe ntly hydrogen or halogen. m is 2, 3, 4, or 5.
Option 5
R' is hydrogen and R² is methyl or -CD₃; R³ is hydrogen; R⁴ is hydrogen; R⁵ is hydrogen;
R⁶ is hydrogen; R⁷ is -CDs; R⁸ is hydrogen, 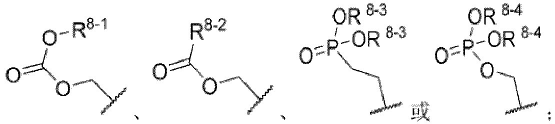
R⁸⁻¹ is C₁~C₆ alkyl, R⁸⁻² is C₁~C₆ alkyl.
Each R⁸⁻³ is independently hydrogen, C₁~C₆ alkyl or sodium, Each R⁸⁻⁴ is independently hydrogen, C₁-C₆ alkyl or sodium, each R⁹ is independently hydrogen or halogen, m is 2, 3, 4, or 5.
Option 6
R' is hydrogen or deuterium, R² is methyl or -CD₃; R³ is hydrogen or deuterium; R⁴ is hy drogen or deuterium; R⁵ is hydrogen or deuterium; R⁶ is hydrogen or deuterium; R⁷ is methyl or -CD₃; R⁸ is 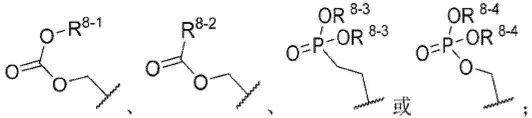
R⁸⁻¹ is C₁~C₆ alkyl, R⁸⁻² is C₁~C₆ alkyl.
Each R⁸⁻³ is independently hydrogen, C₁~C₆ alkyl or sodium, Each R⁸⁻⁴ is independently hydrogen, C₁-C₆ alkyl or sodium, each R⁹ is independently hydrogen or halogen, m is 2, 3, 4, or 5.
Better, R' is hydrogen or deuterium, R² is methyl or -CD₃; R³ is hydrogen or deuterium; R ⁴ is hydrogen or deuterium; R⁵ is hydrogen or deuterium; R⁶ is hydrogen or deuterium; R⁷ is methyl or -CD₃; R⁸ is 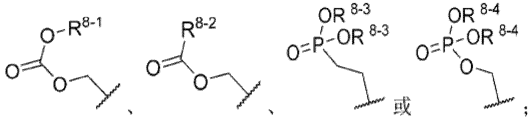 R⁸⁻¹ is C₁~C₆ alkyl, R⁸⁻² is C₁~C₆ alkyl.
Each R⁸⁻³ is independently hydrogen, C₁~C₆ alkyl or sodium, Each R⁸⁻⁴ is independently hy drogen, C₁~C₆ alkyl or sodium, R⁹ is fluorine,

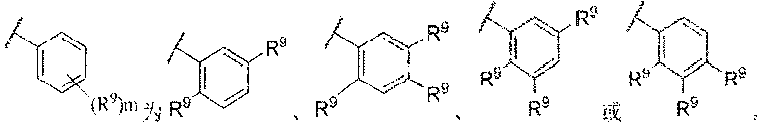

6. A triazine derivative or a pharmaceutically acceptable salt thereof as descri bed in formula (I') of claim 1, **characterized by** the formula
(1) The triazine derivatives shown are any of the following compounds:

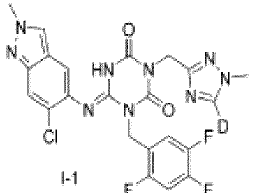

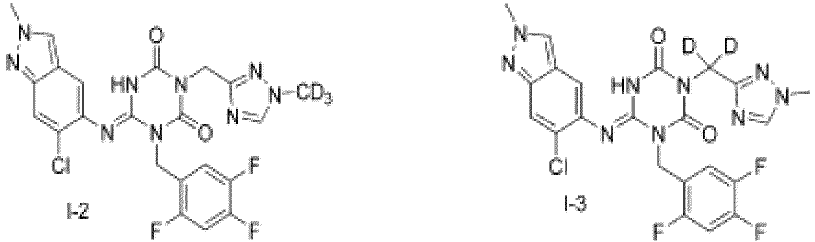

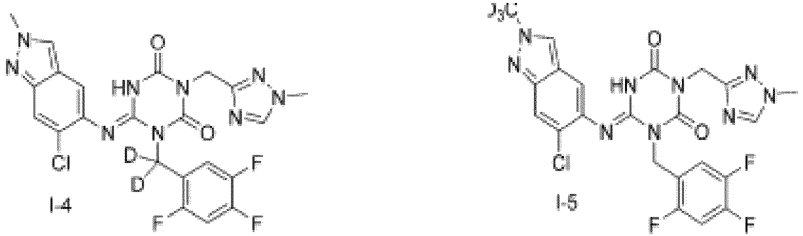

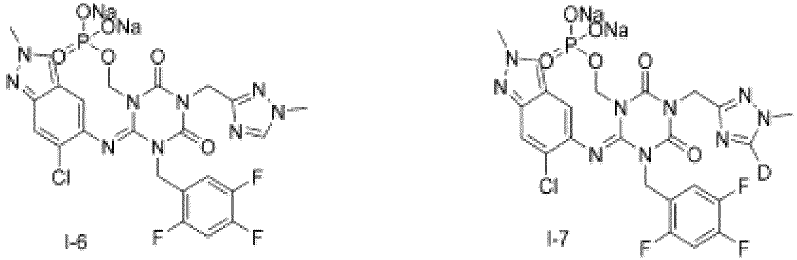

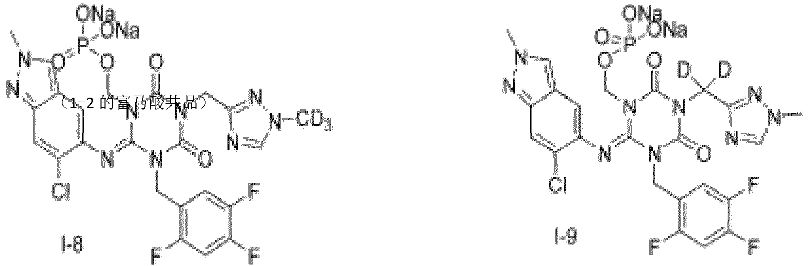

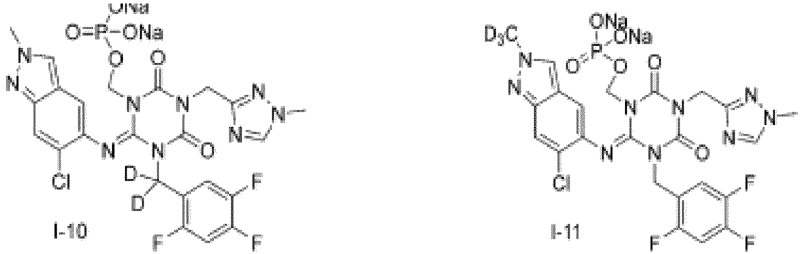

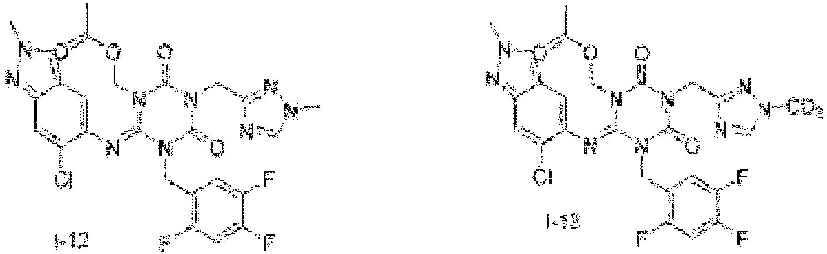

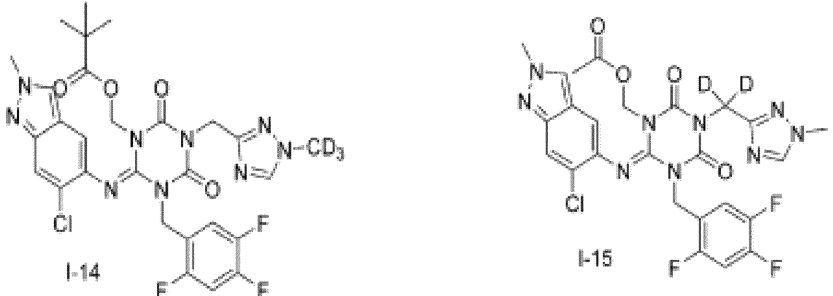

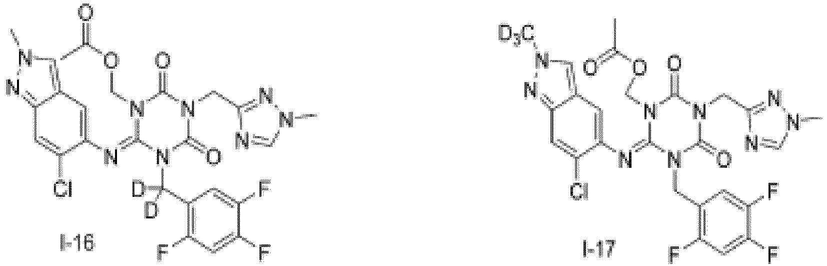

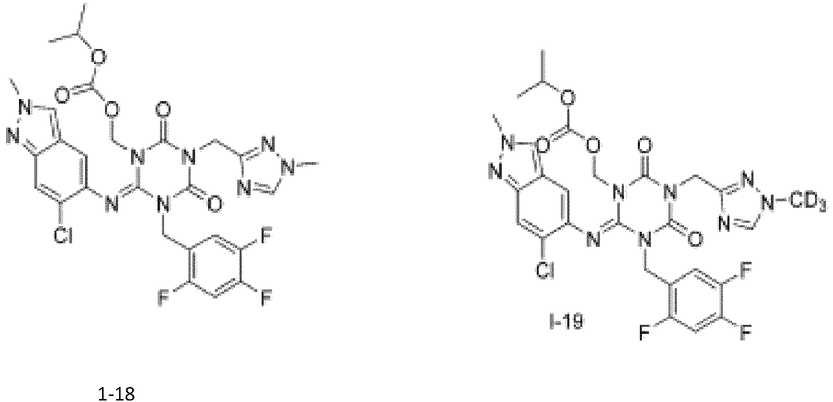

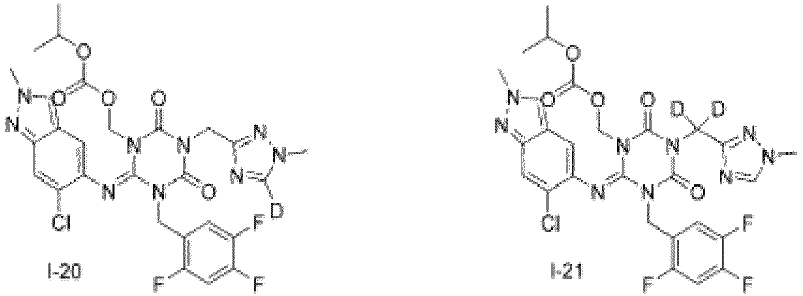

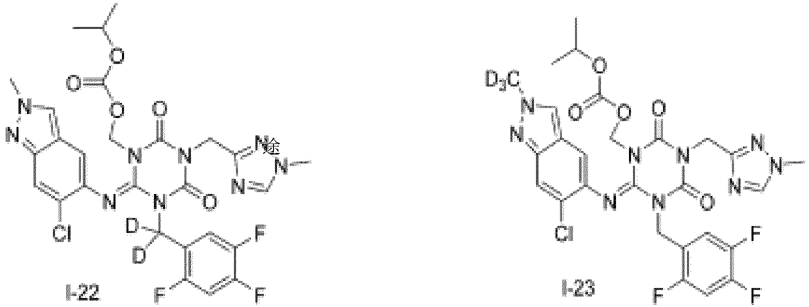

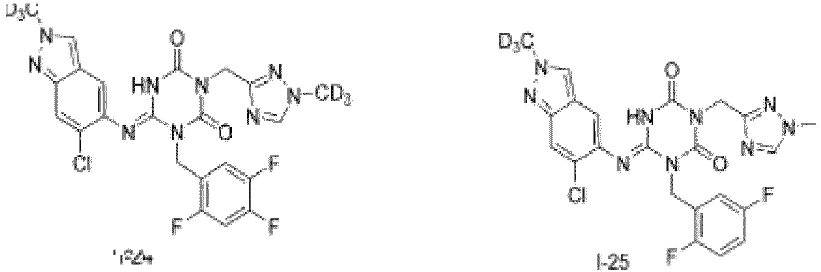

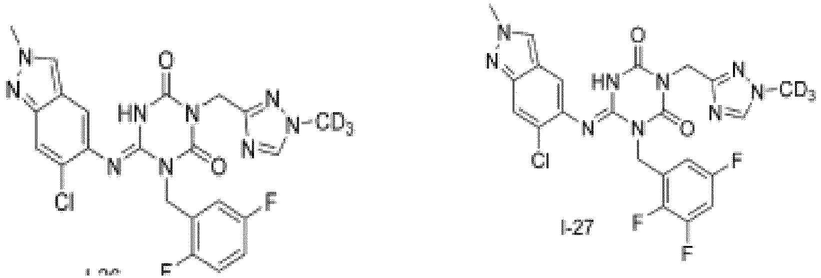

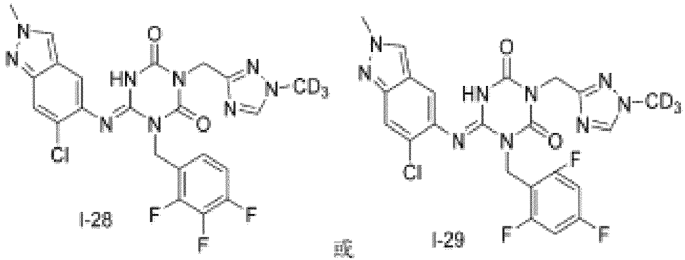

7. A triazine derivative, or a pharmaceutically acceptable salt thereof, as show n in formula (I') of claim 1, is **characterized in that** the triazine derivative show n in formula (I') is the triazine derivative shown in formula (I) or (III). 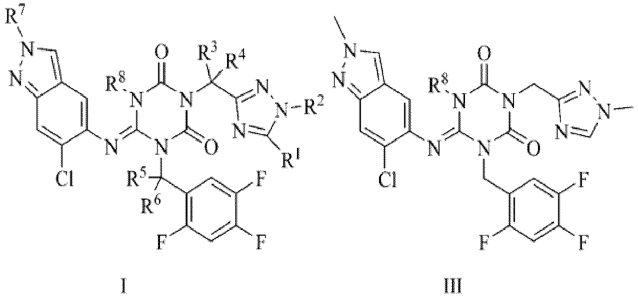
Where R' is hydrogen or deuterium.
R² is a methyl group or a methyl group replaced by 1, 2 or 3 deuterium.
R³ for hydrogen or deuterium.
R⁴ is hydrogen or deuterium.
R⁵ for hydrogen or deuterium.
R⁶ is hydrogen or deuterium.
R⁷ is a methyl group or a methyl group replaced by 1, 2 or 3 deuterium.
R⁸ is hydrogen, 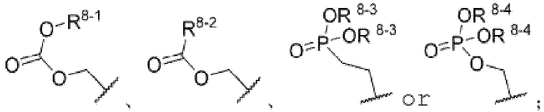
R⁸⁻¹ is C₁-C₁₀ alkyl.
R⁸⁻² is C₁-C₁₀ alkyl.
Each R⁸⁻³ is independently hydrogen, C₁-C₁₀ alkyl, or sodium.
Each R⁸⁻⁴ is independently hydrogen, C₁-C₁₀ alkyl, or sodium.
Moreover, R¹, R², R³, R⁴, R⁵, R⁶ and R⁷ contain at least one deuterium.
Formula (III) is not

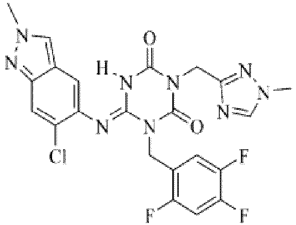

8. A triazine derivative or a pharmaceutically acceptable salt thereof as indicated in for mula (I') of claim 7 is **characterized by** a triazine derivative as indicated in formula (D) or (III) that satisfies one or more of the following conditions:
(1) In R⁸⁻¹, the mentioned C₁-C₁₀ alkyl group is C₁~C₆ alkyl groups, preferably methyl, ethyl, n-propyl, isopropyl, n-butyl, isobutyl, sec-butyl or tert-butyl groups, such as methyl, ethyl, isopropyl.
(2) In R⁸⁻², the mentioned C₁-C₁₀ alkyl group is C₁~C₆ alkyl groups, preferably methyl, ethyl, n-propyl, isopropyl, n-butyl, isobutyl, sec-butyl or tert-butyl, such as methyl, ethyl, isopropyl, n-butyl or tert-butyl.
(3) In R⁸⁻³, the mentioned C₁-C₁₀ alkyl group is C₁~C₆ alkyl groups, preferably methyl, ethyl, n-propyl, isopropyl, n-butyl, isobutyl, sec-butyl or tert-butyl.
(4) In R⁸⁻⁴, the mentioned C₁-C₁₀ alkyl group is C₁~C₆ alkyl groups, preferably methyl, ethyl, n-propyl, isopropyl, n-butyl, isobutyl, sec-butyl or tert-butyl.
(5) When R⁸ is Hydrogen, the three classes of derivatives shown in the formula (I') may also be tautomers of them, e.g. 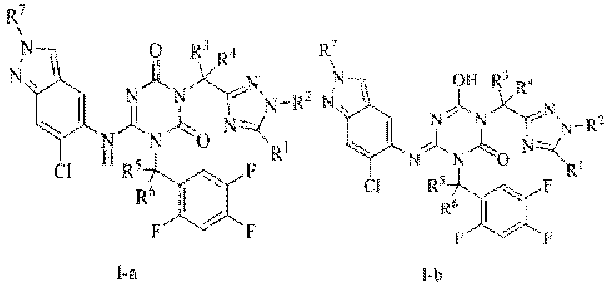 e. g.

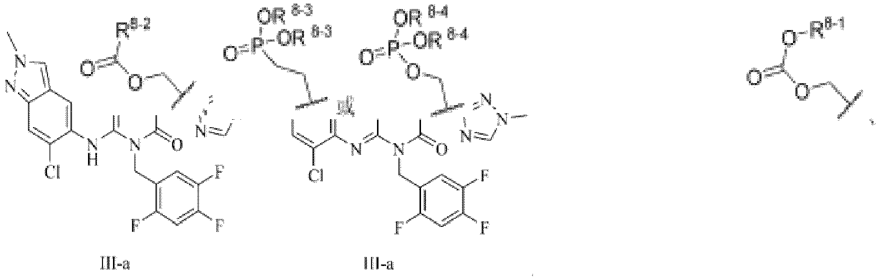

9. A triazine derivative, or a pharmaceutically acceptable salt thereof, as described in formula (I') of claim 7, is **characterized by** the fact that a triazine derivative as described in formula (I) satisfies one or more of the following conditions:
(1) R² is methyl or -CDs.
(2) R⁷ is methyl or -CDs.
(3) R⁸⁻¹ is C1 ~ C6 alkyl.
(4) R⁸⁻² is C1 ~ C3 alkyl.
(5) R⁸⁻³ is sodium.
(6) R⁸⁻⁴ is sodium.
(7) Both R³ and R⁴ are deuterium.
And (8) R⁵ and R⁶ are deuterium simultaneously.

10. Triazine derivatives or pharmaceutically acceptable salts thereof, as indicated in formula (I') of any of the claims 7-9, are **characterized by**,

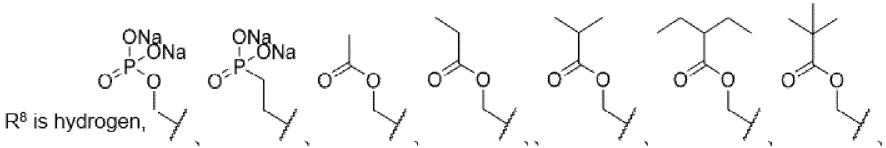

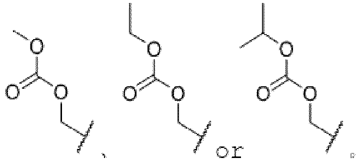

11. A triazine derivative or a pharmaceutically acceptable salt thereof as indicated in formula (I) of claim 7 is **characterized by** the fact that the triazine derivative as indicated in formula (I) is option 1, option 2, option 3, option 4 or option 5,
Option 1.
R¹ is tritium, R² is methyl, R³ is hydrogen, R⁴ is hydrogen, R⁵ is hydrogen, R⁶ is hydrogen,
R⁷ is methyl, R⁸ is hydrogen, 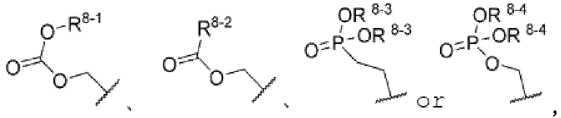
R⁸⁻¹ is C₁-C₁₀ alkyl, R⁸⁻² is C₁-C₁₀ alkyl, Each R⁸⁻³ is independently hydrogen, C₁-C₁₀ alkyl, or sodium,
Each R⁸⁻⁴ is independently hydrogen, C₁-C₁₀ alkyl, or sodium.
Option 2.
R¹ is hydrogen, R² is -CD3, R³ is hydrogen, R⁴ is hydrogen, R⁵ is hydrogen, R⁸ is hydrogen, R⁷ is methyl, R⁸ is hydrogen, 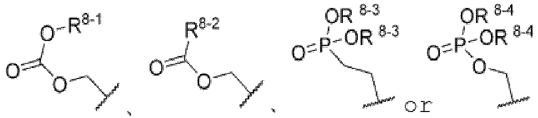
R⁸⁻¹ is C₁-C₁₀ alkyl, R⁸⁻² is C₁-C₁₀ alkyl, Each R⁸⁻³ is independently hydrogen, C₁-C₁₀ alkyl, or sodium,
Each R⁸⁻⁴ is independently hydrogen, C₁-C₁₀ alkyl, or sodium.
Option 3.
R¹ is hydrogen, R² is methyl, R³ is tritium, R⁴ is tritium, R⁵ is hydrogen, R⁸ is hydrogen,
R⁷ is methyl, R⁸ is hydrogen, 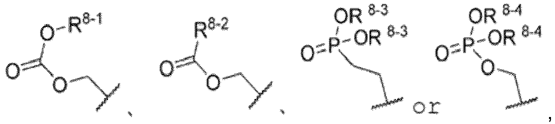
R⁸⁻¹ is C₁-C₁₀ alkyl. R⁸⁻² is C₁-C₁₀ alkyl. Each R⁸⁻³ is independently hydrogen, C₁-C₁₀ alkyl, or sodium.
Each R⁸⁻⁴ is independently hydrogen, C₁-C₁₀ alkyl, or sodium,
Option 4.
R¹ is hydrogen, R² is methyl, R³ is hydrogen, R⁴ is hydrogen, R⁵ is tritium, R⁶ is tritium,
R⁷ is methyl, R⁸ is hydrogen, 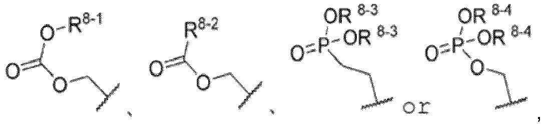
R⁸⁻¹ is C₁-C₁₀ alkyl, R⁸⁻² is C₁-C₁₀ alkyl. Each R⁸⁻³ is independently hydrogen, C₁-C₁₀ alkyl, or sodium.
Each R⁸⁻⁴ is independently hydrogen, C₁-C₁₀ alkyl, or sodium.
Option 5
R' is hydrogen, R² is methyl, R³ is hydrogen, R⁴ is hydrogen, R⁵ is hydrogen, R⁶ is hydro gen,
R⁷ is -CD₃, R⁸ is hydrogen, 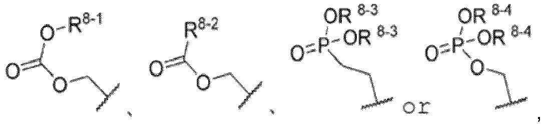
R⁸⁻¹ is C₁-C₁₀ alkyl, R⁸⁻² is C₁-C₁₀ alkyl. Each R⁸⁻³ is independently hydrogen, C₁-C₁₀ alkyl, or sodium.
Each R⁸⁻⁴is independently hydrogen, C₁-C₁₀ alkyl, or sodium.

12. A triazine derivative, or a pharmaceutically acceptable salt thereof, as shown in formula (I') of claim 1, is **characterized in that** the triazine derivative shown in formula (I') is the triazine derivative shown in VI 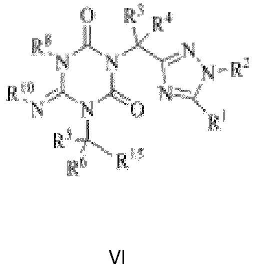
Where R' is hydrogen or deuterium,
R² is a methyl group or a methyl group replaced by 1, 2, or 3 tritium,
R³ is hydrogen or deuterium,
R⁴ is hydrogen or deuterium,
R⁵ is hydrogen or deuterium,
R⁶ is hydrogen or deuterium,
R⁸ is hydrogen, 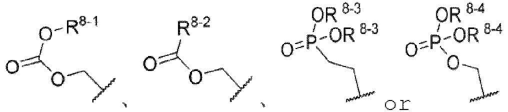
R⁸⁻¹ is C₁-C₁₀ alkyl,
R⁸⁻² is C₁-C₁₀ alkyl,
Each R⁸⁻³ is independently hydrogen, C₁-C₁₀ alkyl, or sodium,
Each R⁸⁻⁴ is independently hydrogen, C₁-C₁₀ alkyl, or sodium,
R¹⁰ is a 6-10 meta heteroaryl group that is not replaced or is replaced by one or more R¹⁰⁻¹; The type of heteroatom of the 6-10 meta heteroaryl group is selected from one or more of N, O and S, and the number of heteroatom is 1, 2 or 3;
Each R¹⁰⁻¹ is independently a C₁~C₆ alkyl, halogen, or deuterium that is not replaced or is replaced by one or more R¹⁰⁻¹⁻¹;
Each R¹⁰⁻¹⁻¹ is independently a halogen or deuterium,
R15 is

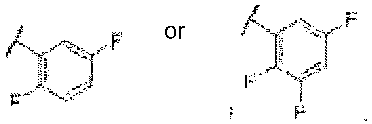

13. A triazine derivative or a pharmaceutically acceptable salt thereof as indicated in formula (T') of claim 12 is **characterized by** a triazine derivative as indicated in formula VI that satisfies one or more of the following conditions:
(1) In R⁸⁻¹, the mentioned C₁-C₁₀ alkyl group is C₁~C₆ alkyl groups, preferably methyl, ethyl, n-propyl, isopropyl, n-butyl, isobutyl, sec-butyl or tert-butyl groups, such as methyl, ethyl, isopropyl.
(2) In R⁸⁻², the mentioned C₁-C₁₀ alkyl group is C₁~C₆ alkyl groups, preferably methyl, ethyl, n-propyl, isopropyl, n-butyl, isobutyl, sec-butyl or tert-butyl, such as methyl, ethyl, isopropyl, n-butyl or tert-butyl.
(3) In R⁸⁻³, the mentioned C₁-C₁₀ alkyl group is C₁~C₆ alkyl groups, preferably methyl, ethyl, n-propyl, isopropyl, n-butyl, isobutyl, sec-butyl or tert-butyl.
(4) In R⁸⁻⁴, the mentioned C₁-C₁₀ alkyl group is C₁~C₆ alkyl groups, preferably methyl, ethyl, n-propyl, isopropyl, n-butyl, isobutyl, sec-butyl or tert-butyl.
(5) In R¹⁰, the 6-10 meta heteroaryl group is 5 and 6 meta heteroaryl group, and/or the 6-10 meta heteroaryl group is a heteroatom class of N and/or O, and/or the 6-10 meta heteroaryl group is a heteroatom number of 2; The 6-10 meta heteraryl group is preferably 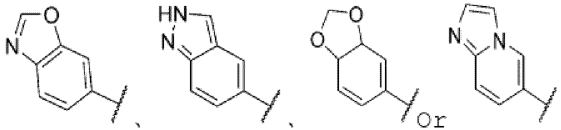
(6) R¹⁰⁻¹, the mentioned C₁~C₆ alkyl group is methyl, ethyl, n-propyl, isopropyl, n-butyl, isobutyl, sec-butyl, or tert-butyl, such as methyl.
(7) the Halogen is fluorine, chlorine, bromine or iodine, such as fluorine or chlorine.
(8) R^{10-1-1,} the halogen is atmosphere, chlorine, bromine or iodine, such as fluorine or chlorine.

14. A triazine derivative or a pharmaceutically acceptable salt thereof as indicated in formula (I') of claim 12 is **characterized by** a triazine derivative as indicated in formula VI that satisfies one or more of the following conditions:
(1) R' is hydrogen.
(2) R² is methyl or -CDs.
(3) R³ is hydrogen.
(4) R⁴ is hydrogen.
(5) R⁵ is hydrogen.
(6) R⁶ is hydrogen.
(7) each R¹⁰⁻¹ is independently chlorine, methyl, fluorine, or-CDs And in (8) R¹⁰, the 6-10 meta heteroaryl group is, 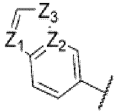 Z₁ is N or O; Z₂ is N or C; Z₃ is C or O. In a better way, R¹⁰ is 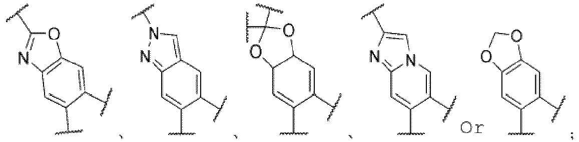 Another example

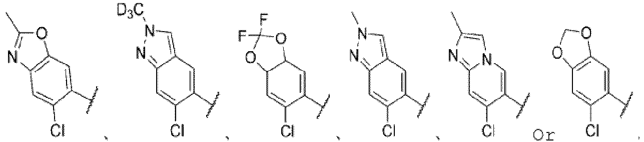

15. A binary coproduct of a triazine derivative with an acid as indicated in formula I' of any of the items in claim 1-14. the acid is malic acid, maleic acid, citric acid, ascorbic acid, mandelic acid, tartaric acid, fumaric acid or acetic acid, preferably fumaric acid or acetic acid. Preferably, the binary eutectic is or 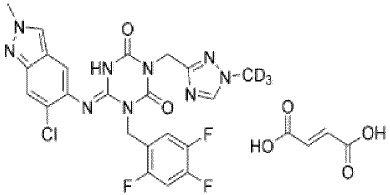 or 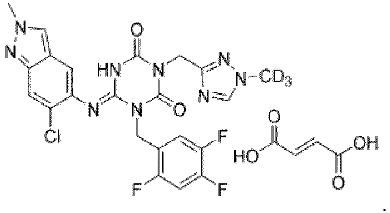
Preferably, the binary coproduct 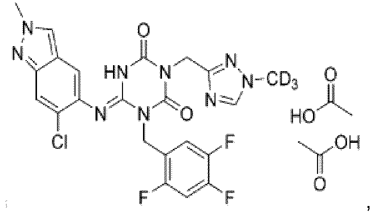
which is represented by an X-ray powder at 2θ angles, diffraction peaks are found at 5.4±0.2, 19.8±0.2, 22.2±0.2, 25.5± 0.2. The X-ray powder diffraction pattern represented by 2θ angles also has diffraction peaks at one or more of 9.9±0.2,10.7±0.2,12.6±0.2,27.3±0.2 and 28.0±0.2. Preferably, its X-ray powder diffraction pattern expressed in 2θ angles is shown in Figure 1:
Preferably, the binary eutectic 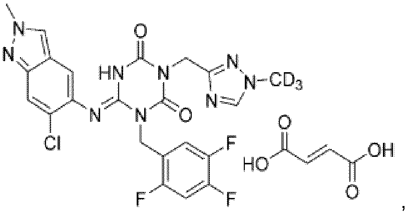
which is represented by 2θ angles in X-rays, the powder diffraction pattern has diffraction peak at 7.85±0.2°, 9.55±0.2°, 10.22±0.2°,12.01±0.2°,13.88±0.2°,14.79±0.2°,17.19±0.2°,18.71±0.2°,19.16±0.2°,23.60± 0.2°, 23.85±0.2°, 24.76±0.2°, 28.95±0.2°. Preferably, the X-ray powder diffraction pattern represented by 2θ angles also has diffraction peaks at one or more places of 6.05±0.2° , 11.02± 0.2° , 11.60±0.2° , 12.39±0.2° , 13.43±0.2° 15.21 ±0.2° 16.41 ±0.2° , 18.13±0.2° , 19.59± 0.2° 19.91 ±0.2° , 20.43±0.2° , 20.97±0.2° , 21.53±0.2° , 22.04±0.2° , 22.70±0.2° ,23.16 ±0.2° , 25.47±0.2° , 27.13±0.2° , 27.77±0.2° , 28.28 ±0.2° , 29.76±0.2° , 31.17±0.2° , 32.11 ±0.2° , 32.64±0.2° , 33.34±0.2° , 34.03±0.2° , and 35.00±0.2° ; Preferably, its X-ray powder diffraction pattern expressed at 2θ angles is shown in Figure 2.

16. A ternary coproduct formed by a triazine derivative with niacinamide and acid as indicated in formula I' ofany item of claim 1-14. The acids are malic acid, maleic acid, citric acid, ascorbic acid, mandelic acid, tartaric acid, fumaric acid or acetic acid, fumaric acid is preferred.
Preferably, the ternary eutectic is 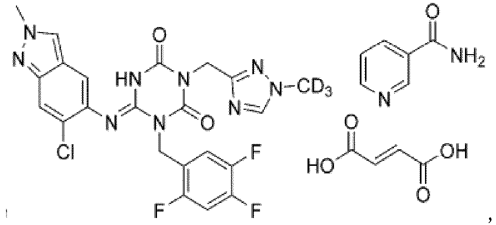
Preferably, the ternary eutectic is 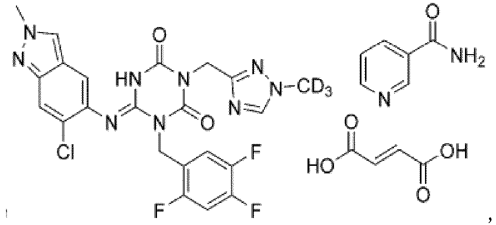 its X-ray powder diffraction pattern represented by 2θ angles has diffraction peaks at 11.2±0.2° , 15.7±0.2° , 18.9±0.2° , 19.3±0.2° , 22.9±0.2° , 24.0±0.2° , 24.9±0.2° and 29.6±0.2° ;
Preferably, It also has diffraction peaks at one or more places of 10.4±0.2° , 11.8±0.2° ,12.2:1:: 0.2° , 12.6±0.2° , 13.6±0.2° , 14.1 ±0.2° , 15.0±0.2° , 17.4±0.2° , 18.2±0.2° , 18.5±0.2° , 19.8±0.2° 20.1 ±0.2° , 20.3±0.2° , 20.6±0.2° , 21.2±0.2° , 21.7±0.2° , 22.2±0.2° , 22.5± 0.2° ,23.3±0.2° , 23.8±0.2° , 25.6±0.2° , 26.0±0.2° , 27.6±0.2° , 28.0±0.2° , 28.9±0.2° , 29.7±0.2° , 29.9±0.2° , 31.4±0.2° , 32.8±0.2° , 33.5±0.2° , 35.1 ±0.2° , 36.3±0.2° and 39.6 ±0.2° ; Preferably, its X-ray powder diffraction pattern expressed at 2θ angles is shown in Figure 3.

17. A pharmaceutical composition **characterized in that** it includes substance B' and one or more pharmaceutically acceptable carriers; Substance B' is a triazine derivative or a pharmaceutically acceptable salt thereof as described in formula I of any of the items in claim 1-14, a binary co-product as described in claim 15, or a ternary co-product as described in claim 16;The amount of substance B' may be a therapeutic effective amount.

18. The use of a substance B' in the preparation of an antiviral drug, which is a triazine derivative or a pharmaceutically acceptable salt thereof as described in formula I of any of the claims 1-14, a binary eutectic as described in claim 15, a ternary eutectic as described in claim 16, or a pharmaceutical composition as described in claim 17. The viruses are coronaviruses, influenza viruses, respiratory syncytial viruses, flaviviridae viruses, filoviridae viruses or porcine Epidemic diarrhea virus (PEDV). Preferably, the coronavirus is selected from one or more of MERS-CoV, SARS-CoV, and SARS-CoV-2, and the coronavirus is preferred as SARS-CoV-2.

19. The application of a substance B' in the preparation of drugs for the treatment and/or prevention of coronavirus-related diseases, which is a triazine derivative or its pharmaceutically acceptable salt as indicated in Formula I' of any of the subparagraphs of claim 1-14, a binary eutectic as described in claim 15. Ternary eutectic as described in claim 16 or pharmaceutical compositions as described in claim 17.

20. A method of preparing triazine derivatives shown in formula (I') is **characterized by** method 1, method 2, method 3, method 4, or method 5.
The method 1 comprises the following steps where, in a solvent (e.g., anhydrous tetrahydrofuran), in the presence of a base (e.g., Hexamethyldisilylamine lithium), a compound shown in formula I'-S1 reacts with heavy water as shown below to obtain a compound shown in formula I'. 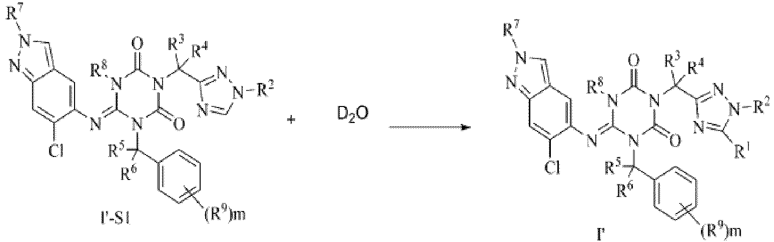 Where R' is deuterium; R², R³, R⁴, R⁵, R⁶, R⁷ and R⁸ are defined in any of the terms of Clai m 1-14.
Method 2 consists of the following steps: In a solvent (e.g., a mixed solution of acetic acid and tert-butanol), a compound shown in formula I'-S2 reacts with a compound shown in formula I'-S3 as shown below to obtain a compound shown in formula I'. 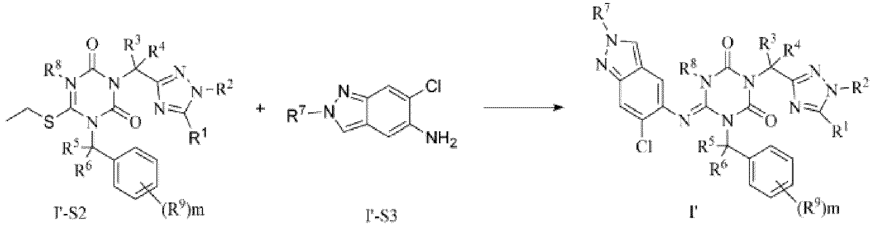
Where R¹, R², R³, R⁴, R⁵, R⁶, R⁷, R⁸, R⁸⁻¹, R⁸⁻², each R⁸⁻³ and each R⁸⁻⁴ are defined in any of the terms of Claim 1-14.
Method 3 consists of the following steps in which, in a solvent (e.g., N, N-dimethylformamide or dimethylacetamide) and in the presence of a base (e.g., anhydrous cesium carbonate or potassium carbonate), a compound shown in formula I'-S4 reacts with a compound shown in formula I'-S5 as shown below to obtain a compound shown in formula I'. 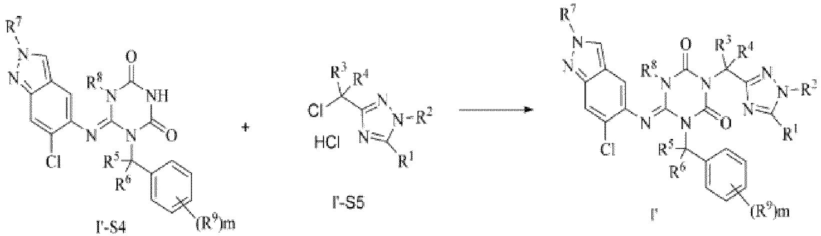
Where R¹, R², R³, R⁴, R⁵, R⁶, R⁷, R⁸, R⁸⁻¹, R⁸⁻², each R⁸⁻³ and each R⁸⁻⁴ are defined in any of the terms of Claim 1-14.
The method 4 consists of the following steps, in a solvent (e.g., dichloromethane), a compound shown in formula I'-S6 reacts with sodium hydroxide (e.g., an ethanol solution of sodium hydroxide) as shown below to obtain a compound shown in formula I' 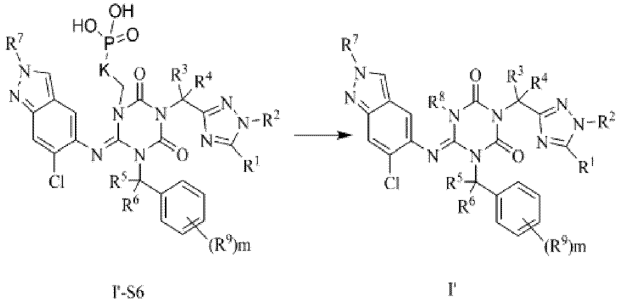
Where K is or CH₂, R⁸ is 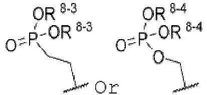
Each R⁸⁻³ and each R⁸⁻⁴are sodium, Where R¹, R², R³, R⁴, R⁵, R⁶ and R⁷ are defined in any of the terms of Claim 1-14.
Method 5 consists of the following steps: In a solvent (e.g., N, N-dimethylformamide or dimethylacetamide), in the presence of a base (e.g., sodium hydride), a compound shown in formula I'-S7 reacts with a compound shown in formula I'-S8 as shown below to obtain a compound shown in formula I', 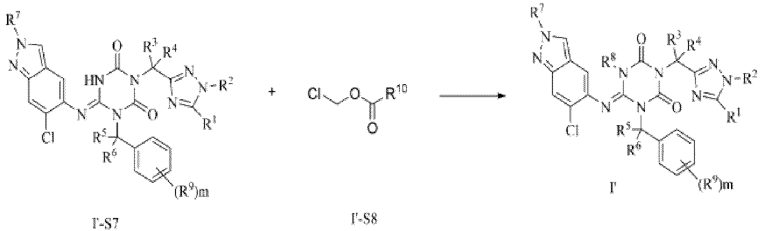
Where R¹⁰ is C₁-C₁₀alkyl or-OC₁-C₁₀ alkyl, R⁸ is 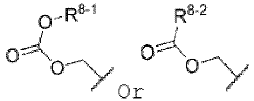
Each R⁸⁻¹ and each R⁸⁻²are independently C₁-C₁₀ alkyl. R¹, R², R³, R⁴, R⁵, R⁶, R⁷ are defined in any of the terms of Claim 1-14.
